# EUROPEAN PATENT APPLICATION

(11) **EP 3 733 715 A1**
(43) Date of publication of application: **04.11.2020**
(21) Application number: 18897585.8
(22) Date of filing: 26.12.2018
(51) Int. Cl.: C07K 16/46, C12N 15/13, C12N 15/70, C12N 15/85, C12N 5/10, C12N 1/21, A61K 39/395, A61P 37/02, A61P 29/00, A61P 31/00, A61P 35/00

(54) **TRIABODY, PREPARATION METHOD AND USE THEREOF**

(30) Priority: 27.12.2017 CN 201711448490; 13.12.2018 CN 201811525053
(71) Applicant: Innovent Biologics (Suzhou) Co., Ltd., Suzhou, Jiangsu 215123 (CN)
(72) Inventor: MIAO, Xiaoniu, Suzhou, Jiangsu 215123 (CN); KUANG, Zhihui, Suzhou, Jiangsu 215123 (CN); HU, Huajing, Suzhou, Jiangsu 215123 (CN); NI, Haiqing, Suzhou, Jiangsu 215123 (CN); LIU, Junjian, Suzhou, Jiangsu 215123 (CN)
(74) Representative: Hoffmann Eitle
(86) International application number: PCT/CN2018/123886
(87) International publication number: WO 2019/129054

(57) **Abstract**

The present invention provides a novel artificial triple-chain antibody comprising three polypeptide chains, wherein a first polypeptide chain comprises a first heavy-chain variable domain, a second polypeptide chain comprises a first light-chain variable domain that is paired with the first heavy-chain variable domain to form a first antigen-binding site; and a third polypeptide chain comprises a second single-domain antigen-binding site and a third single-domain antigen-binding site. The present invention also provides a polynucleotide encoding the triple-chain antibody, a vector comprising the polynucleotide, a host cell comprising the polynucleotide or vector, an immunoconjugate comprising the triple-chain antibody and a pharmaceutical composition comprising the triple-chain antibody or the immunoconjugate thereof, and use of the triple-chain antibody in immunotherapy, prevention and/or diagnosis of diseases.

## Description

### Field of the Invention

The present invention generally relates to the field of immunology and antibody engineering. Specifically, the present invention relates to a novel artificial triple-chain antibody, a polynucleotide encoding the triple-chain antibody, a vector comprising the polynucleotide, a host cell comprising the polynucleotide or the vector, an immunoconjugate comprising the triple-chain antibody and a pharmaceutical composition comprising the triple-chain antibody or the immunoconjugate, and use of the triple-chain antibody in immunotherapy, prevention and/or diagnosis.

### Background of the Invention

Antibody molecules are capable of targeting and specific binding to their corresponding antigens, and are becoming important therapeutic agents, preventive agents, and/or diagnostic agents for a variety of diseases such as cancers, autoimmune diseases, inflammatory diseases, infectious diseases, etc. However, monospecific antibodies against a single target have some limitations in clinical applications. Patients may develop resistance or have no response after receiving monospecific antibody treatment. With researches on cancers and many other diseases, it is recognized that there are often multiple signal transduction pathways involved in the development and progression of diseases, and a single-target immunotherapy is usually insufficient to play a therapeutic role in many diseases.

Because multispecific antibodies such as bispecific antibodies can specifically bind to different antigens, when one antigen is located on a specific immune cell and another antigen is located on a diseased cell, multispecific antibodies such as bispecific antibodies can redirect specific immune cells to diseased cells to enhance the lethality of immune cells to diseased cells. In addition, multispecific antibodies such as bispecific antibodies can also be designed to act on signal transduction pathways of two or more different mediators simultaneously. These advantages have expanded the application of multispecific antibodies such as bispecific antibodies.

Several multispecific antibody (such as bispecific antibody) patterns have been developed through antibody engineering and their suitability in therapeutic applications has been studied. Currently, two bispecific antibody products approved for marketing are blinatumomab developed by Micromet and Amgen, and catumaxomab developed by Trion Pharma. Blinatumomab is the first single-chain bispecific antibody approved for marketing in the United States for the treatment of B-cell non-Hodgkin's lymphoma (NHL) and B precursor acute lymphoblastic leukemia (ALL) with a molecular weight of approximately 55 KDa. It is a fusion of two single-chain Fv molecules against CD19 and CD3 respectively via flexible linker peptide. Through CD19 expressed in almost all B lymphocyte tumors and CD3 expressed on T cells, it connects T cells and target cells (tumor cells) tightly together, allows T cells to release perforin and telomerase into the synaptic space, and causes a series of chemical reactions in the tumor cells, thereby destroying the tumor cells (Nagorsen D. and Baeuerle P.A., Immunomodulatory therapy of cancer with T cell-engaging BiTE antibody blinatumomab, Exp. Cell Res., 2011, 317:1255-1260). Catumaxomab is a chimera composed of two incomplete antibodies derived from a mouse IgG2a isotype and a rat IgG2b isotype respectively. Each incomplete antibody has one light chain and one heavy chain, and the anti-CD3 rat IgG2b portion is used for T cell recognition, and anti-tumor cell surface antigen EpCAM (epithelial cell adhesion molecule) mouse IgG2a portion is used for tumor cell recognition (Chelius D et al., Structural and functional characterization of the trifunctional antibody catumaxomab, MAbs, 2010, 2: 309-319). Catumaxomab (Removab®) was approved in Europe in April, 2009 for the treatment of malignant ascites caused by EpCAM-positive epithelial-derived metastases.

Multispecific antibodies such as bispecific antibodies can be categorized based on different components and construction methods. For example, based on the bilateral symmetry of the structure of multispecific antibodies, they can be categorized into symmetrical antibodies and asymmetric antibodies; based on the presence or absence of IgG Fc region in multispecific antibodies, they can be categorized into complete antibodies and antibody fragments; based on the amount of antigen binding sites in multispecific antibodies, they can be categorized into bivalent, trivalent, or tetravalent antibodies or those of greater valencies, and the like.

Different multispecific antibody designs have their own advantages and disadvantages. For example, although blinatumomab can be produced on a large scale using recombinant Chinese hamster ovary (CHO) cells, it is easy to form aggregates and has a short half-life in the body, and an additional continuous infusion device is required in practice; catumaxomab manufacturing process is complex and mouse heterologous antibodies are more likely to cause immunogenicity issues in humans. In addition, undesired pairing of unrelated heavy and light chains in a four-chain immunoglobulin (Ig)-like multispecific antibody may result in a formation of inactive antigen binding sites and/or other undesired dysfunctional by-products in the clinical scale manufacturing and therapeutic application of antibodies (Klein, C. et. al., Progress in overcoming the chain association issue in bispecific heterodimeric IgG antibodies, mAbs, 2012, 4: 653-663). Theoretically, two heavy chains can be associated in four different combinations, and each of these heavy chains can be associated with a light chain in a random manner, generating 2⁴ (= 16) possible combinations. Among these 16 theoretically possible combinations, 10 combinations are actually found, but only one of them corresponds to the required functional multispecific antibody. Challenges in isolating the required multispecific antibody from a complex mixture and theoretically maximum inherent poor yield of 12.5% make it difficult to produce an Ig-like multispecific antibody of four chains in a cell expression system.

The novel antibody pattern of the present invention overcomes the aforementioned disadvantages. The present invention provides a novel multispecific antibody pattern which is easily generated with an increased yield due to the correct coupling or pairing between various chains and easily expressed in cultured cells in vitro, and requires no complex manufacturing process. In addition, the multispecific antibody pattern of the present invention retains the affinity of each antigen binding site in the multispecific antibody for binding to corresponding different epitopes, and generates no steric hindrance when binding to different epitopes, resulting in good druggability. Further, the multispecific antibody pattern of the present invention is physically stable and biologically stable, which provides the antibody with better productivity and developability.

### Summary

Disclosed herein is a novel triple-chain antibody comprising three polypeptide chains constructed by an antibody engineering technique. The triple-chain antibody is capable of binding to one or more antigens and preferably to two or more antigens with high affinity and high specificity. A nucleic acid molecule encoding the triple-chain antibody, an expression vector, a host cell, and a method for producing the triple-chain antibody are also provided. The invention also provides an immunoconjugate comprising the triple-chain antibody of the present invention and a pharmaceutical composition comprising the triple-chain antibody or an immunoconjugate thereof. The triple-chain antibody disclosed herein can be used alone or in combination with other drugs or other treatment modalities to treat, prevent and/or diagnose diseases such as autoimmune diseases, acute and chronic inflammatory diseases, infectious diseases (e.g., chronic infectious diseases or sepsis), and tumors, etc.

Therefore, in one aspect, the present invention provides a triple-chain antibody having one or more of the following characteristics:
(a) specifically binding to one or more antigens with high affinity, for example, with an affinity constant of at least about 10⁷ M⁻¹, preferably about 10⁸ M⁻¹ and more preferably about 10⁹ M⁻¹ or greater;
(b) easily expressed in cells in vitro, and the three chains can be correctly coupled or paired;
(c) having good physical stability, in particular, having good long-term thermal stability, and being capable of maintaining biological activities for a long time; and
(d) exerting biological functions through the effect on signaling pathways involving one or more antigens to which the antibody specifically binds.

The triple-chain antibody of the present invention is at least a trivalent antibody (i.e., having at least three antigen binding sites). In one embodiment, the triple-chain antibody is a (1 + 2 pattern) trivalent antibody, of which a first polypeptide chain comprises a first heavy-chain variable domain, a second polypeptide chain comprises a first light-chain variable domain that is paired with the first heavy-chain variable domain (hereinafter abbreviated as VH1/VL1 pair) to form a first antigen binding site, and a third polypeptide chain of which comprises a second single-domain antigen binding site and a third single-domain antigen binding site.

In one embodiment, one antigen binding site contained in the triple-chain antibody of the present invention targets a first antigen, and the other two binding sites target the same or different epitope(s) of a second antigen, respectively. In one embodiment, the three antigen binding sites contained in the triple-chain antibody of the present invention target three different antigens. In one embodiment, the three antigen binding sites contained in the triple-chain antibody of the present invention target the same antigen.

In one embodiment, the present invention provides such a triple-chain antibody comprising three polypeptide chains, wherein a first polypeptide chain comprises a first heavy-chain variable domain and an immunoglobulin CHI domain, and a second polypeptide chain comprises a first light-chain variable domain and an immunoglobulin CL domain, the first heavy-chain variable domain paired with the first light-chain variable domain to form a first antigen binding site; and a third polypeptide chain comprises a second single-domain antigen binding site and a third single-domain antigen binding site, wherein the second single-domain antigen binding site and the third single-domain antigen binding site of the third polypeptide chain have or do not have a linker peptide therebetween.

In one embodiment, the second single-domain antigen binding site of the third polypeptide chain and the third single-domain antigen binding site have a linker peptide comprising glycine (G) and serine (S) residues therebetween, for example, a linker peptide comprising 1 to 7 GGGGS repeats, preferably a linker peptide comprising 4 GGGGS repeats.

In a preferred embodiment, the third polypeptide chain in the triple-chain antibody of the present invention does not comprise an immunoglobulin CHI domain; the second single-domain antigen binding site and the third single-domain antigen binding site are independently selected from heavy-chain variable domains of an antibody that naturally lacks a light chain (such as the heavy-chain variable domains of naturally occurring heavy-chain antibodies in Camelidae species), single-domain antigen binding sites in immunoglobulins called novel antigenic receptors (NAR) in fish (such as natural IgNAR in shark serum), and recombinant single-domain antigen binding sites derived therefrom. The heavy-chain variable domain derived from a heavy-chain antibody that naturally lacks a light chain is referred to as VHH herein to distinguish it from the conventional VH of a four-chain immunoglobulin. This VHH molecule can be derived from antibodies produced in camelidae species such as camels, alpacas, dromedaries, llamas, and guanaco. Species other than camelidae can also produce heavy-chain antibodies that naturally lack a light chain, and such VHHs are also within the scope of the present invention.

In one embodiment, in the third polypeptide chain of the triple-chain antibody of the present invention, the second single-domain antigen binding site and third single-domain antigen binding site are a first VHH and a second VHH respectively, the first VHH and the second VHH having the same or different sequence(s) and binding to the same or different antigen(s) or to different epitopes on the same antigen.

In yet another embodiment, the present invention provides such a triple-chain antibody comprising three polypeptide chains, wherein the first polypeptide chain comprises, from N-terminus to C-terminus, the first heavy chain variable domain, the immunoglobulin CHI domain, and an Fc domain; the second polypeptide chain comprises, from N-terminus to C-terminus, the first light chain variable domain and the immunoglobulin CL domain; and the third polypeptide chain comprises, from N-terminus to C-terminus, the second single-domain antigen-binding site, the third single-domain antigen-binding site and an Fc domain, preferably, the immunoglobulin is an IgG1, IgG2 or IgG4, and more preferably, the immunoglobulin is a human IgG1.

In the triple-chain antibody comprising three polypeptide chains provided by the present invention, the inventor also provides amino acid residues which can stabilize the structure of the triple-chain antibody and facilitate correct coupling or pairing between the respective chains. For example, a hinge region having "CPPC" amino acid residues is contained in the Fc domains of the first polypeptide chain and the third polypeptide chain of the triple-chain antibody, such that the first polypeptide chain and the third polypeptide chain are stably associated with each other through a disulfide bonds formed between amino acid residues at the hinge region. In one embodiment, the first polypeptide chain and the third polypeptide chain of the triple-chain antibody of the invention comprise Y349C and S354C, or S354C and Y349C, respectively, in the respective Fc domains (numbering by EU index according to Kabat et al., Sequences of Proteins of Immunological Interest, 5th edition, Public Health Service, National Institutes of Health, Bethesda, MD (1991), hereinafter referred to as "EU numbering"), thus the first polypeptide chain and the third polypeptide chains further forming interchain disulfide bonds in the Fc region to stabilize the correct pairing of the first and third polypeptide chains.

In one embodiment, the first polypeptide chain and/or the third polypeptide chain of the triple-chain antibody of the invention comprises amino acid mutations in the Fc domain which affects the function of the antibody effector. In one specific embodiment, the effector function is antibody-dependent cell-mediated cytotoxicity (ADCC). In one embodiment, the amino acid mutation is present in the CH2 domain of the Fc region. For example, the triple-chain antibody comprises amino acid substitutions at positions 234 and 235 (EU numbering) of the first polypeptide chain and/or the third polypeptide chain. In one specific embodiment, the amino acid substitutions are L234A and L235A (hereinafter referred to as "LALA mutations").

In one embodiment, the first polypeptide chain and the third polypeptide chain of the triple-chain antibody of the invention respectively comprise a protuberance ("protuberance") and a cavity ("cavity") in their Fc domains, or vice versa, and the protuberance or cavity in Fc domains of the first polypeptide chain can be respectively placed at the cavity or protuberance in the Fc domain of the third polypeptide chain, such that the first polypeptide chain and the third polypeptide chain form a stable association of "knob-in-hole". In one embodiment, the amino acid substitution T366W is contained in one of the first polypeptide chain and the third polypeptide chain, and the amino acid substitutions T366S, L368A, and Y407V (EU numbering) are contained in the other one of the first polypeptide chain and the third polypeptide chain. Thus, the protuberance in one chain can be placed in the cavity in the other chain, which promotes the correct pairing of the first polypeptide chain and the third polypeptide chain.

In one embodiment, the immunoglobulin CHI domain and CL domain of the first polypeptide chain and the second polypeptide chain of the triple-chain antibody of the present invention respectively comprise a protuberance and a cavity, or vice versa, and the protuberance or cavity in the CHI domain can be placed at the cavity or protuberance in the CL domain, respectively, such that the first polypeptide chain and the second polypeptide chain also form a stable "knob-in-hole" association with each other.

The first antigen binding site, the second antigen binding site, and the third antigen binding site in the triple-chain antibody of the present invention may bind to epitopes on the same antigen or different antigens. For example, the first antigen binding site binds to an epitope of a first antigen, and the second antigen binding site and the third antigen binding site bind to the same or different epitope(s) on a second antigen, thus the triple-chain antibody being a bispecific antibody against the first antigen and the second antigen. When the first antigen binding site binds to an epitope of a first antigen, and the second antigen binding site and the third antigen binding site bind respectively to an epitope of a second antigen and an epitope of a third antigen, the triple-chain antibody is a trispecific antibody. The type of antigen to which the triple-chain antibody of the present invention specifically binds is not particularly limited, and the antigen may be, for example, a cytokine, a growth factor, a hormone, a signaling protein, an inflammatory mediator, a ligand, a cell surface receptor, or a fragment thereof.

In one embodiment, the antigen to which the triple-chain antibody of the present invention specifically binds is selected from tumor-associated antigens, immune checkpoint molecules, co-stimulatory molecules in the immune system, and ligands and/or receptors of such molecules, such as CD47, PD1, PD-L1, PD-L2, LAG-3, and 4-1BB (CD137).

The present invention exemplifies several types of triple-chain bispecific antibodies as described below.
i) In one embodiment, the triple-chain antibody of the invention is an anti-CD47/PD-L1 bispecific antibody, which is capable of binding to CD47 on the surface of tumor cells with an affinity constant of at least about 10⁷ M⁻¹, preferably about 10⁸ M⁻¹, and more preferably about 10⁹ M⁻¹ or greater, thereby blocking the binding of CD47 to SIRPα on the surface of macrophages and promoting the phagocytosis of tumor cells by macrophages in the tumor tissue infiltration area; and binding to PD-L1 on the surface of tumor cells with an affinity constant of at least about 10⁷ M⁻¹, preferably about 10⁸ M⁻¹, and more preferably about 10⁹ M⁻¹ or greater, thereby inhibiting the binding of PD-1 on T cells to PD-L1 on the surface of tumor cells, inducing T cell activation and exerting anti-tumor effects.
   In one embodiment, the anti-CD47/PD-L1 bispecific antibody of the present invention comprises a first antigen binding site specifically binding to CD47 and comprising a VH1/VL1 pair on the first polypeptide chain and the second polypeptide chain, and a first VHH and a second VHH specifically binding to PD-L1 on the third polypeptide chain.
   In one embodiment, the first antigen binding site specifically binding to CD47 and comprising the VH1/VL1 pair on the first polypeptide chain and the second polypeptide chain, comprises a VH CDR1 shown in GSIEHYYWS (SEQ ID NO: 3), a VH CDR2 shown in YIYYSGSTNYNPSLKS (SEQ ID NO: 4), a VH CDR3 shown in ARGKTGSAA (SEQ ID NO: 5), a VL CDR1 shown in RASQGISRWLA (SEQ ID NO: 10), a VL CDR2 shown in AASSLQS (SEQ ID NO: 11), and a VL CDR3 shown in QQTVSFPIT (SEQ ID NO: 12) derived from anti-CD47 antibody ADI-29341, or sequences having one, two, three, four, or five amino acid changes (e.g., amino acid substitutions or deletions) from one or more of the six CDRs. In another embodiment, the first antigen binding site specifically binding to CD47 and comprising a VH1/VL1 pair on the first polypeptide chain and the second polypeptide chain, comprises paired heavy-chain variable region/light-chain variable region sequences of SEQ ID NOs: 2/9 derived from the anti-CD47 antibody ADI-29341, or sequences having a sequence identity of at least 90%, 91%, 92%, 93%, 94%, 95%, 96%, 97%, 98%, 99% or higher with the paired heavy-chain variable region/light-chain variable region sequences.
   In one embodiment, the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or sequences having one, two, three, four, or five amino acid changes (e.g., amino acid substitutions or deletions) from one or more of the three CDRs. In another embodiment, the second and the third single-domain antigen binding sites specifically binding to PD-L1 on the third polypeptide chain comprise an amino acid sequence shown in SEQ ID NO: 15 and/or SEQ ID NO: 16, or sequences substantially identical (e.g., having at least 80%, 85%, 90%, 92%, 95%, 97%, 98%, 99% or higher identity) thereto.
   In one embodiment, the triple-chain anti-CD47/PD-L1 bispecific antibody of the present invention comprises a first polypeptide chain shown in SEQ ID NO: 1, a second polypeptide chain shown in SEQ ID NO: 8, and a third polypeptide chain shown in SEQ ID NO: 14, or sequences substantially identical (e.g., having at least 80%, 85%, 90%, 92%, 95%, 97%, 98%, 99% or higher identity) to any one of the sequences.
   In another embodiment, the triple-chain anti-CD47/PD-L1 bispecific antibody of the present invention comprises a first polypeptide chain shown in SEQ ID NO: 1, a second polypeptide chain shown in SEQ ID NO: 8, and a third polypeptide chain shown in SEQ ID NO: 22, or sequences substantially identical (e.g., having at least 80%, 85%, 90%, 92%, 95%, 97%, 98%, 99% or higher identity) to any one of the sequences.
ii) In one embodiment, the triple-chain antibody of the present invention is an anti-4-1BB/PD-L1 bispecific antibody, which is capable of binding to 4-1BB on the surface of T cells with an affinity constant of at least about 10⁷ M⁻¹, preferably about 10⁸ M⁻¹, and more preferably about 10⁹ M⁻¹ or greater, thereby activating the co-stimulatory signaling pathway of 4-1BB/4-1BB ligand and inducing the activation, proliferation, and anti-apoptosis activity of T cells; and binding to PD-L1 on the surface of tumor cells with an affinity constant of at least about 10⁷ M⁻¹, preferably about 10⁸ M⁻¹, and more preferably about 10⁹ M⁻¹ or greater, thereby inhibiting the binding of PD-1 on T cells to PD-L1 on the surface of tumor cells, inducing T cell activation and exerting anti-tumor effects.
   In one embodiment, the anti-4-1BB/PD-L1 bispecific antibody of the present invention comprises a first antigen binding site specifically binding to 4-1BB and comprising a VH1/VL1 pair on the first polypeptide chain and the second polypeptide chain, and a first VHH and a second VHH specifically binding to PD-L1 on the third polypeptide chain.
   In one embodiment, the first antigen binding site specifically binding to 4-1BB and comprising a VH1/VL1 pair on the first polypeptide chain and the second polypeptide chain, comprises all of the six heavy-chain complementarity determining regions (CDRs) and light-chain CDRs contained in the paired heavy-chain variable region/light-chain variable region sequences of SEQ ID NOs: 26/28 derived from the anti-4-1BB antibody BMS-663513, or sequences having one, two, three, four, or five amino acid changes (e.g., amino acid substitutions or deletions) from one or more of the six CDRs. In one embodiment, the first antigen binding site specifically binding to 4-1BB and comprising a VH1/VL1 pair on the first polypeptide chain and the second polypeptide chain, comprises paired heavy-chain variable region/light-chain variable region sequences of SEQ ID NOs: 26/28 derived from the anti-4-1BB antibody BMS-663513, or sequences having a sequence identity of at least 90%, 91%, 92%, 93%, 94%, 95%, 96%, 97%, 98%, 99% or higher with the paired heavy-chain variable region/light-chain variable region sequences.
   In one embodiment, the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or sequences having one, two, three, four, or five amino acid changes (e.g., amino acid substitutions or deletions) from one or more of the three CDRs. In another embodiment, the second and the third single-domain antigen binding sites specifically binding to PD-L1 on the third polypeptide chain comprise an amino acid sequence shown in SEQ ID NO: 15 and/or SEQ ID NO: 16, or sequences substantially identical (e.g., having at least 80%, 85%, 90%, 92%, 95%, 97%, 98%, 99% or higher identity) thereto.
   In one embodiment, the triple-chain anti-4-1BB/PD-L1 bispecific antibody of the present invention comprises a first polypeptide chain shown in SEQ ID NO: 25, a second polypeptide chain shown in SEQ ID NO: 27, and a third polypeptide chain shown in SEQ ID NO: 14, or sequences substantially identical (e.g., having at least 80%, 85%, 90%, 92%, 95%, 97%, 98%, 99% or higher identity) to any one of the sequences.
   In another embodiment, the triple-chain anti-4-1BB/PD-L1 bispecific antibody of the present invention comprises a first polypeptide chain shown in SEQ ID NO: 25, a second polypeptide chain shown in SEQ ID NO: 27, and a third polypeptide chain shown in SEQ ID NO: 22, or sequences substantially identical (e.g., having at least 80%, 85%, 90%, 92%, 95%, 97%, 98%, 99% or higher identity) to any one of the sequences.
iii) In one embodiment, the triple-chain antibody of the present invention is an anti-LAG-3/PD-L1 bispecific antibody, which is capable of binding to LAG-3 on the surface of T cells with an affinity constant of at least about 10⁷ M⁻¹, preferably about 10⁸ M⁻¹, and more preferably about 10⁹ M⁻¹ or greater, inhibiting LAG-3 inhibitory signaling pathway in T cells, and thereby restoring CD8+ effector T cells and reducing Treg population; and binding to PD-L1 on the surface of tumor cells with an affinity constant of at least about 10⁷ M⁻¹, preferably about 10⁸ M⁻¹, and more preferably about 10⁹ M⁻¹ or greater, thereby inhibiting the binding of PD-1 on T cells to PD-L1 on the surface of tumor cells, inducing T cell activation and exerting anti-tumor effects.

In one embodiment, the anti-LAG-3/PD-L1 bispecific antibody of the present invention comprises a first antigen binding site specifically binding to LAG-3 and comprising a VH1/VL1 pair on the first polypeptide chain and the second polypeptide chain, and a first VHH and a second VHH specifically binding to PD-L1 on the third polypeptide chain.

In one embodiment, the first antigen binding site specifically binding to LAG-3 and comprising the VH1/VL1 pair on the first polypeptide chain and the second polypeptide chain, comprises a VH CDR1 shown in GSIYSESYYWG (SEQ ID NO: 31), a VH CDR2 shown in SIVYSGYTYYNPSLKS (SEQ ID NO: 32), a VH CDR3 shown in ARVRTWDAAFDI (SEQ ID NO: 33), a VL CDR1 shown in QASQDISNYLN (SEQ ID NO: 36), a VL CDR2 shown in DASNLET (SEQ ID NO: 37), and a VL CDR3 shown in QQVLELPPWT (SEQ ID NO: 38) derived from anti-LAG-3 antibody ADI-31853, or sequences having one, two, three, four, or five amino acid changes (e.g., amino acid substitutions or deletions) from one or more of the six CDRs. In one embodiment, the first antigen binding site specifically binding to LAG-3 and comprising a VH1/VL1 pair on the first polypeptide chain and the second polypeptide chain, comprises paired heavy-chain variable region/light-chain variable region sequences of SEQ ID NOs: 30/35 derived from the anti-LAG-3 antibody ADI-31853, or sequences having a sequence identity of at least 90%, 91%, 92%, 93%, 94%, 95%, 96%, 97%, 98%, 99% or higher with the paired heavy-chain variable region/light-chain variable region sequences.

In one embodiment, the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or sequences having one, two, three, four, or five amino acid changes (e.g., amino acid substitutions or deletions) from one or more of the three CDRs. In another embodiment, the second and the third single-domain antigen binding sites specifically binding to PD-L1 on the third polypeptide chain comprise an amino acid sequence shown in SEQ ID NO: 15 and/or SEQ ID NO: 16, or sequences substantially identical (e.g., having at least 80%, 85%, 90%, 92%, 95%, 97%, 98%, 99% or higher identity) thereto.

In one embodiment, the triple-chain anti-LAG-3/PD-L1 bispecific antibody of the present invention comprises a first polypeptide chain shown in SEQ ID NO: 29, a second polypeptide chain shown in SEQ ID NO: 34, and a third polypeptide chain shown in SEQ ID NO: 14, or sequences substantially identical (e.g., having at least 80%, 85%, 90%, 92%, 95%, 97%, 98%, 99% or higher identity) to any one of the sequences.

In another embodiment, the triple-chain anti-LAG-3/PD-L1 bispecific antibody of the present invention comprises a first polypeptide chain shown in SEQ ID NO: 29, a second polypeptide chain shown in SEQ ID NO: 34, and a third polypeptide chain shown in SEQ ID NO: 22, or sequences substantially identical (e.g., having at least 80%, 85%, 90%, 92%, 95%, 97%, 98%, 99% or higher identity) to any one of the sequences.

In a second aspect, the present invention provides a polynucleotide encoding the first polypeptide chain, the second polypeptide chain, and/or the third polypeptide chain of the triple-chain antibody of the present invention.

In a third aspect, the present invention provides a vector, preferably an expression vector, comprising the polynucleotide encoding the first polypeptide chain, the second polypeptide chain, and/or the third polypeptide chain of the triple-chain antibody of the present invention.

In a fourth aspect, the present invention provides a host cell comprising the polynucleotide or the vector of the present invention. For example, the host cell is a mammalian cell, preferably a CHO cell or a HEK293 cell; the host cell is a prokaryotic cell, preferably an E. coli cell.

In a fifth aspect, the present invention provides a method for producing the triple-chain antibody of the present invention, comprising: (i) cultivating the host cell of the present invention under conditions suitable for expression of the triple-chain antibody, and (ii) recovering the triple-chain antibody from the host cell or the cultures.

In a sixth aspect, the present invention provides an immunoconjugate comprising the triple-chain antibody of the present invention, and a pharmaceutical composition comprising the triple-chain antibody or the immunoconjugate thereof. The triple-chain antibody disclosed herein can be used alone or in combination with other drugs or other treatment modalities to treat, prevent and/or diagnose diseases such as autoimmune diseases, acute and chronic inflammatory diseases, infectious diseases (e.g., chronic infectious diseases or sepsis), and tumors, etc.

In a seventh aspect, the present invention provides use of the triple-chain antibody, the immunoconjugate, and the pharmaceutical composition of the present invention, as a medication for treating and/or preventing a disease in an individual or as a diagnostic tool for a disease, or to increase implantation of hematopoietic stem cells in a subject in need. Preferably, the individual is a mammal, and more preferably a human. In one embodiment, the disease is an autoimmune disease, an acute or chronic inflammatory disease, an infectious disease (e.g., a chronic infectious disease or sepsis), or a tumor.

Unless defined otherwise, all technical and scientific terms used herein have the same meaning as commonly understood by those of ordinary skill in the art to which the present invention belongs. All publications, patent applications, patents, and other references mentioned herein are incorporated by reference in their entireties. In addition, the materials, methods, and examples described herein are illustrative only and are not intended to be limiting. Other features, objectives, and advantages of the present invention will be apparent from the description and drawings, and from the appended claims.

### Brief Description of the Drawings

The preferred embodiments of the present invention described in detail below will be better understood when read in conjunction with the following drawings. For the purpose of illustrating the present invention, currently preferred embodiments are shown in the drawings. However, it should be understood that the present invention is not limited to the precise arrangements and instrumentalities of the embodiments shown in the drawings.

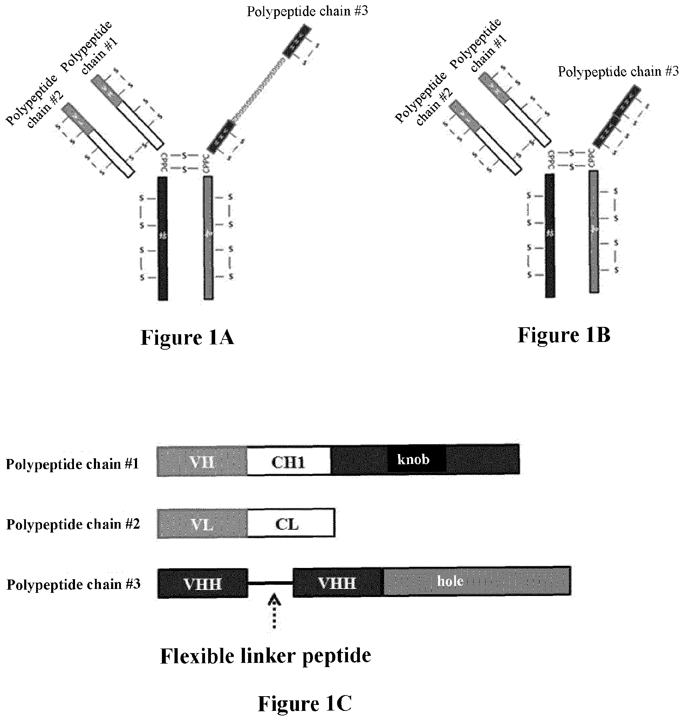

Figs. 1A-1C illustrate the structure of the triple-chain antibody of the present invention. Fig. 1A illustrates one triple-chain antibody of the present invention, wherein a first polypeptide chain is paired with a second polypeptide chain to form a first antigen-binding site, and a third polypeptide chain comprises a second single-domain antigen binding site and a third single-domain antigen binding site, and the second single-domain antigen binding site and the third single-domain antigen binding site of the third polypeptide chain have a flexible linker peptide therebetween. Fig. 1B illustrates another triple-chain antibody of the present invention, wherein a first polypeptide chain is paired with a second polypeptide chain to form a first antigen-binding site, and a third polypeptide chain comprises a second single-domain antigen binding site and a third single-domain antigen binding site, and the second single-domain antigen binding site and the third single-domain antigen binding site of the third polypeptide chain have no flexible linker peptide therebetween. Fig. 1C illustrates the structure of each peptide chain of the triple-chain antibody of the present invention from N-terminus to C-terminus, wherein the flexible linker peptide in the third polypeptide chain indicated by the dashed arrow may be either present or absent in the triple-chain antibody of the present invention as required (e.g., according to the affinity and steric hindrance of the epitope bound specifically).
Figs. 2A and 2B respectively show the purity of two anti-CD47/PD-L1 bispecific antibodies Kh2NF-PC and Kh2NF-PC-NL prepared according to the present invention and measured by size exclusion chromatography (SEC).
Fig. 3 shows the kinetic binding curve, and data of kₒₙ, k_{dis} and K_{D} of anti-CD47/PD-L1 bispecific antibodies Kh2NF-PC and Kh2NF-PC-NL of the present invention and an anti-PD-L1 humanized Nb-Fc antibody and anti-CD47 antibody ADI 29341 as controls, obtained by a kinetic binding assay.
Fig. 4A shows the binding of anti-CD47/PD-L1 bispecific antibodies Kh2NF-PC and Kh2NF-PC-NL, and an anti-PD-L1 humanized Nb-Fc antibody as control to CHO-S cells overexpressing PD-L1, measured by FACS. Fig. 4B shows the binding of anti-CD47/PD-L1 bispecific antibodies Kh2NF-PC and Kh2NF-PC-NL, an anti-CD47 antibody ADI 29341 as a positive control, and an IgG1 antibody as a negitive control to CHO-S cells overexpressing CD47, measured by FACS. The horizontal axis represents the antibody concentration, and the vertical axis represents the mean fluorescence intensity (MFI).
Fig. 5 shows the physical stability and solubility of anti-CD47/PD-L1 bispecific antibodies Kh2NF-PC and Kh2NF-PC-NL, and an antibody Humira (also known as adalimumab, is a humanized monoclonal antibody against TNFα) as positive control, measured by PEG precipitation.
Fig. 6 shows the long-term thermal stability of anti-CD47/PD-L1 bispecific antibodies Kh2NF-PC and Kh2NF-PC-NL. The purity was measured by SEC-HPLC after the antibodies were sit still at 40 °C for 0, 1, 3, 7, 10, 20, and 30 days.
Fig. 7A shows the binding of anti-CD47/PD-L1 bispecific antibodies Kh2NF-PC and Kh2NF-PC-NL to CHO-S cells overexpressing PD-L1 on day 0 and after incubation at 40 °C for 30 days. Fig. 7B shows the binding of anti-CD47/PD-L1 bispecific antibodies Kh2NF-PC and Kh2NF-PC-NL to CHO-S cells overexpressing CD47 on day 0 and after incubation at 40 °C for 30 days. The horizontal axis represents the antibody concentration, and the vertical axis represents the mean fluorescence intensity (MFI).
Fig. 8 shows the effect of anti-CD47/PD-L1 bispecific antibodies Kh2NF-PC and Kh2NF-PC-NL of the present invention, and an anti-PD-L1 humanized Nb-Fc antibody as positive control, and an IgG1 antibody as negative control on the PD-1/PD-L1 signaling pathway, measured by an MOA method.
Fig. 9 shows the ability of anti-CD47/PD-L1 bispecific antibodies Kh2NF-PC and Kh2NF-PC-NL, and an anti-CD47 antibody ADI 29341 as positive control to promote the phagocytosis of tumor cells by macrophages.
Fig. 10 shows the effect of an anti-CD47/PD-L1 bispecific antibody Kh2NF-PC, an anti-CD47 antibody Hu5F9 (corresponding to the "5F9" antibody in US2015/0183874 A1), and an IgG1 control antibody on erythrocyte agglutination.
Fig. 11 shows the ratio of anti-CD47/PD-L1 bispecific antibodies Kh2NF-PC to Kh2NF-PC-NL that selectively bind to the surface of tumor cells when tumor cells and human red blood cells are co-incubated.
Fig. 12 shows the purity of an anti-4-1BB/PD-L1 bispecific antibody Kh2NF-P4 prepared according to the present invention, measured by SEC.
Fig. 13A shows the binding of an anti-4-1BB/PD-L1 bispecific antibody Kh2NF-P4, and an anti-4-1BB antibody BMS-663513 as positive control to CHO-S cells overexpressing 4-1BB, measured by FACS. Fig. 13B shows the binding of an anti-4-1BB/PD-L1 bispecific antibody Kh2NF-P4 and an anti-PD-L1 humanized Nb-Fc antibody as positive control to CHO-S cells overexpressing PD-L1, measured by FACS. The horizontal axis represents the antibody concentration, and the vertical axis represents the mean fluorescence intensity (MFI).
Fig. 14 shows the purity of an anti-LAG-3/PD-L1 bispecific antibody Kh2NF-PL prepared according to the present invention, measured by SEC.
Fig. 15A shows the binding of an anti-LAG-3/PD-L1 bispecific antibody Kh2NF-PL, and an anti-PD-L1 humanized Nb-Fc antibody as positive control to CHO-S cells overexpressing PD-L1, measured by FACS. Fig. 15B shows the binding of an anti-LAG-3/PD-L1 bispecific antibody Kh2NF-PL, and an anti-LAG-3 antibody ADI-31853 as positive control to HEK293 cells overexpressing LAG-3, measured by FACS. The horizontal axis represents the antibody concentration, and the vertical axis represents the mean fluorescence intensity (MFI).
Fig 16 shows the effect of an anti-LAG-3/PD-L1 bispecific antibody Kh2NF-PL on activating T cells in vitro, compared with an anti-LAG-3 antibody ADI-31853, an anti-PD-L1 humanized Nb-Fc antibody, an anti-LAG-3 antibody ADI-31853 combined with anti-PD-L1 humanized Nb-Fc antibody, and an IgG4 control antibody.
Fig. 17 shows the tumor suppressive activities of an anti-PD-L1 humanized Nb-Fc antibody, an anti-CD47 antibody ADI 29341, an anti-PD-L1 humanized Nb-Fc antibody combined with an anti-CD47 antibody ADI 29341, and an anti-CD47/PD-L1 bispecific antibody Kh2NF- PC in a Raji-PD-L1/NOD-SCID mouse model, compared with an h-IgG.

### Detailed Description

### I. Definition

The term "about" used in combination with a numerical value is intended to encompass the numerical values in a range from a lower limit less than the specified numerical value by 5% to an upper limit greater than the specified numerical value by 5%.

As used herein, the term "contain" or "comprise" is intended to mean that the elements, integers or steps are included, but not to the exclusion of any other elements, integers or steps.

The term "antibody" is used herein in the broadest sense, refers to a protein comprising an antigen binding site, and covers natural and artificial antibodies of various structures, including but not limited to monoclonal antibodies, polyclonal antibodies, multispecific antibodies (e.g., bispecific antibodies), single-chain antibodies, triple-chain antibodies, intact antibodies, and antibody fragments.

The terms "whole antibody", "full-length antibody", "complete antibody" and "intact antibody" are used interchangeably herein to refer to a glycoprotein comprising at least two heavy chains (H) and two light chains (L) interconnected by disulfide bonds. Each heavy chain consists of a heavy-chain variable region (abbreviated herein as VH) and a heavy-chain constant region. The heavy-chain constant region consists of 3 domains (CH1, CH2 and CH3). Each light chain consists of a light-chain variable region (abbreviated herein as VL) and a light-chain constant region. The light-chain constant region consists of a domain CL. The VH region and the VL region can be further divided into hypervariable regions (complementarity determining regions, or CDRs), with more conservative regions (framework regions, or FRs) inserted therebetween. Each VH or VL consists of three CDRs and four FRs, arranged from the N-terminus to C-terminus in the following order: FR1, CDR1, FR2, CDR2, FR3, CDR3, FR4. The constant regions are not directly involved in binding of antibodies to antigens, but exhibit a variety of effector functions.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact point"). CDRs are primarily responsible for binding to epitopes. The CDRs of the heavy and light chains are generally referred to as CDR1, CDR2, and CDR3, which are numbered sequentially from the N-terminus. The CDRs located in the variable domain of an antibody heavy chain are referred to as VH CDR1, VH CDR2 and VH CDR3, while the CDRs located in the variable domain of an antibody light chain are referred to as VL CDR1, VL CDR2, and VL CDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature 342:877-883; Al-Lazikani et al., "Standard conformations for the canonical structures of immunoglobulins", Journal of Molecular Biology, 273:927-948 (1997)), Kabat based on antibody sequence variability (Kabat et al., Sequences of Proteins of Immunological Interest, 4th edition, US Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

However, it should be noted that boundaries of CDRs of variable regions of an antibody based on different assignment systems may differ. That is, CDR sequences of variable regions of an antibody defined by different assignment systems differ. Therefore, when it comes to defining an antibody with specific CDR sequences defined in the invention, the scope of antibody also encompasses such antibodies whose variable region sequences comprise the specific CDR sequences, but having claimed CDR boundaries different from the specific CDR boundaries defined by the invention due to a different protocol (e.g., different assignment system rules or their combinations) applied.

Antibodies with different specificities (i.e., different binding sites for different antigens) have different CDRs. However, although CDRs differ from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues of the rest CDR sequences can be determined via antibody structure and protein folding. Therefore, any variants of the CDRs given herein will also be considered in the invention. For example, in a CDR variant, the amino acid residues in the minimal binding unit may remain unchanged, while the other CDR residues defined by Kabat or Chothia may be substituted by conservative amino acid residues.

The term "antigen binding fragment" is a portion or segment of an intact or a complete antibody that has fewer amino acid residues than an intact or a complete antibody, which can bind an antigen or compete with an intact antibody (i.e., an intact antibody from which the antigen binding fragment is derived) for binding an antigen. An antigen binding fragment may be prepared by recombinant DNA techniques, or by enzymatic or chemical cleavage of an intact antibody. Antigen binding fragment includes, but is not limited to, Fab, Fab', F(ab')₂, Fv, single-chain Fv, diabody, and single-domain antibody (sdAb). The Fab fragment is a monovalent fragment consisting of VL, VH, CL and CHI domains, for example, Fab fragment can be obtained by papain digestion of a complete antibody. In addition, the F(ab')₂, a dimer of Fab', is a bivalent antibody fragment produced by pepsin digestion of the portion below disulfide bonds in the hinge region of a complete antibody. F(ab')₂ can be reduced by disrupting the disulfide bond in the hinge region under neutral conditions and the F(ab')₂ dimer is thus converted into Fab' monomers. An Fab' monomer is substantially an Fab fragment with hinge region (for more detailed descriptions of the antibody fragment, see: Fundamental Immunology, edited by W. E. Paul, Raven Press, N.Y., (1993)). The Fv fragment consists of the VL and VH domains of a single arm of an antibody. In addition, although the two domains VL and VH of the Fv fragment are encoded by separate genes, using a recombinant method, they can be linked by a synthetic linker peptide that enables these two domains to be produced as a single protein chain, the VL region and the VH region in the single protein chain being paired to form a single-chain Fv. The antibody fragment can be obtained by a chemical process, a recombinant DNA process, or a protease digestion process.

The term "single-domain antibody" (sdAb) or "single variable domain (SVD) antibody" generally refers to an antibody in which a single variable domain (e.g., a heavy chain variable domain (VH) or a light chain variable domain (VL), such as a heavy chain variable domain derived from a camelid heavy chain antibody, and a VH-like single domain (v-NAR) derived from fish IgNAR) can impart antigen binding. That is, the single variable domain requires no interaction with the other variable domain to recognize the target antigen. Examples of single domain antibodies include single domain antibodies derived from camelids (laminoids and camel) and cartilaginous fishes (e.g., nurse sharks) (WO2005/035572).

The term "camelized human VH domain" refers to transferring key elements derived from the camelidae VHH to a human VH domain, causing that the human VH domain no longer needs to match a VL domain to recognize the target antigen, the camelized human VH domain alone imparting antigen binding specificity.

The term "binding site" or "antigen binding site" as used herein refers to a region in an antibody molecule that actually binds an antigen, which includes VH/VL pairs consisting of an antibody light chain variable domain (VL) and an antibody heavy chain variable domain (VH), heavy chain variable domains derived from camelidae heavy chain antibodies, VH-like single domains of IgNAR (v-NAR) from sharks, camelized human VH domains, humanized camelidae antibody heavy chain variable domains. In embodiments of the present invention, the triple-chain antibody of the present invention comprises at least three antigen binding sites, for example, including a first antigen binding site (also referred to as a "first antigen binding site"), a second antigen binding site (also referred to as a "second antigen binding site"), and a third antigen binding site (also referred to as a "third antigen binding site").

The term "single-domain antigen binding site" refers to a region of an antibody molecule that actually binds an antigen with a single variable domain (e.g., a heavy chain variable domain (VH), a light chain variable domain (VL), a heavy chain variable domain derived from a camelidae heavy chain antibody, a v-NAR of IgNAR from sharks, a camelized human VH domain, a humanized camelidae antibody heavy chain variable domains, and recombinant single domains thereof). In one embodiment, the triple-chain antibody of the present invention comprises two single-domain antigen binding sites, referred to as "second single-domain antigen binding site" and "third single-domain antigen binding site", respectively.

As used herein, the term "monospecific" antibody refers to an antibody having one or more binding sites, each of which binds the same epitope of the same antigen.

As used herein, the term "multispecific" antibody refers to an antibody having at least two antigen binding sites, each of which binds a different epitope of the same antigen or a different epitope of a different antigen. The antibodies provided herein are generally multispecific antibodies, such as bispecific antibodies. A multispecific antibody is an antibody having binding specificities for at least two different epitopes. In one embodiment, provided herein is a bispecific antibody having binding specificities for a first antigen and a second antigen.

The term "immunoglobulin molecule" refers to a protein having a structure of a naturally occurring antibody. For example, an IgG-like immunoglobulin is a heterotetrameric glycoprotein of about 150,000 Daltons consisting of two light chains and two heavy chains which are disulfide-bonded. Each immunoglobulin heavy chain has a heavy chain variable region (VH), also called a heavy chain variable domain, followed by three heavy chain constant domains (CH1, CH2, and CH3) from N-terminus to C-terminus. Similarly, each immunoglobulin light chain has a light chain variable region (VL), also called a light chain variable domain, followed by a light chain constant domain (CL) from N-terminus to C-terminus. The heavy chains of a immunoglobulin can be assigned to one of five classes, α (IgA), δ (IgD), ε (IgE), γ (IgG), or µ (IgM), in which some classes can be further divided into subclasses such as γ1 (IgG₁), γ₂ (IgG₂), γ₃ (IgG3), γ₄ (IgG₄), α₁ (IgA₁), and α₂ (IgA₂). The light chain of immunoglobulin can be divided into one of two categories, referred to as kappa and lambda, based on the amino acid sequence of its constant domain. An immunoglobulin consists essentially of two Fab molecules and an Fc domain connected by an immunoglobulin hinge region.

The term "Fc domain" or "Fc region" is used herein to define a C-terminal region of an immunoglobulin heavy chain comprising at least a portion of the constant region. The term includes Fc regions and variant Fc regions of native sequences. A native immunoglobulin "Fc domain" comprises two or three constant domains, namely a CH2 domain, a CH3 domain, and an optional CH4 domain. For example, in native antibodies, an immunoglobulin Fc domain comprises the second and the third constant domains (CH2 domain and CH3 domain) derived from the two heavy chains of IgG, IgA, and IgD antibodies; or comprises the second, the third and the fourth constant domains (CH2 domain, CH3 domain and CH4 domain) derived from the two heavy chains of IgM and IgE antibodies. Unless otherwise stated, amino acid residues in Fc regions or heavy chain constant regions are numbered according to the EU numbering system (also known as the EU Index) described in, for example, Kabat et al., Sequences of Proteins of Immunological Interes, 5th Edition, Public Health Service, National Institutes of Health, Bethesda, MD, 1991.

The term "effector function" refers to biological activities attributed to an immunoglobulin Fc region that vary with immunoglobulin isotype. Examples of immunoglobulin effector function include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down regulation of cell surface receptors (such as B-cell receptors), and B-cell activation.

The term "chimeric antibody" is an antibody molecule in which: (a) a constant region or a portion thereof is modified, substituted, or exchanged such that antigen binding sites are linked to constant regions of different or modified classes, effector functions, and/or species, or disparate molecules imparting new properties (e.g., enzymes, toxins, hormones, growth factors, drugs) to chimeric antibodies, etc.; or (b) a constant region or a portion thereof is modified, substituted, or exchanged with a variable region of different or modified antigen binding specificities. For example, a mouse antibody can be modified by replacing its constant region with a constant region from a human immunoglobulin. Due to the replacement with a human constant region, the chimeric antibody can retain its specificity for recognizing antigen, while habing reduced antigenicity in human as compared to the original mouse antibody.

A "humanized antibody" is an antibody that retains the antigen-specific reactivity of a non-human antibody (such as a mouse monoclonal antibody) and has lower immunogenicity when administered to humans as a therapeutic agent. This can be achieved, for example, by retaining non-human antigen binding sites and replacing the remainder of the antibodies with their human counterparts (i.e., the constant and variable regions not involved in binding are the corresponding parts of human antibodies). See, e.g., Padlan, Anatomy of the antibody molecule, Mol. Immun., 1994, 31:169-217. Other examples of human antibody engineering techniques include, but are not limited to, the Xoma technology disclosed in US5,766,886.

The term "...valent" antibody refers to the number of antigen binding sites present in an antibody molecule. "Bivalent", "trivalent", and "tetravalent" antibodies refer to the presence of 2 antigen binding sites, 3 binding sites, and 4 binding sites in an antibody molecule, respectively. In one embodiment, the bispecific antibodies reported herein are "trivalent".

The term "flexible linker peptide" or "linker peptide" refers to a peptide composed of amino acids, such as glycine and/or serine residues used alone or in combination, to link various variable domains in an antibody. In one embodiment, the flexible linker peptide is a Gly/Ser linker comprising an amino acid sequence (Gly₄Ser)n, where n is a positive integer equal to or greater than 1, for example, a positive integer from 1 to 7. In one embodiment, the flexible linker peptide is (Gly₄Ser)4 (SEQ ID NO: 20). Also included within the scope of the invention are the linker peptides described in WO2012/138475, which is incorporated herein by reference.

As used herein, the term "binding" or "specific binding" means that the binding effect is selective for antigen and can be distinguished from unwanted or non-specific interactions. The ability of an antigen binding site to bind a particular antigen can be determined by an enzyme-linked immunosorbent assay (ELISA) or a conventional binding assay known in the art.

"Affinity" or "binding affinity" refers to inherent binding affinity that reflects interactions between members of a binding pair. Affinity of molecule X for its partner Y can be generally represented by the dissociation constant (K_{D}), which is the ratio of dissociation rate constant to association rate constant (k_{dis} and kₒₙ, respectively). Affinity can be measured by common methods known in the art. One specific method for measuring affinity is the ForteBio kinetic binding assay described herein.

The term "antigen" refers to a molecule that induce an immune response. Such immune response may involve antibody production or activation of specific immune cells, or both. Those skilled will understand that any macromolecules, including essentially all proteins or peptides, can be used as an antigen. In addition, an antigen may be derived from recombinant or genomic DNA. In some embodiments described herein, a first antigen, a second antigen, and a third antigen are three different antigens.

The terms "tumor-associated antigen" and "cancer antigen" can be used interchangeably and refer to molecules (generally proteins, carbohydrates, or lipids) preferably expressed completely or as fragments (e.g., MHC/peptide) on the surface of cancer cells, as compared to normal cells, and the molecules can be used in the preferential targeting of cancer cells by the agent. In some embodiments, the tumor-associated antigen is a cell surface molecule overexpressed in tumor cells compared to normal cells, such as 1-fold overexpression, 2-fold overexpression, 3-fold overexpression, or more fold overexpression compared to normal cells. In some embodiments, the tumor-associated antigen is a cell surface molecule inappropriately synthesized in tumor cells, such as a molecule comprising deletions, additions or mutations compared to molecules expressed on normal cells. In some embodiments, the tumor-associated antigen is expressed completely or as fragments only on the surface of tumor cells, and is not synthesized or expressed on the surface of normal cells. Many tumor-associated antigens are disclosed in the prior art, such as epidermal growth factor receptor variant III (EGFRvIII), tumor-associated glycoprotein 72 (TAG72), carcinoembryonic antigen (CEA), epithelial cell adhesion molecule (EPCAM), interleukin 11 receptor alpha (IL-11Ra), vascular endothelial growth factor receptor 2 (VEGFR2), epidermal growth factor receptor (EGFR), nerve cell adhesion molecule (NCAM), insulin-like growth factor 1 receptor (IGF-I receptor), melanoma-associated antigen 1 (MAGE-A1), CD72, CD47, and the like.

The term "immune checkpoint" means a class of inhibitory signaling molecules that are present in the immune system, to avoid tissue damage by regulating the persistence and strength of the immune response in peripheral tissues, and to participate in maintaining the tolerance to self antigens (Pardoll DM., The Blockade of Immune Checkpoints in Cancer Immunotherapy, Nat Rev Cancer, 2012, 12 (4): 252-264). Studies have found that one of the reasons that tumor cells can escape the immune system in the body causing uncontrolled proliferations is that they take use of the inhibitory signaling pathway of immune checkpoints, thereby suppressing T lymphocyte activity, disabling T lymphocytes' killing effect on tumors (Yao S, Zhu Y, and Chen L., Advances in Targeting Cell Surface Signaling Molecules for Immune Modulation. Nat Rev Drug Discov, 2013, 12 (2): 130-146). Immune checkpoint molecules include, but are not limited to, programmed death receptor 1 (PD-1), PD-L1, PD-L2, cytotoxic T lymphocyte antigen 4 (CTLA-4), and LAG-3, which directly suppress immune cells. Immune checkpoint molecules, such as PD-L1 and LAG-3, can regulate (e.g., coregulate) T cell functions to promote tumor immune escape.

The term "co-stimulatory molecule" refers to a corresponding binding partner on a T cell that specifically binds to a co-stimulatory ligand to mediate a T-cell co-stimulatory response, such as, but not limited to, proliferation. Co-stimulatory molecules are cell surface molecules other than antigen receptors or their ligands that contribute to an effective immune response. Co-stimulatory molecules include, but are not limited to, MHC class I molecules, TNF receptors, immunoglobulin-like proteins, cytokine receptors, integrins, signaling lymphocyte activation molecules (SLAM proteins), activated NK cell receptors, OX40, CD40, 4-1BB (i.e., CD137), CD27, and CD28. In some embodiments, the "co-stimulatory molecule" is 4-1BB (i.e., CD137), CD27, and/or CD28.

The term "cytokine" is a general term for proteins that are released by a cell population and act as intercellular mediators on another cell. Examples of such cytokines are lymphokines; monokines; interleukins (IL), such as IL-1, IL-1α, IL-2, IL-3, IL-4, IL-5, IL-6, IL-7, IL-8, IL-9, IL-11, IL-12, and IL-15; tumor necrosis factor, such as TNF-α or TNF-β; and other polypeptide factors, including LIF and kit ligand (KL) and γ-interferon. As used herein, the term cytokine includes proteins from natural sources or from recombinant cell cultures and biologically active equivalents of cytokines of native sequence, including small molecule entities produced by artificial synthesis, and pharmacologically acceptable derivatives and salts thereof.

An "immunoconjugate" is an antibody conjugated to one or more heterologous molecule(s), including but not limited to cytotoxic agents.

As used herein, the term "cytotoxic agent" refers to a substance that inhibits or prevents cell function and/or causes cell death or destruction. Cytotoxic agents include, but are not limited to, radioisotopes (e.g., At²¹¹, I¹³¹, I¹²⁵, Y⁹⁰, Re¹⁸⁶, Re¹⁸⁸, Sm¹⁵³, Bi²¹², P³², Pb²¹², and radioisotopes of Lu); chemotherapeutics (e.g., methotrexate, doxorubicin, vinca alkaloids (vincristine, vinblastine, etoposide), doxorubicin, melphalan, mitomycin C, chlorambucil, zorubicin, or other intercalators); growth inhibitors; enzymes and fragments thereof such as nucleolytic enzymes; antibiotics; toxins such as small molecule toxins or enzymatic toxins of bacterial, fungal, plant or animal source, including fragments and/or variants thereof; and various antitumor agents or anticancer agents disclosed below.

The "percent identity (%)" of an amino acid sequence refers to the percentage of amino acid residues in the candidate sequence that are the same as those of a specific amino acid sequence shown in this specification when aligning the candidate sequence with the specific amino acid sequence shown in this specification, with gaps introduced if necessary to achieve maximum percent sequence identity and without considering any conservative substitutions as part of sequence identity.

For polypeptide sequences, "conservative modifications" include substitutions, deletions, or additions to a polypeptide sequences that result in replacement of a certain amino acid with a chemically similar amino acid. Conservative substitution tables that provide functionally similar amino acids are well known in the art. Such conservatively modified variants are additional to polymorphic variants, interspecies homologs, and alleles of the present invention and do not exclude them. The following 8 groups comprises amino acids that are conservatively substituted with each other: 1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M) (see, for example, Creighton, Proteins (1984)). In some embodiments, the term "conservative sequence modification" refers to an amino acid modification that does not significantly affect or alter the binding characteristics of an antibody comprising the amino acid sequence.

The term "N-terminus" refers to the amino acid at the N-terminus, and the term "C-terminus" refers to the amino acid at the C-terminus.

The term "host cell" refers to a cell into which an exogenous polynucleotide has been introduced, including progeny of such cells. Host cells include "transformants" and "transformed cells", which include primary transformed cells and progeny derived therefrom. A host cell can be any cellular system that is used to produce a triple-chain antibody of the present invention. Host cells include cells in culture, as well as cells within a transgenic animal, a transgenic plant, or a cultured plant tissue or an animal tissue.

The term "expression vector" refers to a vector comprising a recombinant polynucleotide, which comprises an expression control sequence operably linked to a nucleotide sequence to be expressed. The expression vector contains sufficient cis-regulatory elements for expression; additional elements for expression can be provided by a host cell or in an in-vitro expression system. The expression vectors include all those known in the art, including cosmids, plasmids (e.g., naked or contained in liposomes), and viruses (e.g., lentiviruses, retroviruses, adenoviruses and adeno-associated viruses) incorporated into recombinant polynucleotides.

The terms "individual" and "subject" can be used interchangeably and refer to a mammal. The mammals include, but are not limited to, domesticated animals (e.g., cows, sheep, cats, dogs, and horses), primates (e.g., human and non-human primates such as monkeys), rabbits and rodents (e.g., mice and rats). In particular, individuals are humans.

The term "treatment" or "therapy" refers to a clinical intervention intended to alter the natural progress of the disease in an individual being treated. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and improving prognosis. In some embodiments, the triple-chain antibody of the present invention is used to delay disease progression or to slow disease progression.

The term "anti-tumor effect" refers to a biological effect that can be demonstrated by a variety of means, including but not limited to, for example, a decrease in tumor volume, a decrease in number of tumor cells, a decrease in tumor cell proliferation, or a decrease in tumor cell viability. The terms "tumor" and "cancer" are used interchangeably herein and encompass solid and liquid tumors.

The terms "cancer" and "cancerous" refer to or describe a physiological disease in mammals that is typically characterized by unregulated cell growth. Examples of cancers include, but are not limited to, carcinomas, lymphomas, blastomas, sarcomas, and leukemias or lymphoid malignancies. More specific examples of such cancers include, but are not limited to, squamous cell carcinoma (e.g., epithelial squamous cell carcinoma), lung cancer (including small cell lung cancer, non-small cell lung cancer, lung adenocarcinoma, and lung squamous cell carcinoma), peritoneal cancer, hepatocellular carcinoma, gastric cancer (including gastrointestinal cancer and gastrointestinal stromal cancer), pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial or uterine cancer, salivary adenocarcinoma, kidney cancer, prostate cancer, vulval cancer, thyroid cancer, liver cancer, anal cancer, penile cancer, melanoma, superficial diffuse melanoma, lentigo maligna melanoma, acral melanoma, nodular melanoma, multiple myeloma and B-cell lymphoma, chronic lymphocytic leukemia (CLL), acute lymphoblastic leukemia (ALL), hairy cell leukemia, chronic myelogenous leukemia, and post-transplant lymphoproliferative disorder (PTLD), as well as abnormal vascular proliferation associated with phakomatoses, edema (such as those associated with brain tumors), and Meigs syndrome, brain tumors and brain cancer, and head and neck cancer, and related metastases. In certain embodiments, cancers suitable for treatment with the antibody of the present invention include lung cancer (e.g., non-small cell lung cancer), liver cancer, gastric cancer, or colon cancer, including metastatic forms of such cancers.

The term "tumor" refers to all neoplastic cell growth and proliferation regardless of whether malignant or benign, and all pre-cancerous and cancerous cells and tissues. The terms "cancer", "cancerous" and "tumor" are not mutually exclusive when referred to herein.

The term "infectious disease" refers to a disease caused by a pathogen, and includes, for example, a viral infection, a bacterial infection, a parasitic infection, or a fungal infection.

### II. The triple-chain antibody of the present invention

The present invention provides a novel triple-chain antibody, which can be used for immunotherapy, prevention and/or diagnosis of various diseases. The triple-chain antibody of the present invention comprises at least 3 antigen binding sites, and can function as a monospecific antibody or a multispecific (e.g., bispecific) antibody, and preferably, function as a multispecific (e.g., bispecific) antibody.

When generating monospecific or multispecific (e.g., bispecific) antibodies with multiple polypeptide chains, issues such as undesired interchain mismatches, decreased antibody affinity, and decreased stability often occur. The triple-chain antibody of the invention can avoid such common issues.

The triple-chain antibody platform constructed by the invention is a triple-chain antibody comprising three polypeptide chains, including a first polypeptide chain comprising a first heavy chain variable domain, a second polypeptide chain comprising a first light chain variable domain that is paired with the first heavy chain variable domain (VH1/VL1 pair) to form a first antigen binding site; and a third polypeptide chain comprising a second single-domain antigen binding site and a third single-domain antigen binding site.

In one embodiment, there is no linker peptide between the second single-domain antigen binding site and the third single-domain antigen binding site in the third polypeptide chain of the triple-chain antibody of the present invention.

In another embodiment, there is a linker peptide between the second single domain antigen binding site and the third single domain antigen binding site in the third polypeptide chain of the triple-chain antibody of the present invention. The type of the linker peptide is not particularly limited. In one embodiment, the linker peptide is a peptide having an amino acid sequence of 1 to 100, particularly 1 to 50, more particularly 1 to 20 amino acids in length. In some embodiments, the linker peptide is (GxS)n or (GxS)nGm, where G = glycine, S = serine, x is any integer from 1 to 4, n is any integer from 1 to 7, and m is any integer from 0 to 3. In one specific embodiment, the linker peptide is (G₄S)₄ (SEQ ID: NO: 20).

The single-domain antigen binding sites in the third polypeptide chain of the triple-chain antibody of the present invention are single variable domains capable of specifically binding a target antigen epitope with a higher affinity, for example, a heavy-chain variable domain (VH), a light-chain variable domain (VL), a heavy-chain variable domain derived from a camelidae heavy-chain antibody, a v-NAR of IgNAR from sharks, a camelized human VH domain, a humanized camelidae antibody heavy-chain variable domains, and recombinant single domains thereof. In one embodiment, the two single-domain antigen binding sites in the third polypeptide chain of the triple-chain antibody of the present invention are heavy chain variable domains derived from a camelidae heavy-chain antibody, a camelized human VH domain and/or a humanized camelidae antibody heavy-chain variable domain.

The size, structure, and antigenicity for human subjects of antibodies obtained from camelidae species (such as camel, alpaca, dromedary, llama, and guanaco) have been characterized in the prior art. Certain natural IgG antibodies from the camelidae lack light chains and are therefore structurally different from common tetra-chain antibody structures with two heavy and two light chains in other animals. See PCT/EP 93/02214 (WO 94/04678 published on March 3, 1994).

A heavy chain variable domain (also referred to as VHH) of a camelidae heavy-chain antibody can be obtained by genetic engineering process. See U.S. Patent No. 5,759,808, issued on June 2, 1998. Similar with other non-human antibody fragments, the amino acid sequence of the camelidae VHH can be altered recombinantly to obtain a sequence that more closely mimics a human sequence, i.e., "humanized", thereby reducing the antigenicity of the camelidae VHH to humans. In addition, key elements derived from the camelidae VHH can also be transferred to the human VH domain to obtain a camelized human VH domain. In one embodiment of the present invention, the single-domain antigen binding site in the third polypeptide chain of the triple-chain antibody of the present invention is a humanized VHH, which has an amino acid sequence shown in SEQ ID NO: 16.

The VHH has a molecular weight that is one-tenth the molecular weight of human IgG molecule, and has a physical diameter of only a few nanometers. The VHH itself has extremely high thermal stability, stability to extreme pH and proteolytic digestion, and low antigenicity, and thus the structure may contribute to the stability of the triple-chain antibody of the present invention and the low antigenicity to human subjects.

In one embodiment, the first polypeptide chain of the triple-chain antibody of the present invention comprises a first heavy-chain variable domain and an immunoglobulin CHI domain, the second polypeptide chain comprises a first light-chain variable domain and an immunoglobulin CL domain; and the third polypeptide chain comprises a second single-domain antigen binding site and a third single-domain antigen binding site. The class, subclass, type, and subtype of the immunoglobulin from which the CHI domain and the CL domain are derived are not particularly limited. Preferably, the CHI domain and the CL domain are both derived from corresponding parts of human immunoglobulin or have substantially identical (for example, at least 80%, 85%, 90%, 92%, 95%, 97%, 98%, 99%, or more) sequence to the corresponding parts.

In one embodiment, the triple-chain antibody of the present invention further comprises a component having a prolonged half-life in vivo. Many factors may affect the half-life of the protein in vivo. For example, renal filtration, metabolism in the liver, degradation by proteolytic enzymes (proteases), and immunogenic reactions (e.g., protein neutralization of antibodies and uptake by macrophages and dendritic cells). A variety of strategies can be used to prolong the half-life of the triple-chain antibody of the present invention. For example, through chemically linked polyethylene glycol (pegylation), polysialytaion, hesylation, or conjugating an albumin or immunoglobulin Fc, etc.

To pegylate an antibody, the triple-chain antibody is generally reacted with polyethylene glycol (PEG) such as an active ester or aldehyde derivative of PEG under conditions in which one or more PEG groups become attached to the triple-chain antibody. Pegylation can be implemented through acylation or alkylation with a reactive PEG molecule (or a similar reactive water soluble polymer). As used herein, the term "polyethylene glycol" includes any form of PEGs that have been used to derivatize other proteins, such as mono (C₁-C₁₀) alkoxy or aryloxy-polyethylene glycol or polyethylene glycol-maleimide. In certain embodiments, the antibody to be pegylated is an aglycoslated antibody. Linear or branched PEG derivatization causing minimal loss of antibody biological activity is used. The degree of conjugation can be closely monitored by SDS-PAGE and mass spectrometry to ensure that the PEG molecule is properly conjugated to the antibody. Unreacted PEG can be separated from the antibody-PEG conjugate by size exclusion chromatography or by ion exchange chromatography. The binding activity of PEG-derived antibodies can be tested using methods well known to those skilled in the art. Methods of pegylating proteins are known in the art and can be applied to the antibody of the present invention. See, for example, EP 0154316 to Nishimura et al.

Polysialylation is another technique that uses a natural polymer polysialic acid (PSA) to prolong the effective life and improve the stability of therapeutic peptides and proteins. PSA is a polymer of sialic acid (a saccharide). Polysialic acid provides a protective microenvironment for the conjugate when used for delivering therapeutic protein and peptide. This increases the effective life of the therapeutic protein in the circulation and prevents it from being recognized by the immune system.

Hesylation can also prolong the circulating half-life of the antibody, resulting in increased biological activity. A wide variety of hesylated immunoconjugates can be prepared by varying different parameters, such as the molecular weight of HES.

It is also possible to introduce an IgG constant domain or an immunoglobulin Fc domain to the triple-chain antibody of the present invention to produce an antibody with increased half-life in vivo. See, for example, international publication No. WO98/23289; international publication No. WO97/34631; and US Patent No. 6277375.

Further, the triple-chain antibody of the present invention may be conjugated with an albumin (e.g., human serum albumin; HSA) such that the triple-chain antibody is more stable or have a longer half-life in vivo. Such techniques are well known in the art. See, for example, international publication No. WO 01/77137; and European patent No. EP 413,622.

In one embodiment, the triple-chain antibody of the present invention comprises an Fc region to prolong the half-life of the antibody of the present invention in vivo.

In another specific embodiment, the Fc domains of the first polypeptide chain and the third polypeptide chain of the triple-chain antibody of the present invention each comprises a hinge region having a "CPPC" amino acid sequence, and/or each comprises Y349C and S354C (according to the "EU numbering" of Kabat), whereby the first polypeptide chain and the third polypeptide chain form interchain disulfide bonds in the Fc region, which also promotes the correct pairing of the first polypeptide chain and the third polypeptide chain of the triple-chain antibody of the present invention.

In one embodiment, the triple-chain antibody of the present invention employs the "knob-in-hole" technology (see, e.g., John B.B Ridgway et al., 'Knobs-into-holes' engineering of antibody CH3 domains for heavy chain heterodimerization. Protein Engineering, 1996.9 (7): p. 617-21; Shane Atwell et. al., Stable heterodimers form remodeling the domain interface of a homodimer using a phage display library. J. Mol. Biol, 1997.270:p. 26-35), which modifies the interface between different chains of the triple-chain antibody of the present invention to promote the correct association of chains of the triple-chain antibody. Generally, this technique involves introducing a "protuberance" at the interface of one chain, and introducing a corresponding "cavity" at the interface of the other chain to be paired, such that the protuberance can be placed in the cavity. A first preferred interface comprises the CH3 domain of the heavy chain constant domain of one chain and the CH3 domain of the heavy chain constant domain of the other chain to be paired. The protuberance can be constructed by replacing small amino acid side chains from the interface of the CH3 domain of the heavy chain constant domain of the one chain with larger side chains, such as tyrosine or tryptophan. The compensating cavity with the same or similar size as the protuberance is constructed at the interface of the CH3 domain of the heavy chain constant domain of the other chain to be paired, by replacing large amino acid side chains with smaller side chains (such as alanine or threonine). A second preferred interface comprises the CL domain of the light chain and the CHI domain of the heavy chain, where a knob-in-hole interaction can be constructed as described above.

In one embodiment, the Fc region of the triple-chain antibody of the present invention comprises a binding affinity modification for the Fc receptor. In one embodiment, the Fc receptor is an Fcγ receptor, in particular a human Fcγ receptor. In one embodiment, the Fc receptor is an activating Fc receptor. In one embodiment, the modification reduces the effector function of the triple-chain antibody of the present invention. In one specific embodiment, the effector function is antibody-dependent cell-mediated cytotoxicity (ADCC). In one embodiment, the modification is in the Fc region of the immunoglobulin molecule, particularly in its CH2 region. In one embodiment, the immunoglobulin molecule comprises an amino acid substitution at position 329 (EU numbering) of a heavy chain. In one specific embodiment, the amino acid substitution is P329G. In one embodiment, the triple-chain antibody of the present invention comprises amino acid substitutions at positions 234 and 235 (EU numbering) of a heavy chain of immunoglobulin. In one specific embodiment, the amino acid substitutions are L234A and L235A (LALA mutations) (Armour KL et al., Recombinant human IgG molecules lacking Fcgamma receptor I binding and monocyte triggering activities, Eur. J. Immunol., 1999, 29(8):2613-24). In one embodiment, a triple-chain antibody of the present invention comprises amino acid substitutions at positions 234, 235, and 329 (EU numbering) of a heavy chain of immunoglobulin. In one specific embodiment, the immunoglobulin molecule comprises amino acid substitutions L234A, L235A, and P329G (EU numbering) in a heavy chain of immunoglobulin.

As shown in the schematic diagram of FIG. 1A, an exemplary triple-chain antibody of the present invention is a trivalent triple-chain antibody in which: a first heavy-chain variable domain in a first polypeptide chain and a first light-chain variable domain in a second polypeptide chain are paired to form a first antigen binding site; and a third polypeptide chain comprises a second single-domain antigen binding site and a third single-domain antigen binding site, and the second and third single-domain antigen binding sites have a linker peptide therebetween.

As shown in the schematic diagram of FIG. 1B, an exemplary triple-chain antibody of the present invention is a trivalent triple-chain antibody in which: a first heavy chain variable domain in a first polypeptide chain and a first light chain variable domain in a second polypeptide chain are paired to form a first antigen binding site; and a third polypeptide chain comprises a second single-domain antigen binding site and a third single-domain antigen binding site, and the second and third single-domain antigen binding sites have no linker peptide therebetween.
i) In one embodiment, the triple-chain antibody is an anti-CD47/PD-L1 bispecific antibody or a multispecific antibody.

PD-L1 (also known as cluster of differentiation 274 (CD274) or B7 homolog 1 (B7-H1)) is a 40-kDa type I transmembrane protein PD-L1 binds to its receptor PD-1 present on activated T cells and down-regulates T cell activation (Latchman et. al., 2001, Nat. Immunol., 2:261-8; Carter et. al., 2002, Eur. J. Immunol., 32:634-43). PD-L1 expression has been found in many cancers, including human lung cancer, ovarian cancer, colon cancer, and multiple myelomas, and PD-L1 expression is often associated with poor prognosis of cancers (Iwai et. al., (2002) PNAS, 99:12293-7; Ohigashi et. al., (2005) Clin. Cancer Res. 11:2947-53; Okazaki et. al., (2007) Intern. Immun., 19:813-24; Thompson et. al., (2006) Cancer Res., 66:3381-5). It has been proposed that immunosuppression can be reversed in a portion of tumor patients by suppressing the local interaction of PD1 and PD-L1.

The anti-PD-L1 antibodies atezolizumab developed by Roche, the anti-PD-L1 antibody avelumab developed by Merck KGaA and Pfizer, and durvalumab developed by AstraZeneca have shown their effect of treating some tumor patients. Other anti-PD-L1 antibodies include YW243.55.S70 (the heavy and light chain variable regions are shown in SEQ ID NOs 20 and 21 in WO2010/077634), anti-PD-L1 antibodies disclosed in WO2007/005874, and the like.

CD47 is first identified as an integrin-associated protein (IAP) (Brown E. J., et. al., Integrin-associated protein (CD47) and its ligands, Trends Cell Biol., 2001, 11(3): 130-135), and is a tumor-associated antigen expressed on cell surface. CD47 interacts with SIRPα, a cell surface immunoglobulin as its ligand mainly expressed by macrophages and dendritic cells, producing a series of cascade reactions, and thereby inhibiting the uptake and phagocytosis of cells expressing CD47 by macrophages and dendritic cells.

CD47 expression is ubiquitous in normal tissues. For example, CD47 is expressed on the surface of viable red blood cells. Part of CD47's function is to protect the viable red blood cells from phagocytosis (Oldenborg P.A. et. al., Role of CD47 as a marker of self on red blood cells, Science, 2000, 288(5473):2051-2054). In addition, CD47 overexpression has been observed in tumors. The expressed CD47 releases a "don't eat me" signal by binding to SIRPα on the surface of macrophages, thereby inhibiting the phagocytosis of tumor cells by macrophages in the tumor infiltrated area. Subsequently, the decrease in the uptake of tumor cells by antigen presenting cells (APCs) indirectly leads to a decrease in T cell activation. In addition, CD47 activation on naive T cells promotes the generation of immunosuppressive regulatory T cells (Tregs) and inhibits the generation of Thl effector cells (Avice MN et. al., CD47 ligation inhibit inhibits the development of human naive T cells into Th1 effectors, J. Immunol., October 2000, 165(8):4624-4631).

A number of anti-CD47 antibodies have been reported. For example, US patent US2015/0183874 A1 reports a chimeric human IgG1 monoclonal antibody derived from B6H12 and a humanized B6H12 antibody generated by CDR transplantation. Chinese patent application No. CN201710759828.9 reports a monoclonal antibody "ADI-29341" that can specifically bind to CD47.

The anti-CD47/PD-L1 bispecific or multispecific triple-chain antibody of the present invention simultaneously targets at least antibodies of CD47 and PD-L1, and three antigen binding sites bind to CD47 and/or PD-L1 molecules. As mentioned above, although CD47 is overexpressed on cancer cells, the expression of CD47 in many normal tissues leads to the non-specific binding of antibodies targeting CD47 only to normal circulating cells, which causes antigen sink. The anti-CD47/PD-L1 bispecific antibody of the present invention simultaneously targets CD47 and PD-L1 on tumor cells. The specific binding to PD-L1 on tumor cells promotes the selective binding of the anti-CD47/PD-L1 bispecific antibody of the invention to tumor cells, and avoids the binding to CD47 expressed in many normal tissues. Thus the anti-CD47/PD-L1 bispecific triple-chain antibody of the present invention reduces side effects while enhancing phagocytosis.

In one embodiment, the anti-CD47/PD-L1 bispecific triple-chain antibody of the present invention comprises a first antigen binding site comprising a VH1/VL1 pair on first and second polypeptide chains that specifically binds to PD-L1 or CD47, and a second single-domain antigen binding site that specifically binds to PD-L1 or CD47 and a third single-domain antigen binding site that specifically binds to PD-L1 or CD47 on a third polypeptide chain. In one embodiment, the triple-chain antibody comprises a first antigen binding site specifically binding to CD47 and comprising a VH1/VL1 pair on the first polypeptide chain and the second polypeptide chain, and a first VHH and a second VHH specifically binding to PD-L1 on the third polypeptide chain. In another embodiment, the triple-chain antibody comprises a first antigen binding site specifically binding to PD-L1 and comprising a VH1/VL1 pair on the first polypeptide chain and the second polypeptide chain, and a first VHH and a second VHH specifically binding to CD47 on the third polypeptide chain.

For the first antigen binding site comprising a VH1/VL1 pair that specifically binds to PD-L1 or CD47, the "VH1/VL1 pair" comprises six CDRs derived from VH1/VL1 pairs of any anti-PD-L1 antibody reported in the prior art (e.g., the anti-PD-L1 antibody exemplified above) and anti-PD-L1 antibodies developed in the future, or sequences having one, two, three, four, or five amino acid changes (e.g., amino acid substitutions or deletions) from one or more of the six CDRs; or comprises six CDRs derived from VH1/VL1 pairs of any anti-CD47 antibody reported in the prior art (e.g., the anti-CD47 antibody exemplified above) and anti-CD47 antibodies developed in the future, or sequences having one, two, three, four, or five amino acid changes (e.g., amino acid substitutions or deletions) from one or more of the six CDRs.

The single-domain antigen binding sites that specifically bind to PD-L1 or CD47 include heavy-chain variable domains (VH) and light-chain variable domains (VL) specifically binding to PD-L1 or CD47, heavy chain variable domains of antibodies from camelidae serum that are naturally free of light chain and composed of only two heavy chains, VH-like single domains of IgNAR from sharks, camelized human VH domains, humanized camelidae antibody heavy-chain variable domains.

In one embodiment, the triple-chain anti-CD47/PD-L1 bispecific antibody of the present invention comprises a first antigen binding site comprising a VH1/VL1 pair on the first and second polypeptide chains that specifically bind to CD47, and a second and a third single-domain antigen binding sites specifically binding to PD-L1, wherein the second and third single-domain antigen binding sites specifically bind to PD-L1 bind to the same epitope or different epitopes on PD-L1.

In one embodiment, the first antigen binding site specifically binding to CD47 and comprising the VH1/VL1 pair on the first polypeptide chain and the second polypeptide chain, comprises a VH CDR1 shown in GSIEHYYWS (SEQ ID NO: 3), a VH CDR2 shown in YIYYSGSTNYNPSLKS (SEQ ID NO: 4), a VH CDR3 shown in ARGKTGSAA (SEQ ID NO: 5), a VL CDR1 shown in RASQGISRWLA (SEQ ID NO: 10), a VL CDR2 shown in AASSLQS (SEQ ID NO: 11), and a VL CDR3 shown in QQTVSFPIT (SEQ ID NO: 12) derived from anti-CD47 antibody ADI-29341, or sequences having one, two, three, four, or five amino acid changes (e.g., amino acid substitutions or deletions) from one or more of the six CDRs.

In one embodiment, the first antigen binding site specifically binding to CD47 and comprising a VH1/VL1 pair on the first polypeptide chain and the second polypeptide chain, comprises paired heavy-chain variable region/light-chain variable region sequences of SEQ ID NOs: 2/9 derived from the anti-CD47 antibody ADI-29341, or sequences having a sequence identity of at least 90%, 91%, 92%, 93%, 94%, 95%, 96%, 97%, 98%, 99% or higher with the paired heavy-chain variable region/light-chain variable region sequences.

In one embodiment, the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or sequences having one, two, three, four, or five amino acid changes (e.g., amino acid substitutions or deletions) from one or more of the three CDRs. In another embodiment, the second and the third single-domain antigen binding sites specifically binding to PD-L1 on the third polypeptide chain comprise an amino acid sequence shown in SEQ ID NO: 15 and/or SEQ ID NO: 16, or sequences substantially identical (e.g., having at least 80%, 85%, 90%, 92%, 95%, 97%, 98%, 99% or higher identity) thereto.

The type of the heavy chain constant region of the immunoglobulin in the first polypeptide chain and the third polypeptide chain in the triple-chain antibody of the present invention is not particularly limited, and is preferably the heavy chain constant region of the IgG1, IgG2, or IgG4 immunoglobulin, or a sequence that is substantially identical (e.g., at least 80%, 85%, 90%, 92%, 95%, 97%, 98%, 99% or higher identity) to it. More preferably, the heavy chain constant region is a heavy chain constant region of a human IgG1 immunoglobulin, or a sequence substantially identical (for example, at least 80%, 85%, 90%, 92%, 95%, 97%, 98%, 99%, or higher identity) thereto.

In one embodiment, the triple-chain anti-CD47/PD-L1 bispecific antibody of the present invention comprises a heavy chain constant region used in IgG4 (e.g., human IgG4). In yet another embodiment, a triple-chain anti-CD47/PD-L1 bispecific antibody of the present invention comprises a heavy chain constant region for IgG1 (e.g., human IgG1). For example, each Fc domain of the first polypeptide chain and the third polypeptide chain of the triple-chain antibody comprises a hinge region having a "CPPC" amino acid sequence, and/or each comprises Y349C and S354C (according to the "EU numbering" of Kabat), whereby the first polypeptide chain and the third polypeptide chain form interchain disulfide bonds in the Fc region and thus stabilize the correct pairing of the first polypeptide chain and the third polypeptide chain.

In one embodiment, the first polypeptide chain and/or the third polypeptide chain of the triple-chain antibody of the invention comprises amino acid mutations in the Fc domain which affects the function of the antibody effector. In a specific embodiment, the amino acid substitution is LALA mutation.

In yet another embodiment, the second polypeptide chain of the triple-chain anti-CD47/PD-L1 bispecific antibody of the present invention comprises a kappa light chain constant region or a lambda light chain constant region, for example, a human kappa light chain constant region or a human lambda light chain constant region. In one embodiment, the light chain constant region comprises an amino acid sequence shown in SEQ ID NO: 13, or a sequence substantially identical (e.g., having at least 80%, 85%, 90%, 92%, 95%, 97%, 98%, 99% or higher identity) thereto.

In one embodiment, Fc domains of the first polypeptide chain and the third polypeptide chain of the triple-chain anti-CD47/PD-L1 bispecific antibody of the present invention each comprise a stable association of "knob-in-hole". In one embodiment, the amino acid substitution T366W is contained in one of the first polypeptide chain and the third polypeptide chain, and the amino acid substitutions T366S, L368A, and Y407V (EU numbering) are contained in the other one of the first polypeptide chain and the third polypeptide chain. Thus, the protuberance in one chain can be placed in the cavity in the other chain, which promotes the correct pairing of the first polypeptide chain and the third polypeptide chain.

In one embodiment, the immunoglobulin CHI domain and CL domain of the first polypeptide chain and the second polypeptide chain of the anti-CD47/PD-L1 bispecific triple-chain antibody of the present invention respectively comprise a protuberance and a cavity, or vice versa, and the protuberance or cavity in the CHI domain can be placed at the cavity or protuberance in the CL domain, respectively, such that the first polypeptide chain and the second polypeptide chain also form a stable "knob-in-hole" association with each other.

In one embodiment, the triple-chain anti-CD47/PD-L1 bispecific antibody of the present invention comprises a first polypeptide chain shown in SEQ ID NO: 1, a second polypeptide chain shown in SEQ ID NO: 8, and a third polypeptide chain shown in SEQ ID NO: 14, or sequences substantially identical (e.g., having at least 80%, 85%, 90%, 92%, 95%, 97%, 98%, 99% or higher identity) to any one of the sequences.

In another embodiment, the triple-chain anti-CD47/PD-L1 bispecific antibody of the present invention comprises a first polypeptide chain shown in SEQ ID NO: 1, a second polypeptide chain shown in SEQ ID NO: 8, and a third polypeptide chain shown in SEQ ID NO: 22, or sequences substantially identical (e.g., having at least 80%, 85%, 90%, 92%, 95%, 97%, 98%, 99% or higher identity) to any one of the sequences.

The triple-chain anti-CD47/PD-L1 bispecific antibody of the present invention can simultaneously bind to PD-L1 and CD47 proteins, and maintains the affinity constant of the parent antibody, thereby being able to block the SIRPα/CD47 signaling pathway and the PD1/PD-L1 signaling pathway.

In some embodiments, the conditions associated with the SIRPα/CD47 signaling pathway and the PD1/PD-L1 signaling pathway are various blood diseases and solid tumors, including but not limited to, acute myeloid leukemia (AML), chronic myeloid leukemia, acute lymphoblastic leukemia (ALL), non-Hodgkin's lymphoma (NHL), multiple myeloma (MM), lymphoma, breast cancer, gastric cancer, lung cancer, esophageal cancer, bowel cancer, ovarian cancer, cervical cancer, kidney cancer, pancreatic cancer, bladder cancer, glioma, melanoma and other solid tumors. In addition, a human stem cell implantation in a NOD mouse line can be enhanced by blocking the SIRPα/CD47 signaling pathway (WO 2009/046541), thus, the triple-chain anti-CD47/PD-L1 bispecific antibody of the present invention also has potential benefits for use in human stem cell transplantation.

Chinese Patent No. ZL201080064426.3 discloses that a tetravalent CD47-antibody constant region fusion protein that blocks the SIRPα/CD47 signaling pathway is capable of treating, preventing or diagnosing autoimmune and inflammatory conditions mediated by SIRPα + cells, such as allergic asthma or ulcerative colitis. These conditions include acute and chronic inflammatory conditions, allergies and allergic diseases, autoimmune diseases, ischemic conditions, severe infections, and rejection of cell or tissue or organ grafts, including non-human tissue grafts (heterograft), therefore, the triple-chain anti-CD47/PD-L1 bispecific antibody of the present invention is expected to play a role in the treatment, prevention, or diagnosis of these diseases.
ii) In one embodiment, the triple-chain antibody is an anti 4-1BB/PD-L1 bispecific antibody or a multispecific antibody.

PD-L1 is an immune checkpoint suppressor molecule. PD-L1 expressed on tumor cells activates the PD-1/PD-L1 signaling pathway by binding to PD-1 on T lymphocytes, thereby inhibiting T lymphocyte activity, making T lymphocytes unable to effectively exert the killing effect on tumors, which is one of the reasons that tumor cells can escape the immune system in the body and cause uncontrolled proliferation (Yao S, Zhu Y and Chen L., Advances in targeting cell surface signaling molecules for immune modulation. Nat Rev Drug Discov, 2013, 12 (2): 130-146).

4-1BB (also known as CD137, TNFRSF9) is a costimulatory receptor that is induced by activation and expressed on activated T cells and natural killer (NK) cells, and is a member of the tumor necrosis factor receptor superfamily. 4-1BB has an amino acid sequence as provided by GenBank Accession No. AAA62478.2, or an equivalent amino acid sequence from a non-human species such as mouse, rodent, monkey, ape, and the like. 4-1BB ligation on T cells triggers a signal cascade that results in up regulation of anti-apoptotic molecules, cytokine secretion and enhanced effector function. In dysfunctional T cells with a reduced cytotoxicity ability, 4-1BB ligation shows effective ability to restore effector function (Li SY, Liu Y. Immunotherapy of melanoma with the immune costimulatory monoclonal antibodies targeting CD137. Clin Pharmacol, 2013, 5(Suppl 1): 47-53). On NK cells, 4-1BB signaling can increase antibody-dependent cell-mediated cytotoxicity.

An agonistic monoclonal antibody against 4-1BB has been developed to use 4-1BB signaling for cancer immunotherapy (Ye Q, Song DG, Poussin M, Yamamoto T, Best A, Li C, et al., CD137 accurately identifies and enriches for naturally occurring tumor-reactive T cells in tumor. Clin Cancer Res, 2014, 20 (1): 44-55). Preclinical results of multiple induced tumor models and spontaneous tumor models show that targeting 4-1BB with agonistic antibodies can lead to tumor clearance and durable anti-tumor immunity. WO2004010947A2 discloses a humanized anti-4-1BB monoclonal antibody that binds to human 4-1BB and allows human 4-1BB to bind to human 4-1BB ligand.

WO2016/061142 generally mention an administration of an anti-PD-L1 antibody molecule with a 4-1BB receptor targeting agent (e.g., an antibody that stimulates signaling via 4-1BB (CD-137), such as PF-2566).

Because in tumor immunity, the treatment schemes that target multiple targets can cooperate with each other, it is beneficial to prevent tumor immune escape, at present, co-administration of antibodies targeting different targets in tumor immunity has also entered clinical trials. However, co-administration requires an injection of two separate antibody products or a single injection of a combined preparation of two different antibodies. Although two injections allow flexibility in dosage and timing, it causes inconvenience and pain for patients. In addition, although the combined preparation may provide some flexibility in dosage, it is often difficult to find preparation conditions that allow the chemical and physical stability of two antibodies in solution because of the different molecular characteristics of the two antibodies. Moreover, therapies of co-administration and combined preparation of two different antibodies may increase the extra cost of the patient and/or payer, therefore, there is a need for alternative immunotherapy for the treatment of tumors, and preferably such alternative immunotherapy involves bispecific antibodies.

The anti-4-1BB/PD-L1 bispecific antibody or multispecific antibody of the present invention is an antibody that at least simultaneously targets 4-1BB and PD-L1, and the three antigen binding sites respectively bind to 4-1BB and/or PD-L1 molecules, can block the PD-1/PD-L1 signaling pathway and activate the 4-1BB/4-1BB ligand signaling pathway in T cells and natural killer (NK) cells. In one embodiment, the triple-chain antibody comprises a first antigen binding site comprising a VH1/VL1 pair on the first and second polypeptide chains that specifically binds to PD-L1 or 4-1BB, and a second single domain antigen binding site that specifically binds to PD-L1 or 4-1BB and a third single domain antigen binding site that specifically binds to PD-L1 or 4-1BB on the third polypeptide chain. In one embodiment, the triple-chain antibody comprises a first antigen binding site specifically binding to 4-1BB and comprising a VH1/VL1 pair on the first polypeptide chain and the second polypeptide chain, and a first VHH and a second VHH specifically binding to PD-L1 on the third polypeptide chain. In another embodiment, the triple-chain antibody comprises a first antigen binding site specifically binding to PD-L1 and comprising a VH1/VL1 pair on the first polypeptide chain and the second polypeptide chain, and a first VHH and a second VHH specifically binding to 4-1BB on the third polypeptide chain.

For the first antigen binding site comprising a VH1/VL1 pair that specifically binds to PD-L1 or 4-1BB, "VH1/VL1 pair" comprises six CDRs derived from VH1/VL1 pairs of any anti-PD-L1 antibody reported in the prior art (e.g., the anti-PD-L1 antibody exemplified above) and anti-PD-L1 antibodies developed in the future, or sequences having one, two, three, four, or five amino acid changes (e.g., amino acid substitutions or deletions) with one or more of the six CDRs; comprises six CDRs derived from VH1/VL1 pairs of any anti-4-1BB antibody reported in the prior art (e.g., the anti-4-1BB antibody exemplified above) and anti-4-1BB antibodies developed in the future, or sequences having one, two, three, four, or five amino acid changes (e.g., amino acid substitutions or deletions) with one or more of the six CDRs.

For the single domain antigen binding site that specifically binds to PD-L1 or 4-1BB, it comprises a heavy chain variable domain (VH) and a light chain variable domain (VL) that specifically bind to PD-L1 or 4-1BB, a heavy chain variable domain in the camelidae antibody that are naturally light chain-free and composed of only two heavy chains from camelidae serum, a VH-like single domain of IgNAR from the shark family, a camelized human VH domain, a humanized camelidae antibody heavy chain variable domain.

In one embodiment, the triple-chain anti-4-1BB/PD-L1 bispecific antibody of the present invention comprises a first antigen binding site comprising a VH1/VL1 pair on the first and second polypeptide chains that specifically binds to 4-1BB, and a second and a third single domain antigen binding sites that specifically bind to PD-L1, wherein the second and third single domain antigen binding site that specifically binds to PD-L1 bind to the same epitope or different epitopes on PD-L1.

In one embodiment, the first antigen binding site specifically binding to 4-1BB and comprising a VH1/VL1 pair on the first polypeptide chain and the second polypeptide chain, comprises all of the six heavy-chain complementarity determining regions (CDRs) and light-chain CDRs contained in the paired heavy-chain variable region/light-chain variable region sequences of SEQ ID NOs: 26/28 derived from the anti-4-1BB antibody BMS-663513, or sequences having one, two, three, four, or five amino acid changes (e.g., amino acid substitutions or deletions) from one or more of the six CDRs.

In one embodiment, the first antigen binding site specifically binding to 4-1BB and comprising a VH1/VL1 pair on the first polypeptide chain and the second polypeptide chain, comprises paired heavy-chain variable region/light-chain variable region sequences of SEQ ID NOs: 26/28 derived from the anti-4-1BB antibody BMS-663513, or sequences having a sequence identity of at least 90%, 91%, 92%, 93%, 94%, 95%, 96%, 97%, 98%, 99% or higher with the paired heavy-chain variable region/light-chain variable region sequences.

In one embodiment, the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or sequences having one, two, three, four, or five amino acid changes (e.g., amino acid substitutions or deletions) from one or more of the three CDRs. In another embodiment, the second and the third single-domain antigen binding sites specifically binding to PD-L1 on the third polypeptide chain comprise an amino acid sequence shown in SEQ ID NO: 15 and/or SEQ ID NO: 16, or sequences substantially identical (e.g., having at least 80%, 85%, 90%, 92%, 95%, 97%, 98%, 99% or higher identity) thereto.

The type of the heavy chain constant region of the immunoglobulin in the first polypeptide chain and the third polypeptide chain in the triple-chain anti-4-1BB/PD-L1 bispecific antibody of the present invention is not particularly limited, and is preferably the heavy chain constant region of the IgG1, IgG2, or IgG4 immunoglobulin, or a sequence that is substantially identical (e.g., at least 80%, 85%, 90%, 92%, 95%, 97%, 98%, 99% or higher identity) to it. More preferably, the heavy chain constant region is a heavy chain constant region of a human IgG1 immunoglobulin, or a sequence substantially identical (for example, at least 80%, 85%, 90%, 92%, 95%, 97%, 98%, 99%, or higher identity) thereto.

In one embodiment, the triple-chain anti-4-1BB/PD-L1 bispecific antibody of the present invention comprises a heavy chain constant region used in IgG4 (e.g., human IgG4). In yet another embodiment, a triple-chain anti-4-1BB/PD-L1 bispecific antibody of the present invention comprises a heavy chain constant region for IgG1 (e.g., human IgG1). For example, each Fc domain of the first polypeptide chain and the third polypeptide chain of the triple-chain antibody comprises a hinge region having a "CPPC" amino acid sequence, and/or each comprises Y349C and S354C (according to the "EU numbering" of Kabat), whereby the first polypeptide chain and the third polypeptide chain form interchain disulfide bonds in the Fc region and thus stabilize the correct pairing of the first polypeptide chain and the third polypeptide chain.

In one embodiment, the first polypeptide chain and/or the third polypeptide chain of the triple-chain anti-4-1BB/PD-L1 bispecific antibody of the present invention comprise amino acid mutations in the Fc domain which affect the function of the antibody effector. In one specific embodiment, the amino acid substitution is LALA mutation.

In yet another embodiment, the second polypeptide chain of the triple-chain anti-4-1BB/PD-L1 bispecific antibody of the present invention comprises a kappa light chain constant region or a lambda light chain constant region, for example, a human kappa light chain constant region or a human lambda light chain constant region. In one embodiment, the light chain constant region comprises an amino acid sequence shown in SEQ ID NO: 13, or a sequence substantially identical (e.g., having at least 80%, 85%, 90%, 92%, 95%, 97%, 98%, 99% or higher identity) thereto.

In one embodiment, Fc domains of the first polypeptide chain and the third polypeptide chain of the triple-chain anti-4-1BB/PD-L1 bispecific antibody of the present invention each comprise a stable association of "knob-in-hole". In one embodiment, the amino acid substitution T366W is contained in one of the first polypeptide chain and the third polypeptide chain, and the amino acid substitutions T366S, L368A, and Y407V (EU numbering) are contained in the other one of the first polypeptide chain and the third polypeptide chain. Thus, the protuberance in one chain can be placed in the cavity in the other chain, which promotes the correct pairing of the first polypeptide chain and the third polypeptide chain.

In one embodiment, the immunoglobulin CHI domain and CL domain of the first polypeptide chain and the second polypeptide chain of the triple-chain anti-4-1BB/PD-L1 bispecific antibody of the present invention respectively comprise a protuberance and a cavity, and the protuberance or cavity in the CHI domain can be placed in the protuberance or cavity in the CL domain, respectively, so that the first polypeptide chain and the second polypeptide chain also form a "knob-in-hole" stable association with each other.

In one embodiment, the triple-chain anti-4-1BB/PD-L1 bispecific antibody of the present invention comprises a first polypeptide chain shown in SEQ ID NO: 25, a second polypeptide chain shown in SEQ ID NO: 27, and a third polypeptide chain shown in SEQ ID NO: 14, or sequences substantially identical (e.g., having at least 80%, 85%, 90%, 92%, 95%, 97%, 98%, 99% or higher identity) to any one of the sequences.

In another embodiment, the triple-chain anti-4-1BB/PD-L1 bispecific antibody of the present invention comprises a first polypeptide chain shown in SEQ ID NO: 25, a second polypeptide chain shown in SEQ ID NO: 27, and a third polypeptide chain shown in SEQ ID NO: 22, or sequences substantially identical (e.g., having at least 80%, 85%, 90%, 92%, 95%, 97%, 98%, 99% or higher identity) to any one of the sequences.

The triple-chain anti-4-1BB/PD-L1 bispecific antibody of the present invention can simultaneously bind to PD-L1 and 4-1BB proteins and maintain the affinity constant of the parent antibody, thereby being able to block the PD-1/PD -L1 signaling pathway and activate the 4-1BB/4-1BB ligand signaling pathway in T cells and natural killer (NK) cells. The triple-chain anti-4-1BB/PD-L1 bispecific antibody of the present invention can be used for the treatment, prevention, or diagnosis of diseases associated with the signaling pathway.
iii) In one embodiment, the triple-chain antibody is an anti-LAG-3/PD-L1 bispecific antibody or a multispecific antibody.

The embodiment relates to an anti-LAG-3/PD-L1 bispecific antibody or multispecific antibody that blocks the PD-1/PD-L1 signaling pathway and acts on the LAG-3 signaling pathway.

LAG-3 (lymphocyte activating gene-3, also known as CD223) is a cell surface molecule expressed on activated T cells and B cells, and is a cell consumption marker with immunosuppressive activity, and the cell surface molecule has been shown to play a role in CD8+ T cell depletion.

Class II histocompatibility complex (MHC-II) is a ligand of LAG-3, and other ligands of LAG-3 (such as L-selectin and galectin-3) have also been identified (Anderson A.C. et al., Lag-3, tim-3, and TIGIT: Co-inhibitory receptors with specialized functions in immune regulation, Immunity, 2016, 44 (5): 989-1004; Kouo T. et al., Galectin-3 shapes antitumor immune responses by suppressing CD8 + T cells via LAG-3 and inhibiting expansion of plasmacytoid dendritic cells, Cancer Immunol Res. 2015 April; 3 (4): 412-423).

In cancer, regulatory T cells (Tregs) expressing LAG-3 have enhanced antitumor activity, while cytotoxic CD8+ T cells expressing LAG-3 have a reduced proliferation rate and effector cytokine production (Scurr M. et al., Highly prevalent colorectal cancer-infiltrating LAP+ Foxp3- T cells exhibit more potent immunosuppressive activity than Foxp3+ regulatory T cells, Mucosal Immunol. 2014, 7 (2): 428-439). Splicing variants of LAG-3 that are cleaved by metalloproteinases and secreted in the cell microenvironment have immune activation when bind to MHC-II on antigen-presenting cells (Casati C. et al., Soluble human LAG-3 molecule amplifies the in vitro generation of type 1 tumor-specific immunity, Clinical Cancer Research. American Association for Cancer Research; 2006, 66 (8): 4450-4460).

LAG-3+ tumor infiltrating lymphocytes (TIL) have been reported in patients with melanoma, colon cancer, pancreatic cancer, breast cancer, lung cancer, hematopoietic cancer, and head and neck cancer (Demeure C.E. et al., T Lymphocytes infiltrating various tumour types express the MHC class II ligand lymphocyte activation gene-3 (LAG-3): role of LAG-3 / MHC class II interactions in cell-cell contacts, Eur. J. Cancer, 2001, 37 (13): 1709-1718). An antibody-based LAG-3 blockade restores CD8+ effector T cells and reduces Treg populations in various cancer mouse models.

The present inventors have developed a monoclonal antibody that inhibits the LAG-3 signaling pathway, which is an anti-LAG-3 antibody ADI-31853, having a paired heavy chain variable region sequence/light chain variable region sequence of SEQ ID NOs: 30/35.

The triple-chain anti-LAG-3/PD-L1 bispecific antibody or multispecific antibody of the present invention at least simultaneously targets antibodies of LAG-3 and PD-L1, and three antigen binding sites bind to LAG-3 and/or PD-L1 molecules, respectively. In one embodiment, the triple-chain anti-LAG-3/PD-L1 bispecific antibody comprises a first antigen binding site comprising a VH1/VL1 pair on the first and second polypeptide chains that specifically binds to PD-L1 or LAG-3, and a second single domain antigen binding site that specifically binds to PD-L1 or LAG-3 and a third single domain antigen binding site that specifically binds to PD-L1 or LAG-3 on the third polypeptide chain. In one embodiment, the anti-LAG-3/PD-L1 bispecific antibody comprises a first antigen binding site which specifically binds to the LAG-3 and comprises a VH1/VL1 pair on the first polypeptide chain and the second polypeptide chain, and a first VHH and a second VHH which specifically binds to the PD-L1 on the third polypeptide chain. In another embodiment, the triple-chain anti-LAG-3/PD-L1 bispecific antibody comprises a first antigen binding site which specifically binds to the LAG-3 and comprises a VH1/VL1 pair on the first polypeptide chain and the second polypeptide chain, and a first VHH and a second VHH which specifically binds to the PD-L1 on the third polypeptide chain.

For the first antigen binding site comprising a VH1/VL1 pair that specifically binds to PD-L1 or LAG-3, "VH1/VL1 pair" comprises six CDRs derived from VH1/VL1 pairs of any anti-PD-L1 antibody reported in the prior art (e.g., the anti-PD-L1 antibody exemplified above) and anti-PD-L1 antibodies developed in the future, or sequences having one, two, three, four, or five amino acid changes (e.g., amino acid substitutions or deletions) with one or more of the six CDRs; comprises six CDRs derived from VH1/VL1 pairs of any anti-LAG-3 antibody reported in the prior art (e.g., the anti-LAG-3 antibody exemplified above) and anti-LAG-3 antibodies developed in the future, or sequences having one, two, three, four, or five amino acid changes (e.g., amino acid substitutions or deletions) with one or more of the six CDRs.

For the single domain antigen binding site that specifically binds to PD-L1 or LAG-3, it comprises a heavy chain variable domain (VH) and a light chain variable domain (VL) that specifically bind to PD-L1 or LAG-3, a heavy chain variable domain in the camelidae antibody that are naturally light chain-free and composed of only two heavy chains from camelidae serum, a VH-like single domain of IgNAR from the shark family, a camelized human VH domain, a humanized camelidae antibody heavy chain variable domain.

In one embodiment, the triple-chain anti-LAG-3/PD-L1 bispecific antibody of the present invention comprises a first antigen binding site comprising a VH1/VL1 pair on the first and second polypeptide chains that specifically binds to LAG-3, and a second and a third single domain antigen binding sites that specifically bind to PD-L1, wherein the second and third single domain antigen binding site that specifically binds to PD-L1 bind to the same epitope or different epitopes on PD-L1.

In one embodiment, the first antigen binding site specifically binding to LAG-3 and comprising the VH1/VL1 pair on the first polypeptide chain and the second polypeptide chain, comprises a VH CDR1 shown in GSIYSESYYWG (SEQ ID NO: 31), a VH CDR2 shown in SIVYSGYTYYNPSLKS (SEQ ID NO: 32), a VH CDR3 shown in ARVRTWDAAFDI (SEQ ID NO: 33), a VL CDR1 shown in QASQDISNYLN (SEQ ID NO: 36), a VL CDR2 shown in DASNLET (SEQ ID NO: 37), and a VL CDR3 shown in QQVLELPPWT (SEQ ID NO: 38) derived from anti-LAG-3 antibody ADI-31853, or a sequence having one, two, three, four, or five amino acid changes (e.g., amino acid substitutions or deletions) from one or more of the six CDRs; In one embodiment, the first antigen binding site specifically binding to LAG-3 and comprising a VH1/VL1 pair on the first polypeptide chain and the second polypeptide chain, comprises paired heavy-chain variable region/light-chain variable region sequences of SEQ ID NOs: 30/35 derived from the anti-LAG-3 antibody ADI-31853, or sequences having a sequence identity of at least 90%, 91%, 92%, 93%, 94%, 95%, 96%, 97%, 98%, 99% or higher with the paired heavy-chain variable region/light-chain variable region sequences.

In one embodiment, the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or sequences having one, two, three, four, or five amino acid changes (e.g., amino acid substitutions or deletions) from one or more of the three CDRs. In another embodiment, the second and the third single-domain antigen binding sites specifically binding to PD-L1 on the third polypeptide chain comprise an amino acid sequence shown in SEQ ID NO: 15 and/or SEQ ID NO: 16, or sequences substantially identical (e.g., having at least 80%, 85%, 90%, 92%, 95%, 97%, 98%, 99% or higher identity) thereto.

The type of the heavy chain constant region of the immunoglobulin in the first polypeptide chain and the third polypeptide chain in the triple-chain anti-LAG-3/PD-L1 bispecific antibody of the present invention is not particularly limited, and is preferably the heavy chain constant region of the IgG1, IgG2, or IgG4 immunoglobulin, or a sequence that is substantially identical (e.g., at least 80%, 85%, 90%, 92%, 95%, 97%, 98%, 99% or higher identity) to it. More preferably, the heavy chain constant region is a heavy chain constant region of a human IgG1 immunoglobulin, or a sequence substantially identical (for example, at least 80%, 85%, 90%, 92%, 95%, 97%, 98%, 99%, or higher identity) thereto.

In one embodiment, the triple-chain anti-LAG-3/PD-L1 bispecific antibody of the present invention comprises a heavy chain constant region used in IgG4 (e.g,, human IgG4). In yet another embodiment, a triple-chain anti-LAG-3/PD-L1 bispecific antibody of the present invention comprises a heavy chain constant region for IgG1 (e.g., human IgG1). For example, each Fc domain of the first polypeptide chain and the third polypeptide chain of the triple-chain antibody comprises a hinge region having a "CPPC" amino acid sequence, and/or each comprises Y349C and S354C (according to the "EU numbering" of Kabat), whereby the first polypeptide chain and the third polypeptide chain form interchain disulfide bonds in the Fc region and thus stabilize the correct pairing of the first polypeptide chain and the third polypeptide chain.

In one embodiment, the first polypeptide chain and/or the third polypeptide chain of the triple-chain anti-LAG-3/PD-L1 bispecific antibody of the present invention comprises amino acid mutations in the Fc domain which affects the function of the antibody effector. In one specific embodiment, the amino acid substitution is LALA mutation.

In yet another embodiment, the second polypeptide chain of the triple-chain anti-LAG-3/PD-L1 bispecific antibody of the present invention comprises a kappa light chain constant region or a lambda light chain constant region, for example, a human kappa light chain constant region or a human lambda light chain constant region. In one embodiment, the light chain constant region comprises an amino acid sequence shown in SEQ ID NO: 13, or a sequence substantially identical (e.g., having at least 80%, 85%, 90%, 92%, 95%, 97%, 98%, 99% or higher identity) thereto.

In one embodiment, Fc domains of the first polypeptide chain and the third polypeptide chain of the triple-chain anti-LAG-3/PD-L1 bispecific antibody of the present invention each comprise a stable association of "knob-in-hole". In one embodiment, the amino acid substitution T366W is contained in one of the first polypeptide chain and the third polypeptide chain, and the amino acid substitutions T366S, L368A, and Y407V (EU numbering) are contained in the other one of the first polypeptide chain and the third polypeptide chain. Thus, the protuberance in one chain can be placed in the cavity in the other chain, which promotes the correct pairing of the first polypeptide chain and the third polypeptide chain.

In one embodiment, the immunoglobulin CHI domain and CL domain of the first polypeptide chain and the second polypeptide chain of the triple-chain anti-LAG-3/PD-L1 bispecific antibody of the present invention respectively comprise a protuberance or a cavity, and the protuberance or cavity in the CHI domain can be placed in the protuberance or cavity in the CL domain, respectively, so that the first polypeptide chain and the second polypeptide chain also form a "knob-in-hole" stable association with each other.

In one embodiment, the triple-chain anti-LAG-3/PD-L1 bispecific antibody of the present invention comprises a first polypeptide chain shown in SEQ ID NO: 29, a second polypeptide chain shown in SEQ ID NO: 34, and a third polypeptide chain shown in SEQ ID NO: 14, or sequences substantially identical (e.g., having at least 80%, 85%, 90%, 92%, 95%, 97%, 98%, 99% or higher identity) to any one of the sequences.

In another embodiment, the triple-chain anti-LAG-3/PD-L1 bispecific antibody of the present invention comprises a first polypeptide chain shown in SEQ ID NO: 29, a second polypeptide chain shown in SEQ ID NO: 34, and a third polypeptide chain shown in SEQ ID NO: 22, or sequences substantially identical (e.g., having at least 80%, 85%, 90%, 92%, 95%, 97%, 98%, 99% or higher identity) to any one of the sequences.

The triple-chain anti-LAG-3/PD-L1 bispecific antibody of the present invention can simultaneously bind to PD-L1 and LAG-3 proteins, and maintains the affinity constant of the parent antibody, thereby being able to block the PD-1/PD-L1 signaling pathway and block the LAG-3 signaling pathway. The triple-chain anti-LAG-3/PD-L1 bispecific antibody of the present invention can be used for the treatment, prevention, or diagnosis of diseases associated with the signaling pathway.

### III. The triple-chain antibody variants of the present invention

In certain embodiments, amino acid sequence variants of the bispecific antibodies exemplified herein are contemplated. For example, it may be desirable to improve the binding affinity and/or other biological properties of a bispecific antibody. Amino acid sequence variants of a bispecific antibody can be prepared by introducing appropriate modifications to the nucleotide sequence encoding the bispecific antibody or by peptide synthesis. Such modifications include, for example, deleting residues from the amino acid sequence of an antibody and/or inserting residues into the amino acid sequence and/or replacing residues in the amino acid sequence. Any combination of deletions, insertions and substitutions can be made to obtain a final construct, so long as the final construct possesses the desired characteristics, such as antigen binding effect.

Conservative substitutions are shown in Table 1 under the heading "conservative substitutions". More obvious changes are shown in Table 1 under the heading "exemplary substitutions" and referred to amino acid side chain categories are described further below. Amino acid substitutions can be introduced into the antibody of interest and screened for desired activity on the product, for example, retained/improved antigen binding effect or reduced immunogenicity.

**Table 1**

| Primitive residue | Exemplary substitution | Preferred replacement |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

Amino acids can be grouped according to common side chain characteristics:
(1) Hydrophobicity: norleucine, Met, Ala, Val, Leu; Ile;
(2) Neutral hydrophilic: Cys, Ser, Thr, Asn; Gln;
(3) Acidic: Asp, Glu;
(4) Alkaline: His, Lys, Arg;
(5) Residues affecting chain direction: Gly, Pro;
(6) Aromatic: Trp, Tyr, Phe.

Non-conservative substitutions will exchange members of one of these classes for members of another class.

### IV. Immunoconjugate

The triple-chain antibody of the present invention is capable of recombinant fusion or chemical conjugation (including covalent and non-covalent conjugation) to a heterologous protein or polypeptide to produce a fusion protein. Methods of fusion or conjugation of a protein, polypeptide or peptide to an antibody are known in the art. See, for example, U.S. Patent Nos. 5,336,603, 5,622,929 and EP 367,166.

In addition, the triple-chain antibody of the present invention can be fused to a labeled sequence (such as a peptide) to facilitate purification. In a preferred embodiment, the labeled amino acid sequence is a hexahistidine peptide, such as a tag provided in a pQE vector (QIAGEN, Inc., 9259 Eton Avenue, Chatsworth, CA, 91311), etc., many of which are commercially available. As described in Gentz et al., 1989, Proc. Natl. Acad. Sci. USA 86: 821-824, for example, a hexahistidine provides convenient purification of fusion proteins. Other peptide tags for purification include, but are not limited to, hemagglutinin ("HA") tags, which correspond to epitopes derived from influenza hemagglutinin proteins (Wilson et al., 1984, Cell 37: 767) and "flag" tag.

In other embodiments, the triple-chain antibody of the present invention is conjugated to a diagnostic or detectable agent. Such antibodies can be used as a part of clinical test methods (e.g., to determine the effect of a particular therapy), for monitoring or predicting the onset, development, progression, and/or severity of a disease or condition. Such diagnosis and detection can be achieved by coupling antibodies to a detectable substance including but not limited to, a variety of enzymes, such as but not limited to, horseradish peroxidase, alkaline phosphatase, β-galactosidase or acetylcholinesterase; prosthetic groups, such as but not limited to, streptavidin/biotin and avidin/biotin; fluorescent substances, such as but not limited to, umbelliferone, fluorescein, isothiocyanate fluorescence , rhodamine, dichlorotriazamine fluorescein, dansyl chloride or phycoerythrin; luminescent substances such as but not limited to, luminol; bioluminescent substances, such as but not limited to luciferase, luciferin and aequorin luminous protein; radioactive materials, such as but not limited to, iodine (¹³¹I, ¹²⁵I, ¹²³I and ¹²¹I), carbon (¹⁴C), sulfur (³⁵S), thallium (³H), indium (¹¹⁵In, ¹¹³In, ¹¹²In and ¹¹¹In), and thorium (⁹⁹Tc), Thallium (²⁰¹Ti), Gallium (⁶⁸Ga, ⁶⁷Ga), Palladium (¹⁰³Pd), Molybdenum (99Mo), Xenon (133Xe), Fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ¹¹³Sn and ¹¹⁷Tin; and positron-emitting metal and non-radioactive paramagnetic metal ions used in various positron emission imaging techniques.

The present invention also includes the use of a triple-chain antibody conjugated to a therapeutic moiety. A triple-chain antibody can be conjugated to a therapeutic moiety, such as a cytotoxin (e.g., a cytostatic agent or cytotoxic agent), a therapeutic agent, or a radioactive metal ion, such as an alpha emitter. The term "cytotoxin" or "cytotoxic agent" includes any substance that is harmful to cells.

In addition, a triple-chain antibody can be conjugated to a therapeutic or drug moiety that modulates a given biological response. The therapeutic or drug moiety should not be interpreted as limited to classic chemotherapeutics. For example, the drug moiety may be a protein, peptide or polypeptide possessing the desired biological activity. Such proteins may, for example, include toxins such as acacia toxin, ricin A, pseudomonas exotoxin, cholera toxin, or diphtheria toxin; proteins such as tumor necrosis factor, alpha-interferon, beta-interferon, nerve growth factors, platelet-derived growth factors, tissue plasminogen activators, apoptotic agents, anti-angiogenic agents, or biological response modifiers, such as lymphokines.

In addition, antibodies can be conjugated to therapeutic moieties such as radioactive metal ions, such as alpha emitters such as ²¹³Bi or can be used to conjugate radioactive metal ions (including but not limited to ¹³¹In, ¹³¹LU, ¹³¹Y, ¹³¹Ho, ¹³¹Sm) to macrocyclic chelating agent of polypeptide. In certain embodiments, macrocyclic chelating agent is 1,4,7,10-tetraazacyclododecane-N, N', N", N"'-tetraacetic acid (DOTA), which can be attached to the antibody via a linker molecule. Such linker molecules are well known in the art and are described in Denardo et al., 1998, Clin Cancer Res. 4 (10): 2483-90, each of which is incorporated by reference in its entirety.

Techniques for conjugating a therapeutic moiety to an antibody are well known, see, e.g., Arnon et al., "Monoclonal Antibodies For Immunotargeting Of Drugs In Cancer Therapy", cited in Monoclonal Antibodies And Cancer Therapy, Reisfeld et al. (authoring), pp. 243-256 (Alan R. Liss, Inc. 1985).

Antibodies can also be attached to a solid phase support, which is particularly useful for immunoassays or purification of target antigens. Such solid phase supports include, but are not limited to, glass, cellulose, polyacrylamide, nylon, polystyrene, polyvinyl chloride, or polypropylene.

### V. Production and purification of the triple-chain antibody of the present invention

The triple-chain antibody of the present invention can be obtained, for example, by solid state peptide synthesis (for example, Merrifield solid phase synthesis) or recombinant production. For recombinant production, a polynucleotide encoding the first polypeptide chain, a polynucleotide encoding the second polypeptide chain, and/or a polynucleotide encoding the third polypeptide chain of the triple-chain antibody are isolated and inserted into one or more vectors for further cloning and/or expression in a host cell. The polynucleotides can be easily isolated and sequenced using conventional methods. In one embodiment, a vector, preferably an expression vector, comprising one or more polynucleotides of the present invention is provided.

Methods known to those skilled in the art can be used to construct expression vectors. The expression vector includes, but is not limited to, a virus, a plasmid, a cosmid, a lambda phage or a yeast artificial chromosome (YAC).

Once an expression vector comprising one or more polynucleotides of the present invention has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, lipid-based transfection, or other conventional techniques.

In one embodiment, a host cell comprising one or more polynucleotides of the present invention is provided. In some embodiments, a host cell comprising an expression vector of the present invention is provided. As used herein, the term "host cell" refers to any kind of cell system that can be engineered to produce a triple-chain antibody of the present invention. Host cells suitable for replication and support for expression of the triple-chain antibody of the present invention are well known in the art. Such cells can be transfected or transduced with a specific expression vector as needed, and a large number of cells comprising vectors can be cultivated for inoculation of large-scale fermenters to obtain a sufficient amount of the triple-chain antibody of the present invention for clinical application. Suitable host cells include prokaryotic microorganisms, such as E. coli, eukaryotic microorganisms, such as filamentous fungi or yeast, or various eukaryotic cells, such as Chinese hamster ovary cells (CHO), insect cells, and the like. A mammalian cell line suitable for suspension growth may be used. Examples of useful mammalian host cell lines include monkey kidney CV1 line (COS-7) transformed by SV40; human embryonic kidney line (HEK 293 or 293F cells), baby hamster kidney cell (BHK), monkey kidney cell (CV1), African green monkey kidney cell (VERO-76), human cervical cancer cell (HELA), canine kidney cell (MDCK), buffalo rat liver cell (BRL 3A), human lung cell (W138), human liver cell (Hep G2), CHO cell, NSO cell, myeloma cell line such as YO, NS0, P3X63 and Sp2/0, etc. Reviews of certain mammalian host cell lines suitable for protein production, see, for example, Yazaki and Wu, Methods in Molecular Biology, vol. 248 (edited by.B. K. C. Lo, Humana Press, Totowa, NJ), pp. 255-268 (2003). In a preferred embodiment, the host cell is a CHO, HEK293 or NSO cell.

Standard techniques for expressing exogenous genes in these host cell systems are known in the art. In one embodiment, a method of producing a triple-chain antibody of the present invention is provided, wherein the method includes culturing a host cell comprising a polynucleotide encoding the triple-chain antibody as provided herein under conditions suitable for expressing the triple-chain antibody, and recovering the triple-chain antibody from the host cell (or host cell culture medium).

The triple-chain antibody prepared as described herein can be purified by known prior art such as high performance liquid chromatography, ion exchange chromatography, gel electrophoresis, affinity chromatography, size exclusion chromatography, and the like. The actual conditions used to purify a particular protein also depend on factors such as net charge, hydrophobicity, hydrophilicity, etc., and these will be apparent to those skilled in the art.

The purity of the triple-chain antibody of the present invention can be determined by any one of a variety of well-known analytical methods including size exclusion chromatography, gel electrophoresis, high-performance liquid chromatography, and the like. The physical/chemical properties and/or biological activity of the triple-chain antibody provided herein can be identified, screened, or characterized by a variety of assays known in the art.

### VI. Pharmaceutical compositions and kits

In another aspect, the present invention provides a composition, e.g., a pharmaceutical composition comprising the triple-chain antibody described herein formulated together with a pharmaceutically acceptable carrier. As used herein, a "pharmaceutically acceptable carrier" includes any and all solvents, dispersion media, isotonic agent and absorption delaying agent, and the like that are physiologically compatible. The pharmaceutical composition of the present invention is suitable for intravenous, intramuscular, subcutaneous, parenteral, rectal, spinal or epidermal administration (e.g., by injection or infusion).

Also disclosed herein are compositions obtained by combining the triple-chain antibody described herein with more than one therapeutic agent, which is selected from one, two, or all of the following categories (i)-(iii): (i) drugs that enhance antigen presentation (e.g., tumor antigen presentation); (ii) drugs that enhance effector cell responses (e.g., B cell and/or T cell activation and/or mobilization); or (iii) drugs that reduce immunosuppression.

The compositions of the present invention may be in a variety of forms. These forms include, for example, liquid, semi-solid, and solid dosage forms, such as liquid solutions (e.g., injectable solutions and infusible solutions), dispersions or suspensions, liposomes, and suppositories. The preferred form depends on the intended mode of administration and therapeutic use. Commonly preferred compositions are in the form of injectable solutions or infusible solutions. The preferred mode of administration is parenteral (e.g., intravenous, subcutaneous, intraperitoneal (i.p.), intramuscular) injection. In a preferred embodiment, the triple-chain antibody is administered by intravenous infusion or injection. In another preferred embodiment, the triple-chain antibody is administered by intramuscular, intraperitoneal or subcutaneous injection.

As used herein, the phrases "parenteral administration" and "administered parenterally" mean modes of administration other than enteral and topical administration, typically by injection, and include, but are not limited to, intravenous, intramuscular, intra-arterial, intradermal, intraperitoneal, transtracheal, subcutaneous injection and infusion.

Therapeutic compositions generally should be sterile and stable under the conditions of manufacture and storage. The composition can be formulated as a solution, microemulsion, dispersion, liposome, or lyophilized form. Sterile injectable solutions can be prepared by adding the active compound (i.e., a triple-chain antibody) in the required amount to a suitable solvent, followed by filtration and sterilization. Generally, dispersions are prepared by incorporating the active compound into a sterile vehicle, which comprises a basic dispersion medium and the other ingredients. Coating agents such as lecithin and the like can be used. In the case of dispersions, the proper fluidity of a solution can be maintained by using a surfactant. Prolonged absorption of injectable compositions can be caused by including in the composition delayed absorption substances such as monostearate and gelatin.

In certain embodiments, the triple-chain antibody of the present invention can be administered orally, such as administered orally with an inert diluent or an edible carrier. The triple-chain antibody of the present invention can also be enclosed in hard or soft shell gelatin capsules, compressed into tablets, or incorporated directly into the subject's diet. For oral therapeutic administration, the compounds can be incorporated with excipients and used in the form of ingestible tablets, buccal tablets, troches, capsules, elixirs, suspensions, syrups, wafers, and the like. In order to administer the triple-chain antibody of the present invention by a method other than parenteral administration, it may be necessary to coat or co-administer the triple-chain antibody with a material to prevent its inactivation. Therapeutic compositions can also be administered using medical devices known in the art.

The pharmaceutical composition of the present invention may comprise a "therapeutically effective amount" or a "prophylactically effective amount" of the triple-chain antibody of the present invention. A "therapeutically effective amount" refers to an amount effective to achieve a desired therapeutic result at a necessary dosage for a necessary period of time. A therapeutically effective amount can be varied according to a variety of factors such as disease state, age, gender, and weight of the individual. A therapeutically effective amount is an amount in which any toxic or harmful effect is outweighed by the therapeutically beneficial effect. A "therapeutically effective amount" preferably inhibits a measurable parameter (e.g., tumor growth rate) by at least about 20%, more preferably at least about 40%, even more preferably at least about 60%, and still more preferably at least about 80%, relative to untreated subjects. The ability of a triple-chain antibody of the present invention to inhibit a measurable parameter (e.g., cancer volume) can be evaluated in an animal model system that predicts efficacy in human tumors.

A "prophylactically effective amount" refers to an amount effective to achieve a desired prophylactic result at a necessary dosage for a necessary period of time. Generally, since a prophylactic dose is used in subjects before or at an earlier stage of a disease, the prophylactically effective amount will be less than the therapeutically effective amount.

A kit comprising a triple-chain antibody described herein is also within the scope of the present invention. A kit may include one or more other elements, including, for example : instructions for use; other reagents, such as a label or a reagent for coupling; a pharmaceutically acceptable carrier; and a device or other material for administration to a subject.

### VII. Uses of the triple-chain antibody

The triple-chain antibodies disclosed herein have in vitro and in vivo diagnostic uses, as well as therapeutic and prophylactic uses. For example, these molecules can be administered to in vitro or ex vivo cultured cells or to a subject, such as a human subject, to treat, prevent, and/or diagnose a variety of antigen related diseases, such as cancers, autoimmune diseases, acute and chronic inflammatory diseases, and infectious diseases (e.g., chronic infectious diseases or sepsis).

In one aspect, the present invention provides a diagnostic method for detecting the presence of related antigens in a biological sample, such as serum, semen, or urine or tissue biopsy samples (e.g., from a hyperproliferative or cancerous lesion), in vitro or in vivo. The diagnostic method includes: (i) contacting a sample (and optionally a control sample) with the triple-chain antibody as described herein or administering the triple-chain antibody to a subject under conditions that allow the interaction to occur and (ii) detecting the formation of a complex between the triple-chain antibody and the sample (and optionally a control sample). The formation of a complex indicates the presence of the related antigen and may show the suitability or need for the treatment and/or prevention described herein.

In some embodiments, the related antigen is detected prior to the treatment, e.g., prior to the initial treatment or prior to a certain treatment after a treatmen interval. Detection methods that can be used include immunohistochemistry, immunocytochemistry, FACS, ELISA assays, PCR techniques (e.g., RT-PCR), or in vivo imaging techniques. Generally, triple-chain antibodies used in vivo and in vitro detection methods are directly or indirectly labeled with a detectable substance to facilitate the detection of bound or unbound conjugates. Suitable detectable substances include a variety of biologically active enzymes, prosthetic groups, fluorescent substances, luminescent substances, paramagnetic (e.g., nuclear magnetic resonance active) substances, and radioactive substances.

In some embodiments, the level and/or distribution of the related antigen is determined in vivo, for example, the detectable substance labeled triple-antibody of the present invention is determined in a non-invasive manner (e.g., detected by using appropriate imaging techniques (e.g., positron emission tomography (PET) scanning)). In one embodiment, for example, the level and/or distribution of the related antigen is determined in vivo by detecting the triple-chain antibody of the present invention that is detectably labeled with a PET reagent (e.g., ¹⁸F-fluorodeoxyglucose (FDG)).

In one embodiment, the invention provides a diagnostic kit comprising the triple-chain antibody described herein and instructions for use.

In another aspect, the present invention relates to the use of triple-chain antibodies in vivo for the treatment or prevention of diseases requiring modulation of an immune response in a subject, thereby inhibiting or reducing the occurrence or recurrence of related diseases such as cancerous tumors, autoimmune diseases, acute and chronic inflammatory diseases, infectious diseases (for example, chronic infectious diseases or sepsis). The triple-chain antibody of the present invention may be used alone. Alternatively, the triple-chain antibody can be administered in combination with other cancer therapeutic/prophylactic agents. When the triple-chain antibody of the present invention is administered in combination with one or more other drugs, such combinations can be administered in any order or simultaneously.

Accordingly, in one embodiment, the present invention provides a method for modulating an immune response in a subject, which includes administering to the subject a therapeutically effective amount of the triple-chain antibody described herein. In another embodiment, the present invention provides a method for preventing the occurrence or recurrence of a disease in a subject, which includes administering to the subject a prophylactically effective amount of the triple-chain antibody described herein.

In some embodiments, cancers treated and/or prevented with triple-chain antibodies include, but are not limited to, solid tumors, hematological cancers (e.g., leukemia, lymphoma, myeloma such as multiple myeloma), and metastatic lesions. In one embodiment, the cancer is a solid tumor. Examples of solid tumors include malignancies, such as sarcomas and cancers of multiple organ systems, such as those cancers that invade the lung, breast, ovary, lymphoid, gastrointestinal tract (e.g., colon), anus, genital and genitourinary (e.g., kidney, bladder epithelium, bladder cells, prostate), pharynx, CNS (e.g., brain, neurological or glial cells), head and neck, skin (e.g., melanoma), nasopharynx (e.g., differentiated or undifferentiated metastatic or locally recurrent nasopharyngeal carcinoma) and pancreas, as well as adenocarcinoma, including malignant tumors such as colon cancer, rectal cancer, renal cell carcinoma, liver cancer, non-small cell lung cancer, small intestine cancer, and esophageal cancer. The cancer can be at an early, intermediate, or advanced stage or a metastatic cancer.

In some embodiments, the cancer is selected from melanoma, breast cancer, colon cancer, esophageal cancer, gastrointestinal stromal tumor (GIST), kidney cancer (e.g., renal cell carcinoma), liver cancer, non-small cell lung cancer (NSCLC), ovarian cancer, pancreatic cancer, prostate cancer, head and neck tumors, gastric cancer, and hematological malignancy (e.g., lymphoma).

In some embodiments, infectious diseases treated and/or prevented with the triple-chain antibody include pathogens for which no effective vaccine is currently present or pathogens for which conventional vaccines have not been fully effective. These include, but are not limited to, HIV, (A, B, and C) hepatitis, influenza, herpes, giardia, malaria, leishmania, staphylococcus aureus, and pseudomonas aeruginosa. The blocking effect of the triple-chain antibody exemplified by the present invention on PD-L1 can be particularly used to fight infections established by pathogens (such as HIV) that develop mutant antigens as the infection progresses. These mutant antigens can be regarded as foreign antigens when the anti-human PD-L1 antibody is administered, and thus the triple-chain antibody exemplified by the present invention can stimulate a strong T cell response that is not inhibited by a negative signal through PD-L1.

In some embodiments, the triple-chain antibody of the present invention is used to treat and/or prevent inflammatory and autoimmune diseases and graft versus host diseases (GvHD) to down regulate the immune system. Examples of autoimmune diseases that can be treated and/or prevented by administration of the triple-chain antibody of the present invention include, but are not limited to, alopecia areata, ankylosing spondylitis, autoimmune hepatitis, segmental ileitis, lupus erythematosus, ulcerative colitis, uveitis, etc. Examples of inflammatory diseases that can be treated and/or prevented by administration of the triple-chain antibody of the present invention include, but are not limited to, asthma, encephalitis, inflammatory bowel disease, allergic disease, septic shock, pulmonary fibrosis, arthritis and chronic inflammation due to chronic viral or bacterial infections.

The following examples are described to assist in understanding the present invention. The embodiments are not intended and should not be interpreted in any way as limiting the protection scope of the present invention.

### Examples

### Example 1. Construction, expression, purification, characterization and pharmacodynamic test of the anti-CD47/PD-L1 bispecific antibody

### Example 1.1. Construction of the anti-CD47/PD-L1 bispecific antibody

In this embodiment, two kinds of anti-CD47/PD-L1 bispecific antibodies were constructed and named as (1) bispecific antibody Kh2NF-PC, the schematic diagram of which is shown in Fig. 1A; and (2) bispecific antibody Kh2NF-PC-NL, the schematic diagram of which is shown in Fig. 1B.

It can be seen from the structural schematic diagram of FIG. 1A that the bispecific antibody Kh2NF-PC is composed of three polypeptide chains. Polypeptide chain #1 has an amino acid sequence shown in SEQ ID NO: 1, comprising a VH amino acid sequence derived from anti-CD47 antibody ADI29341 and shown in SEQ ID NO: 2, a CHI amino acid sequence derived from human IgG1 and shown in SEQ ID NO: 6 at C-terminus of the VH amino acid sequence, and a Fc region amino acid sequence derived from human IgG1 and shown in SEQ ID NO: 7 at C-terminus of the CHI amino acid sequence; Peptide # 2 has the amino acid sequence shown in SEQ ID NO: 8, comprising a VL amino acid sequence derived from anti-CD47 antibody ADI29341 and shown in SEQ ID NO: 9 and a human κ light chain constant region (CL) amino acid sequence shown in SEQ ID NO: 13 at C-terminus of the VL amino acid sequence; and peptide chain # 3 has the amino acid sequence shown in SEQ ID NO: 14, comprising the first and second anti-PD-L1 VHH amino acid sequences shown in SEQ ID NO: 16, a linker peptide amino acid sequence shown in SEQ ID NO: 20 between the first anti-PD-L1 VHH amino acid sequence and the second anti-PD-L1 VHH amino acid sequence and a Fc region amino acid sequence derived from human IgG1 and shown in SEQ ID NO: 21 at C-terminus of the second anti-PD-L1 VHH amino acid sequence.

As can be seen from the schematic diagram of Fig. 1B, Kh2NF-PC-NL also consists of three polypeptide chains. Peptide chain #1 has an amino acid sequence shown in SEQ ID NO: 1, peptide chain #2 has an amino acid sequence shown in SEQ ID NO: 8, and peptide chain # 3 has the amino acid sequence shown in SEQ ID NO: 22 from N-terminus to C-terminus, comprising a first and a second anti-PD-L1 VHH amino acid sequences shown in SEQ ID NO: 16 (there's no linker peptide between the first and second anti-PD-L1 VHH amino acid sequences) and a Fc region amino acid sequence derived from human IgG1 and shown in SEQ ID NO: 21 at C-terminus of the second anti-PD-L1 VHH amino acid sequence.

Three chains of the anti-CD47/PD-L1 bispecific antibodies Kh2NF-PC and Kh2NF-PC-NL were constructed as followed: the VH C-terminus of the CD47 antibody ADI-29341 was linked to the N-terminus of the constant region of human IgG1 to obtain peptide chain #1, in which the Fc region comprises a LALA mutation to reduce the effector function of the antibody of the present invention, and comprises a "knob-in-hole" mutation to stably associate with peptide #3; peptide #2 was derived from the VL and human kappa light chain constant regions of ADI-29341; peptide chain #3 comprises the first and second anti-PD-L1 VHH in tandem, with no linker peptide (in the bispecific antibody Kh2NF-PC-NL case) or with a flexible peptide (in the bispecific antibody Kh2NF-PC case) having 20 amino acid residues (G₄S)₄ between the two anti-PD-L1 VHH, and the C-terminus of the second anti-PD-L1 VHH was linked to the N-terminus of the Fc region amino acid sequence derived from IgG1 to obtain peptide chain #3, wherein the Fc region comprises LALA mutation to reduce the effector function of the antibody of the present invention, and comprises a "knob-in-hole" mutation to stably associate with peptide #1.

### Example 1.2. Expression and purification of the anti-CD47/PD-L1 bispecific antibody

In this embodiment, the nucleotide sequences encoding each of the three chains of the anti-CD47/PD-L1 bispecific antibodies Kh2NF-PC and Kh2NF-PC-NL constructed in Example 1.1 were all linked into a commercially available eukaryotic expression vector pTT5 via multiple cloning sites, and the bispecific antibodies Kh2NF-PC and Kh2NF-PC-NL were expressed using eukaryotic cells and purified. The specific operation is as followed.

Genewiz Suzhou was entrusted to synthesize the coding nucleotide sequences of the above three chains of the bispecific antibodies Kh2NF-PC and Kh2NF-PC-NL. The synthesized encoding nucleotide sequences of the above three chains were digested with restriction enzymes Xbal (New England Biolabs) and NotI (New England Biolabs) respectively, and then linked with vector pTT5 which was also digested by both Xbal and NotI, under the action of T4 DNA ligase (New England Biolabs). Three recombinant vectors each comprising one of the three coding nucleotide sequences were obtained.

The three recombinant vectors were used for subsequent expression after being verified by sequencing.

HEK293 cells (purchased from Invitrogen) were subcultured in Expi293 medium (purchased from Invitrogen). The cultures were centrifuged one day before transfection, and the resulting cell precipitate was resuspended using fresh Expi293 culture medium, with the cell density adjusted to 1×10⁶ cells/mL. HEK293 cells were further cultivated such that the cell density in the culture on the day of transfection was about 2×10⁶ cells/ml. F17 culture medium (purchased from Gibco, catalog number A13835-01) 1/10 of a final volume of the HEK293 cell suspension was taken as the transfection buffer.Each milliliter of transfection buffer was added with 10 µg of recombinant plasmids of nucleic acid sequences encoding peptide #1, peptide #2, and peptide #3 in a 1:1:1 molar ratio, and mixed well. The mixture was added with 30 µg of polyethylenimine (PEI; Polysciences, Cat. #23966) and mixed well. After 10 minutes of incubation, the PEI/DNA mixture was gently added into HEK293 cell suspension. The cultures were mixed gently and cultivated overnight at 8% CO₂ and 36.5 °C.

After an overnight incubation, 1/50 the volume of culture after transfection of 200 g/L FEED (Sigma, catalog number: H6784-100G) and 1/50 the volume of culture after transfection of 200 g/L glucose solution were supplemented to the culture flask. The system was mixed gently and incubated at 8% CO₂ and 36.5 °C. After 20 hours, VPA (Gibco, catalog number: 11140-050) was added to a final concentration of 2 mM/L. Continuous culture to day 7 or when the cell viability was ≤60%, the cultures were harvested, centrifuged at 7,500 rpm for 30 minutes. The cell supernatant was filtered with SARTOPORE (Sartorius, catalog number: 5441307H4), and purified by affinity chromatography and ion exchange chromatography on an *AKTApure* system (GE Healthcare).

Specific affinity chromatography purification steps are as follows: A MabSelect SuRe (GE Healthcare, catalog number: 17-5438-03) affinity chromatography column was selected and mounted in the *AKTApure* system. The *AKTApure* system equipped with a MabSelect SuRe affinity chromatography column was sterilized with 0.1 M NaOH overnight, and then the system was washed and the column was equilibrated with 5 times column volume of binding buffer (Tris 20 mM, NaCl 150 mM, pH 7.2). The filtered cell supernatant was loaded on the column. The column was reequilibrated with 5 to 10 times column volume of binding buffer and monitored to UV flatness using the UV detection device equipped with the *AKTApure* system. Then, the antibody was eluted with an elution buffer (citric acid + sodium citrate 100 mM, pH 3.5), and samples were collected based on the UV absorption value. 80 µL of neutralization buffer (Tris-HCl 2M) was added to each 1 mL of the collection solution for neutralizing for further ion exchange chromatography.

Specific ion exchange chromatography purification steps are as follows: a Superdex 200 Increase 10/300 GL (GE Healthcare, catalog number: 10245605) ion exchange chromatography column was selected and mounted in the *AKTApure* system. The *AKTApure* system equipped with a Superdex 200 Increase 10/300 GL ion exchange chromatography column was sterilized with 0.1 M NaOH overnight. Then, the system and the column were washed with distilled water. The column was equilibrated with 5 to 10 times column volume of loading buffer (citric acid + sodium citrate 100 mM, pH 5.0) until the conductivity and pH were stable. The obtained neutralization buffer (Tris-HCl 2M) containing the sample was exchanged with a loading buffer, and the sample was then loaded; the column was reequilibrated with 5 times column volume of loading buffer. A linear elution was employed with a gradient of 0-100% elution buffer 2 (citric acid + sodium citrate 100 mM, NaCl 1 M, pH 5.0). The sample was eluted with a total of 20 column volume of eluent, and was collected according to the UV absorption.

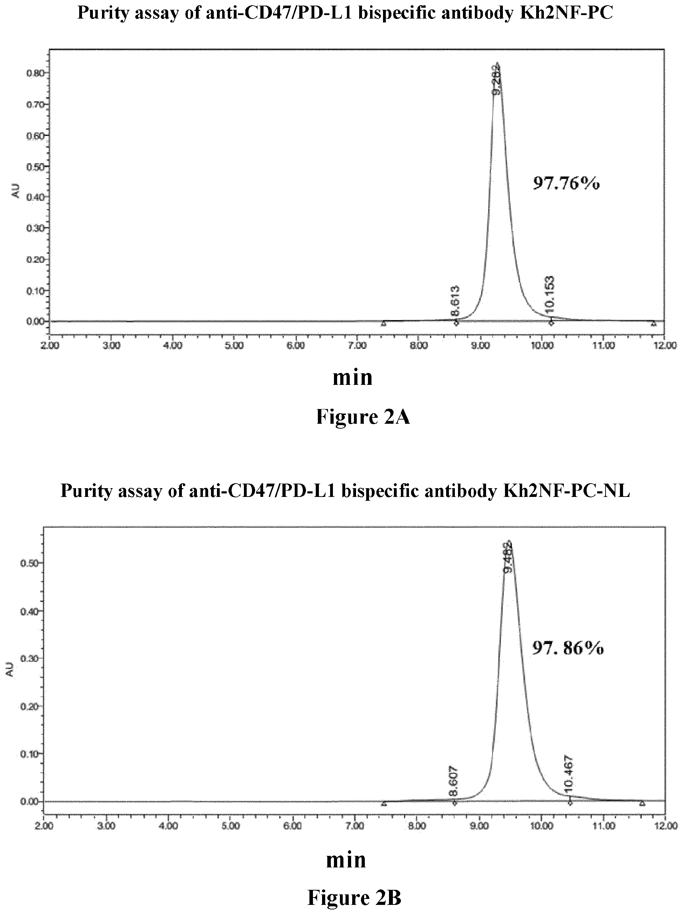

Fractions of collected sample were analyzed by size exclusion chromatography (SEC). Based on SEC results, fractions with a purity greater than 95% were combined. The SEC results are shown in Figs. 2A and 2B. The purity of the bispecific antibody Kh2NF-PC was 97.76%, and the purity of Kh2NF-PC-NL was 97.86%.

The purified bispecific antibody solution was centrifuged in a 15 mL ultrafiltration centrifuge tube at 4,500 rpm for 30 minutes. The protein was diluted with PBS and further centrifuged at 4,500 rpm for 30 minutes, and this operation was repeated twice to exchange the buffer. The antibodies after buffer exchange were combined to measure the antibody concentration.

### Example 1.3. Determination of the dissociation constant of the anti-CD47/PD-L1 bispecific antibody

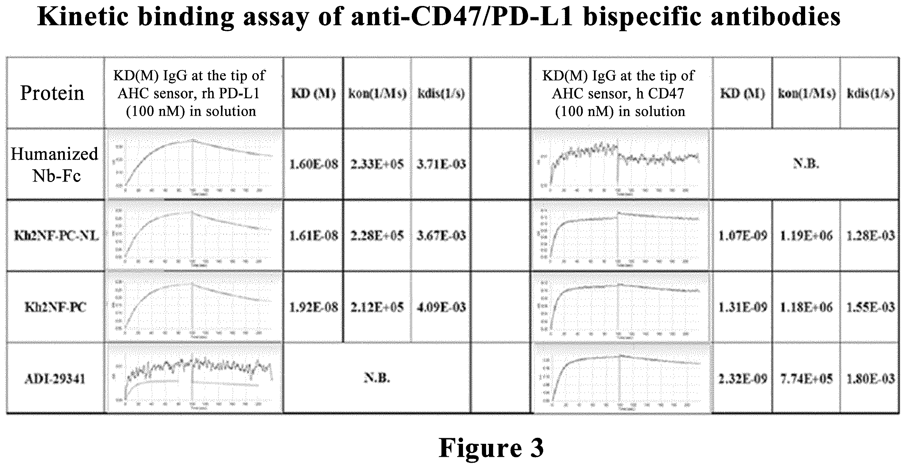

The equilibrium dissociation constants (K_{D}) of the two exemplary anti-CD47/PD-L1 bispecific antibodies Kh2NF-PC and Kh2NF-PC-NL of the present invention binding to CD47 and PD-L1 were measured using a kinetic binding assay using the Octet system (produced by ForteBio). The ForteBio affinity determination was performed according to the method reported in the literature (Estep, P, et al., High throughput solution Based measurement of antibody-antigen affinity and epitope binning. MAbs, 2013, 5 (2): p. 270-278). Briefly, the AHC sensor (Pall, catalog number: 1506091) was immersed in SD buffer (PBS 1 ×, BSA 0.1%, Tween 20 0.05%) and equilibrated at room temperature, half an hour before the experiment. 100 µL of SD buffer as a blank control (for background subtraction), 100 µL of 100 nM purified bispecific antibodies Kh2NF-PC, Kh2NF-PC-NL, and an anti-PD-L1 humanized Nb-Fc antibody (PCT/CN2017/095884) as a control, an anti-CD47 antibody ADI 29341, and 100 µL of solutions of rh PD-L1 (100 nM) and h CD47 (100 nM) (Acrobiosystems) diluted in SD buffer as antigens, were added to the wells of a 96-well black polystyrene half-well microplate (Greiner), respectively. The anti-human IgG Fc biosensor AHC was immersed in each well containing the antibody solution, and immersed at room temperature for 600 seconds to load the samples. The sensor was then washed in SD buffer until it returned to the baseline, and then immersed in a well containing 100 µL of the antigen solution to monitor the association of the antibody with the antigen. The sensor was then transferred to a well containing 100 ul of SD buffer to monitor the antibody dissociation. The rotation speed was 400 rpm and the temperature was 30°C. The background-corrected association and dissociation curves were plotted by the Octet analysis software (ForteBio) to generate the association (kₒₙ) rate constant and dissociation (k_{dis}) rate constant, which are then used to calculate the equilibrium dissociation constant (K_{D}). The kₒₙ, k_{dis} and K_{D} data of the antibodies are shown in Table 2, Table 3 and Fig. 3.

**Table 2. The dissociation constants (K_{D}) of antibodies to rh PD-L1 determined by the ForteBio kinetic binding assay**

| Antibody | Kₒₙ (M⁻¹s⁻¹) | k_{dis} (s⁻¹) | K_{D} (M) |
|---|---|---|---|
| Humanized Nb-Fc | 2.33E+05 | 3.71E -03 | 1.60E-08 |
| Kh2NF-PC-NL | 2.28E+05 | 3.67E -03 | 1.61E-08 |
| Kh2NF -PC | 2.12E+05 | 4.09E -03 | 1.92E-08 |
| ADI 29341 | N.B. | N.B. | N.B. |

| | | | |
|---|---|---|---|
| Note: N.B: Not detectable. | | | |

**Table 3. The dissociation constants (K_{D}) of antibodies to h CD47 determined by the ForteBio kinetic binding assay**

| Antibody | Kₒₙ (M⁻¹s⁻¹) | k_{dis} (s⁻¹) | K_{D} (M) |
|---|---|---|---|
| Humanized Nb-Fc | N.B. | N.B. | N.B. |
| Kh2NF-PC-NL | 1.19E+06 | 1.28E -03 | 1.07E-09 |
| Kh2NF -PC | 1.18E+06 | 1.55E -03 | 1.31E-09 |
| ADI 29341 | 7.74E+05 | 1.80E -03 | 2.32E-09 |

From the above data, it can be seen that the bispecific antibodies Kh2NF-PC and Kh2NF-PC-NL of the present invention can simultaneously bind to PD-L1 and CD47 proteins in solution, and maintain the affinity constant of the parent antibody.

### Example 1.4. Binding assay of the anti-CD47/PD-L1 bispecific antibody of the present invention to CHO cells overexpressing CD47 or PD-L1

The binding of anti-CD47/PD-L1 bispecific antibodies Kh2NF-PC and Kh2NF-PC-NL of the present invention to CD47- or PD-L1-overexpressing CHO cells was determined by FACS.

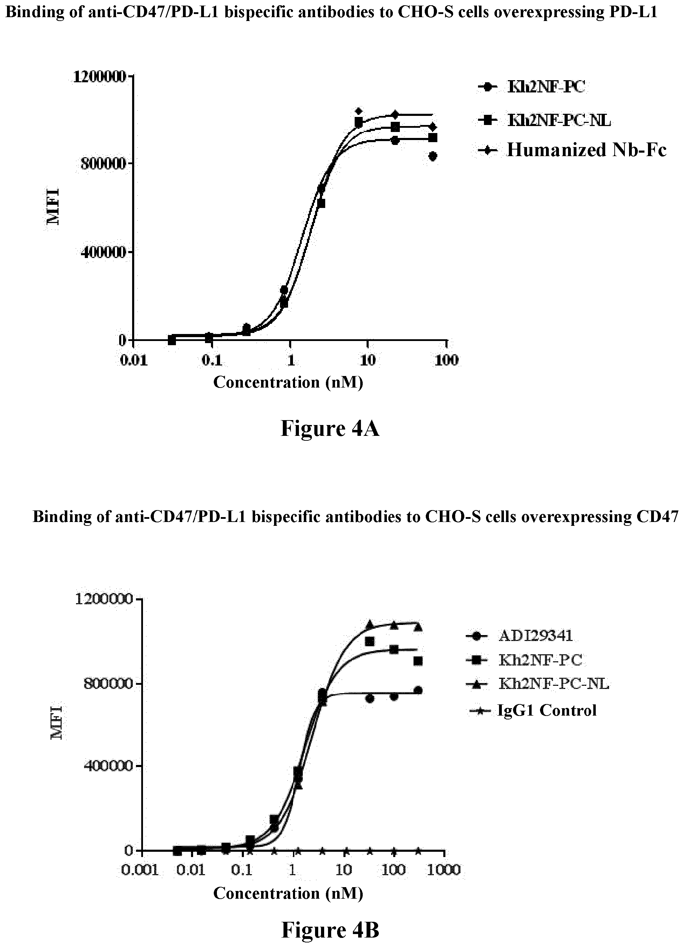

In short, by using the ExpiCHO™ Expression System Kit (Invitrogen, catalog number: A29133) according to the instructions of the manufacturer, the pCHO1.0 vector (Invitrogen) carrying human PD-L1 cDNA (Sino Biological) cloned to a multiple cloning site (MCS) was transfected into Chinese hamster ovary cancer cells (CHO-S) (Invitrogen) to obtain human-PD-L1-overexpressing CHO cells (CHO-S-PD-L1 cells). CHO-S-PD-L1 cells were counted, and the cell suspension was diluted to 1 × 10⁶ cells/ml with cell medium, and then added to a U-bottom 96-well plate at 100 µL/well. The cell suspension was centrifuged at 400 g on a centrifuge for 5 minutes to remove the cell medium. 100 µL of each of serial dilutions of bispecific antibodies Kh2NF-PC and Kh2NF-PC-NL of the present invention and humanized Nb-Fc as a control were added to a U-shaped plate, and then the cells were resuspended and put onto the ice to stand for 30 minutes. The cell suspension was centrifuged at 400 g for 5 minutes, and then the supernatant was removed, and the cells were washed with PBS to remove the unbound antibodies. The obtained cell suspension was centrifuged at 400 g for 5 minutes to remove PBS. 100 µL of the solution of PE-conjugated anti-human Fc antibody (Jackson Immuno Research) diluted at a ratio of 1:200 was added to each well, and the cells were incubated in the dark on the ice for 30 minutes. Then the cell suspension was centrifuged at 400 g for 5 minutes and the supernatant was removed. The cells were washed with PBS to remove the unbound PE-conjugated anti-human Fc antibody. The cells were resuspended with 100 µL of PBS, and the binding of the antibodies to cells was assayed by FACS. The results are shown in FIG. 4A.

It can be seen from FIG. 4A that the bispecific antibodies Kh2NF-PC and Kh2NF-PC-NL of the present invention both can bind to PD-L1 expressed on the surface of cells, and demonstrate the EC50 for binding of parental antibodies.

Likewise, the pCHO1.0 vector (Invitrogen) carrying human CD47 cDNA (Sino Biological) cloned to a multiple cloning site (MCS) was transfected into Chinese hamster ovary cancer cells (CHO-S) (Invitrogen) to obtain human CD47-overexpressing CHO-S cells (CHO-S-CD47 cells).

FACS assay was carried out for CHO-CD47. The experimental operation was the same as the above FACS assay for CHO-S-PD-L1 cells except that a different cell line was used, ADI 29341 antibody was used as a positive control antibody, and IgG1 was used as a negative control antibody. The IgG1 negative control used in the present application has a heavy-chain (HC) amino acid sequence shown as SEQ ID NO: 23 and a light-chain (LC) amino acid sequence shown as SEQ ID NO: 24.

The results are shown in FIG. 4B. It can be seen from FIG. 4B that the bispecific antibodies Kh2NF-PC and Kh2NF-PC-NL of the present invention both can bind to CD47 expressed on the surface of cells, and demonstrate the EC50 for binding of parental antibodies.

### Example 1.5: Solubility assay of anti-CD47/PD-L1 bispecific antibodies of the present invention

Polyethylene glycol (PEG) is a polar nonionic precipitant. The dissolution of anti-CD47/PD-L1 bispecific antibodies of the present invention in PEG of different concentrations was assayed by the PEG precipitation method (Li li, et al., Application of a PEG precipitation method for solubility screening: A tool for developing high protein concentration formulations. Protein Science, 2013.22: p. 1118-23).

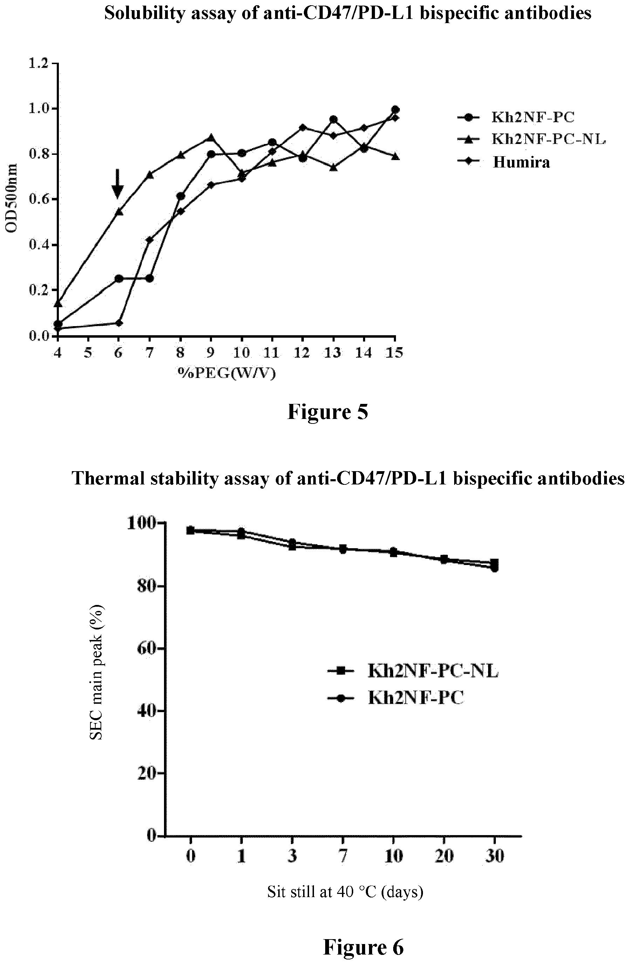

The solutions of anti-CD47/PD-L1 bispecific antibodies Kh2NF-PC and Kh2NF-PC-NL of the present invention were concentrated to 5 mg/ml. The bispecific antibodies were added to a 96-well cell culture plate at 40 µL/well. Humira was used as a positive control. 26.7 µL, 40 µL, 46.7 µL, 53.3 µL, 60 µL, 66.7 µL, 73.3 µL, 80 µL, 86.7 µL, 93.3 µL and 100 µL of 30% PEG6000 (Sigma, catalog number: 81255-250G) were respectively added to the first to eleventh columns of the 96-well cell culture plate from the left to the right. Each well was replenished with PBS to a total volume of 200 µL, so that the final concentration of the antibody in each well was 1 mg/ml, and the PEG concentrations in the first to eleventh columns of the 96-well cell culture plate from the left to the right were respectively 4%, 6%, 7%, 8%, 9%, 10%, 11%, 12%, 13%, 14% and 15%. The 96-well cell culture plate was placed at room temperature for 1 h. Absorbance was determined at OD500 nm. The experimental results are shown in FIG. 5.

It can be seen from FIG. 5 that the anti-CD47/PD-L1 bispecific antibody Kh2NF-PC of the present invention has similar solubility to Humira

### Example 1.6: Thermal stability assay of anti-CD47/PD-L1 bispecific antibodies of the present invention

With Differential scanning fluorimetry (DSF), information about protein structure stability can be provided according to the process of fluorescence change in a protein atlas, the configuration change of a protein can be detected, and the melting temperature (Tₘ) of a protein can be obtained. DSF is employed in the present example to determine the Tₘ values of anti-CD47/PD-L1 bispecific antibodies of the present invention.

The solutions of anti-CD47/PD-L1 bispecific antibodies Kh2NF-PC and Kh2NF-PC-NL of the present invention were diluted with a PBS solution to 1 mg/ml.

196 µL of PBS was added to 4 µL of SYPRO Orange Protein Gel Stain (Gibco, catalog number: S6650), so that SYPRO Orange Protein Gel Stain was 50-fold diluted.

50 µL of the aforementioned solution of the bispecific antibody with a concentration of 1 mg/ml, 10 µL of the aforementioned 50-fold-diluted SYPRO Orange Protein Gel Stain and 40 µL of water were added to each well of a 96-well PCR plate (Nunc) in sequence. The 96-well PCR plate was put into a 7,500 Real Time PCR system (Themro Fisher) for assay. The temperature of the system was set to rise by 0.5 °C/min, and the temperature when a peak absolute value appeared in a fluorescence curve was Tₘ of the protein.

The experimental results are shown in Table 4 below. The bispecific antibodies of the present invention have three Tₘ values each higher than 55°C, and thus have good thermal stability.

**Table 4.**

| | Tm1 | Tm2 | Tm3 |
|---|---|---|---|
| Kh2NF-PC | 57.80 | 64.05 | 76.95 |
| Kh2NF-PC-NL | 55.90 | 62.54 | 76.76 |

Further, the changes of the purities and biological activities of the bispecific antibodies of the present invention were studied 10, 20 and 30 days after the antibodies were placed at 40 °C, in order to evaluate the long-term thermal stabilities of the antibodies. Specifically speaking, the solutions of anti-CD47/PD-L1 bispecific antibodies Kh2NF-PC and Kh2NF-PC-NL of the present invention in PBS were concentrated to 5 mg/ml, then aliquoted into EP tubes at 200 µL/tube and placed in the dark at 40 °C.

The purity of the anti-CD47/PD-L1 bispecific antibody of the present invention was determined by SEC-HPLC with one tube of sample on day 0, day 1, day 3, day 7, day 10, day 20 and day 30 of standing at 40 °C. The experimental results are shown in FIG. 6. The anti-CD47/PD-L1 bispecific antibodies of the present invention still had high purities even after being placed at 40 °C for up to 30 days.

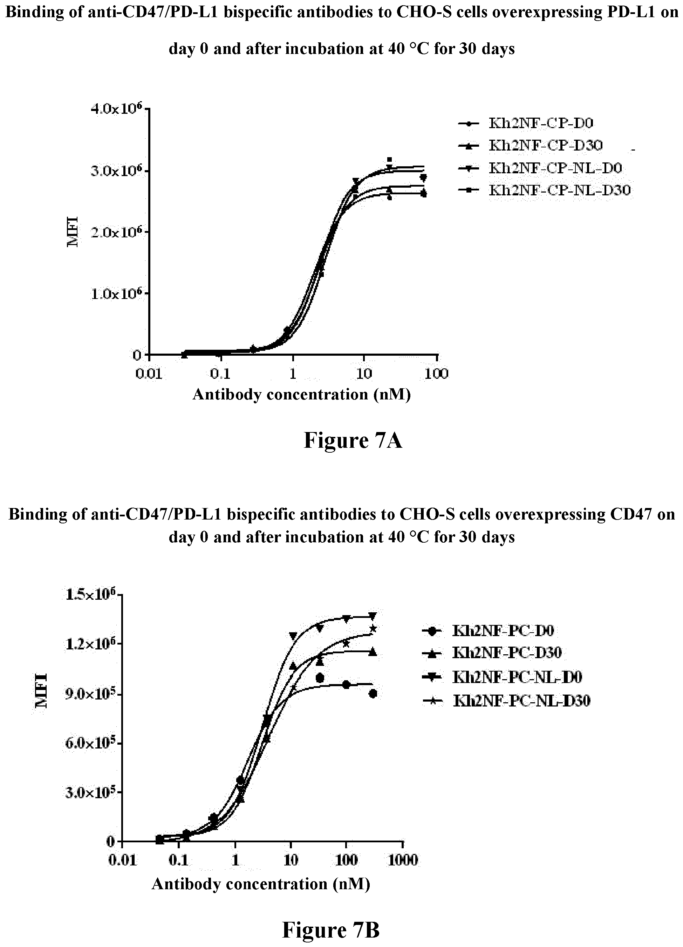

In addition, as described in example 1.4 above, the binding activities of the anti-CD47/PD-L1 bispecific antibodies of the present invention with CD47- or PD-L1-expressing CHO-S cells were assayed on day 0 and day 30. The experimental results are shown in FIG. 7A and FIG. 7B.

It can be seen from FIG. 6, FIG. 7A and FIG. 7B that the bispecific antibodies of the present invention have good long-term thermal stability and can retain their biological activities.

### Example 1.7: MOA-based Assay of Anti-PD-L1 Activities of Bispecific Antibodies of the Present Invention

Considering that the exploration of an antibody should be based on the understanding of the mechanisms of action (MOA) and the biological activity of the antibody, PD-1/PD-L1 Blockade Bioassay, Cell Propagation Model (Promega) was adopted in the present example to study the anti-PD-L1 biological activities of the bispecific antibodies of the present invention.

As an MOA-based determination method related to biology, PD-1/PD-L1 Blockade Bioassay of Promega can be used to determine the potency and stability of an antibody capable of blocking PD-1/PD-L1 interaction. This determination method comprises two types of genetically engineered cell lines:
- PD-1 effector cells: Jurkat T cells expressing luciferase induced by the nuclear factor of activated T cells (NFAT) and stably expressing human PD-1.
- PD-L1 aAPC/CHO-K1 cells: CHO-K1 cells stably expressing human PD-L1 and activating cell surface proteins of related TCRs in an antigen-independent manner.

The binding of PD-1 to PD-L1 can block the transduction of NFAT downstream signal, thereby inhibiting the expression of luciferase. When the PD-1 antibody or the PD-L1 antibody is added, such blocking effect is reversed, and luciferase is expressed, and as a result, a fluorescence signal is detected. This determination method has good sensitivity, specificity, accuracy and stability.

An assay was carried out according to the product instructions of the manufacturer. 1-2 days before the MOA method was conducted, PD-L1 aAPC/CHO-K1 cells were subcultured. The culture supernatant was discarded, and the cells were washed with PBS (Gibco). An appropriate amount of trypsin (Gibco) was added to digest the cells for 3 to 5 min at 37 °C/5% CO₂. Then, culture medium of a volume 4 times that of trypsin was added, and the cells were transferred into a 50 ml centrifuge tube and counted. A needed volume of cell suspension was taken and centrifuged at 230 g for 10 min. The cells were resuspended to 4 × 10⁵ cells/ml with RPMI 1640 medium (Gibco). The cell suspension was added to a 96-well white cell culture plate (Nunclon) at 100 µl/well, and replenished with a PBS solution to 200 µl/well. The cells were cultured overnight in an incubator at 37 °C/5% CO₂.

Likewise, 1-2 days before the MOA method was conducted, PD-1 effector cells were subcultured. The cells were counted, and a needed volume of cell suspension was taken and centrifuged at 170 g for 5 min. The cells were resuspended to 1.25 × 10⁶ cells/ml with assay buffer (RPMI 1640 medium + 1% FBS).

95 µl/well of PD-L1 aAPC/CHO-K1 cell culture supernatant was discarded. Then, 40 µl of each tested antibody, an anti-PD-L1 humanized Nb-Fc antibody as a control and an IgG1 as a negative control were added. 40 µl of PD-1 effector cell suspension prepared above was added to each well of the assay plate. The cells were cultured in an incubator at 37 °C/5% CO₂ for 6 h.

Bio-Glo™ buffer (Promega) was thawed and added with Bio-Glo™ substrate, and the mixture was well mixed. The obtained Bio-Glo™ reagent was added to the wells of the aforementioned assay plate at 80 µl/well after 6 h of incubation. The assay plate was placed at room temperature for 5 to 10 min, and the fluorescence signal values were read.

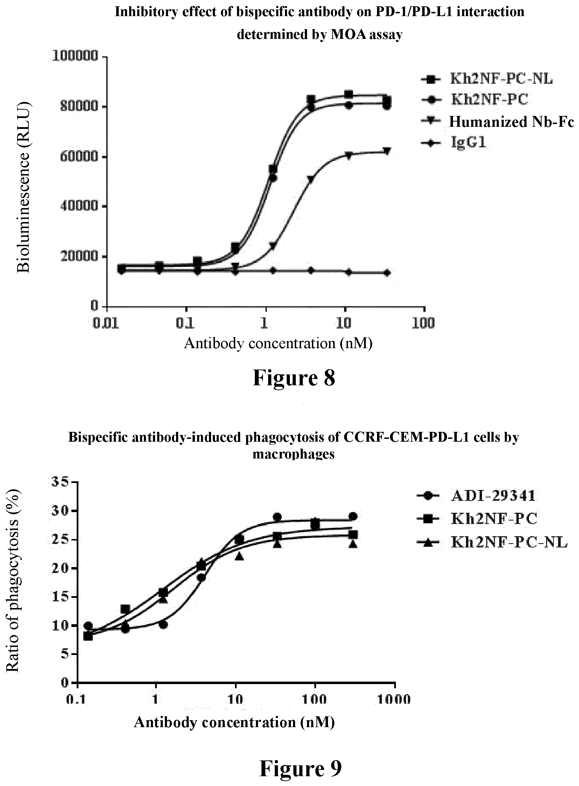

The results are shown in FIG. 8. The bispecific antibodies of the present invention can reverse the inhibitory effect of PD-1/PD-L1 interaction on the NFAT signaling pathway, and are superior to the humanized Nb-Fc antibody alone used as an anti-PD-L1 antibody in terms of activity.

### Example 1.8: Assay of ability of Bispecific Antibodies of the Present Invention to promote the Phagocytosis of Tumor Cells by Macrophages

The abilities of antibodies Kh2NF-PC and Kh2NF-PC-NL of the present invention to promote the phagocytosis of tumor cells by macrophages were determined by a flow cytometry-based assay method.

Density gradient centrifugation was carried out for fresh blood taken from a donor to obtain peripheral blood mononuclear cells (PBMCs). The isolated PBMCs were purified according to the scheme of the instructions of EasySep™ Human CD14 Positive Selection Kit (STEMCELL Technologies) to obtain CD14-positive mononuclear cells. In short, 10 ng/mL of granulocyte-macrophage colony-stimulating factor (GM-CSF, R&D Systems) was added, and the cells were incubated adherently for 7 days, wherein, on day 5, 20 ng/mL of interferon-y (IFN-γ, AcroBiosystem) was added for 1 h of stimulation, 100 ng/mL of lipopolysaccharide (LPS, Sigma) was then added for 48 h of stimulation, and the precursor cells among the peripheral blood mononuclear cells were induced to differentiate into macrophages.

Human PD-L1 was transferred into tumor cells CCRF-CEM (purchased from ATCC) by electroporation to construct target tumor cells CCRF-CEM-PD-L1. The target tumor cells CCRF-CEM-PD-L1 were fluorescently labeled with fluorochrome, carboxyfluorescein diacetate, succinimidyl ester (CFSE), according to the instructions of CellTrace™ CFSE Cell Proliferation Kit (Invitrogen, catalog number: C34554). The labeled tumor cells were co-cultured with the aforementioned completely-differentiated macrophages at a ratio of 4:1 in terms of cell number, and meanwhile, tested antibody solutions of different concentrations were added. The cells were incubated at 37 °C for 3 h. The cells were then washed at least twice, added with CD14 antibody (purchased from BD) labeled by allophycocyanin (APC), and incubated in PBS containing 0.1% BSA on the ice (in the dark) for 30 min. The cells were washed at least twice with PBS and analyzed by flow cytometry. The phagocytized cell population was the CD14-positive and CFSE-positive cell population among the living cells. The experimental results are shown in FIG. 9.

It can be seen from FIG. 9 that the anti-CD47/PD-L1 bispecific antibodies can effectively induce macrophages to phagocytize the target cells co-expressing CD47 and PD-L1, and have similar inducction activities to that of anti-CD47 monoclonal antibodies.

### Example 1.9: Assay of Activities of Anti-CD47/PD-L1 Bispecific Antibodies of the Present Invention in Promoting Erythrocyte Agglutination

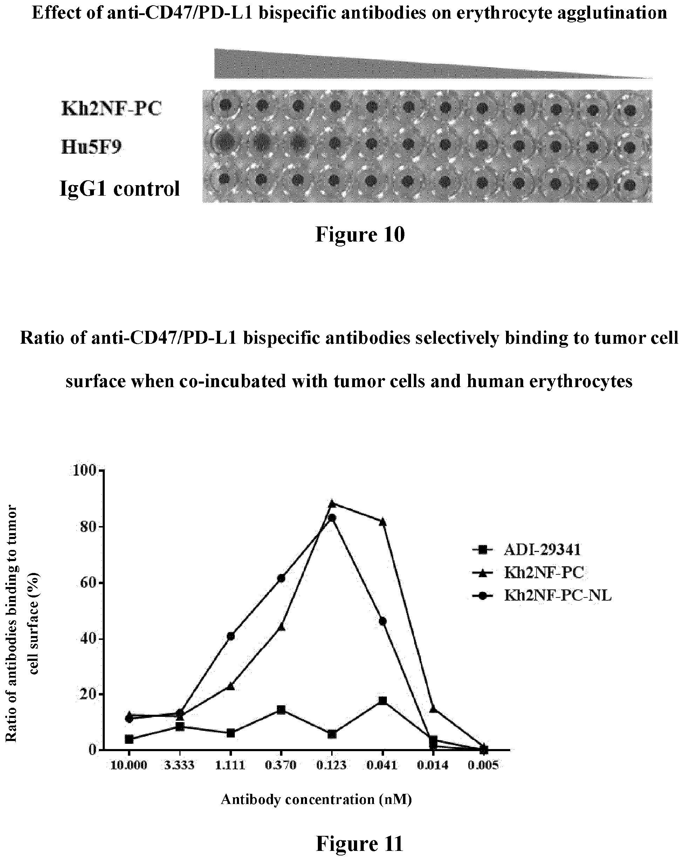

Most of the anti-CD47 antibodies known in the prior art have the side effect of promoting erythrocyte agglutination, so the therapeutic use of these anti-CD47 antibodies is limited. For this reason, the inventors further studied the influence of the anti-CD47/PD-L1 bispecific antibodies of the present invention on erythrocyte agglutination. The specific assay method is as follows:
Fresh human blood was collected, washed three times with PBS and then prepared into 10% human red blood cell suspension. The human red blood cells and the tested antibodies (the highest concentration was 60 ug/ml, and three-fold serial dilution was conducted to obtain 11 diluted concentrations in total) were incubated at 37 °C for 2-6 h, and the reaction took place in a 96-well round-bottom plate. After the reaction was completed, pictures were taken, and results were observed. The result determination standard was as follows: If the red blood cells sunk at the well bottom and spread out like a net, then erythrocyte agglutination occurred (see the result of Hu5F9 (the "5F9" antibody in US2015/0183874 A1) in FIG. 10); and if the red blood cells sunk at the well bottom as dots, then erythrocyte agglutination did not occur (see the IgG1 control in FIG. 10).

In the assay carried out by the above determination method, the erythrocyte agglutination results are shown in FIG. 10. The activity of the antibody Kh2NF-PC of the present invention in promoting erythrocyte agglutination is very low, and is significantly lower than that of the control group Hu5F9. It can be seen that the anti-CD47/PD-L1 bispecific antibodies of the present invention have a significantly reduced effect in promoting erythrocyte agglutination, resulting in significantly reduced side effects in clinical treatment, and therefore can be widely used in the treatment of a variety of cancers.

### Example 1.10: Study on Selective Binding Activities of Anti-CD47/PD-L1 Bispecific Antibodies of the Present Invention to Tumor Cells

As CD47 protein is expressed on the surface of normal erythrocytes in the human body, most of anti-CD47 monoclonal antibodies tend to bind to normal erythrocytes, and this is one of the main reasons causing the side effects of anti-CD47 monoclonal antibodies. Tumor cells and human red blood cells were co-incubated in the present example to assay the selective binding activities of bispecific antibodies of the present invention to tumor cells. The specific experimental process was as follows:
50 ml of fresh blood was collected from a donor, and added with PBS of 2.5-fold volume. The mixture was then gently added to 4 tubes each with 12.5 ml of FiColl (Thermo). The samples were centrifuged at 400 g for 30 min with zero deceleration. The upper serum and PBMC were removed to obtain red blood cells in the lowest layer.

Tumor cells H292 (ATCC) were fluorescently labeled with fluorochrome CFSE according to the instructions of CellTrace™ CFSE Cell Proliferation Kit (Invitrogen, catalog number: C34554). The labeled tumor cells were co-cultured with the aforementioned isolated human red blood cells at a ratio of 1:20 in terms of cell number, and meanwhile, tested antibody solutions of different concentrations were added. The cells were incubated at 4 °C for 30 min. The cells were then washed at least twice, added with human Fc antibody (purchased from Biolegend, catalog number: 409306) labeled by allophycocyanin (APC), and incubated in PBS containing 0.1% BSA on the ice (in the dark) for 30 min. The cells were washed at least twice with PBS and analyzed by flow cytometry.

The experimental results are shown in table 5 and FIG. 11. At the antibody concentrations of 1.111 nM to 0.041 nM, the bispecific antibodies Kh2NF-PC and Kh2NF-PC-NL of the present invention are more inclined to bind to tumor cells than the anti-CD47 monoclonal antibody ADI-29341 (at a concentration of 0.12 nM, 88.51% of Kh2NF-PC and 83.24% of Kh2NF-PC-NL bind to the surface of H292 cells, while only 5.74% of ADI-29341 binds to the surface of H292 cells).

**Table 5: Proportions of Anti-CD47/PD-L1 Bispecific Antibodies binding to H292 Cells**

| Antibody concentration (nM) | 10.000 | 3.333 | 1.111 | 0.370 | 0.123 | 0.041 | 0.014 |
|---|---|---|---|---|---|---|---|
| ADI-29341 | 3.90% | 8.42% | 6.15% | 14.37% | 5.74% | 17.68% | 3.59% |
| Kh2NF -PC | 12.53% | 12.10% | 22.98% | 44.47% | 88.51% | 81.97% | 14.93% |
| Kh2NF-PC-NL | 11.27% | 13.22% | 40.95% | 61.60% | 83.24% | 46.32% | 1.41% |

### Example 1.11: In-vivo Anti-tumor Effect of Anti-CD47/PD-L1 Bispecific Antibodies of the Present Invention

In the present example, NOD-SCID mice were inoculated with Raji-PD-L1 cells to obtain tumor-bearing mice, and the anti-tumor effect of anti-CD47/PD-L1 antibodies of the present invention were determined with these mice.

### NOD-SCID Mice:

Female NOD-SCID mice (42-62 days old) were purchased from Beijing Vital River Laboratory Animal Technology Co., Ltd. The grade: SPF; the number: 100; the quality inspection organization: Beijing Vital River Laboratory Animal Technology Co., Ltd; and the certificate number: NO. 11400700284978. Before the study, the mice were adapted for 7 days after arrival.

### Cell Culture and Mouse Inoculation:

PCHO1.0 vector (Invitrogen) carrying human PD-L1 cDNA (Sino Biological) cloned to a multiple cloning site (MCS) was transfected into Raji host cells purchased from ATCC, and Raji cells stably expressing human PD-L1 (Raji-PD-L1 cells) were obtained through pressure screening. The Raji-PD-L1 cells were subcultured conventionally for subsequent in-vivo study on the mice.

The cultured Raji-PD-L1 cells were collected by centrifugation, and the cells were resuspended with 1×PBS. The Raji-PD-L1 cells with a cell density of 10 × 10⁶ cells/ml were mixed with Matrigel gel (Corning, catalog number: 356231) at a ratio of 1:1 to obtain a cell suspension with a cell density of 5 × 10⁶ cells/ml. On day 0, 0.2 ml of the cell suspension was subcutaneously inoculated into the right abdominal region of the NOD-SCID mice to establish Raji-PD-L1 tumor-bearing mouse models.

### Grouping and Administration of Tumor-bearing Mice:

The tumor volume of each mouse was measured 8 days after the tumor cells were inoculated, and the mice with a tumor volume ranging from 50.56 mm³ to 115.39 mm³ were grouped in a serpentine manner according to the tumor volume(6 mice in each group). Dosages of and route of administration are shown in Table 6. h-IgG (purchased from Equitech-Bio, batch number: 160308-02, specification: 1g/vial, and prepared to 10 mg/ml with PBS) was used as a negative control. Specifically speaking, the mice were administrated on day 8, day 10, day 12, day 14, day 16, day 18 and day 20 after Raji-PD-L1 cell inoculation, and the tumor volume and the weight of the mice were monitored two to three times a week. The monitoring ended 33 days after inoculation. On day 33 after inoculation, tumor growth inhibition (TGI%) was calculated by the following formula: TGI% = 100% ^{∗} (h-IgG control group tumor volume - treatment group tumor volume)/(h-IgG control group tumor volume - h-IgG control group tumor volume before administration). The average tumor volume of the h-IgG control group before administration was 80.86 mm³. Tumor volume measurement: The maximum length of major axis (L) and maximum length of minor axis (W) of tumors were measured with a vernier caliper, and tumor volume was calculated using the following formula: V = L × W²/2. The mice were weighted using an electronic balance.

**Table 6. Experiment Design Table**

| Group | Dosage of Administration | Number of Administration | Route of administration |
|---|---|---|---|
| h-IgG | 0.2mg/kg | Q2Dx7 | Intraperitoneal |
| Humanized Nb-Fc | 0.1mg/kg | Q2Dx7 | Intraperitoneal |
| ADI-29341 | 0.05mg/kg | Q2Dx7 | Intraperitoneal |
| Humanized Nb-Fc + ADI-29341 | 0.1mg/kg +0.1 mg/kg | Q2Dx7 | Intraperitoneal |
| Kh2NF-PC | 0.17mg/kg | Q2Dx7 | Intraperitoneal |

| | | | |
|---|---|---|---|
| *Administration is conducted every two days, seven times in total. | | | |

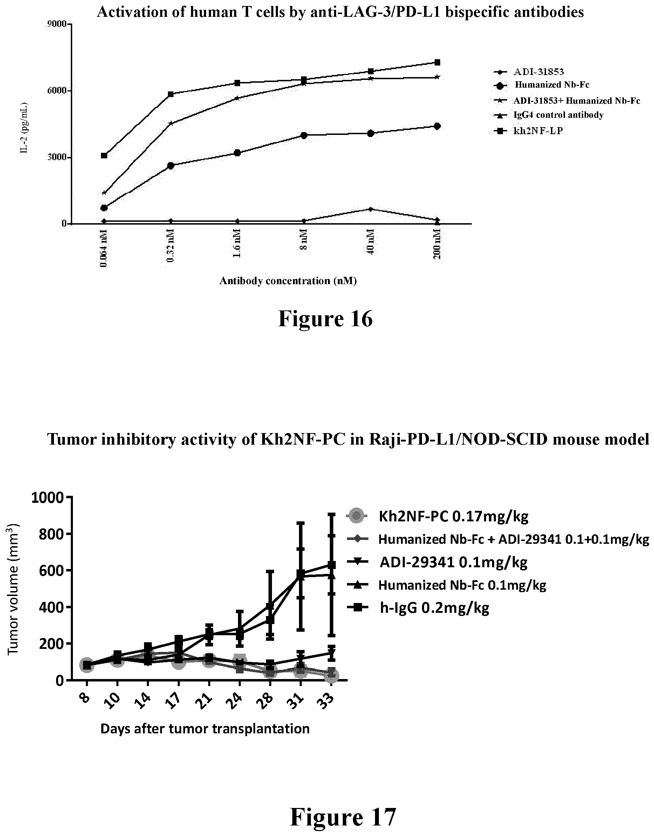

The results of tumor growth inhibition are shown in FIG. 17 and Table 7. On day 33 after inoculation, compared with h-IgG, the tumor growth inhibition of 0.1 mg/kg of ADI-29341 alone is 88%; the tumor growth inhibition of 0.1 mg/kg of ADI-29341 used in combination with 0.1 mg/kg of humanized Nb-Fc is 107%; and the tumor growth inhibition of 0.17 mg/kg of anti-CD47/PD-L1 bispecific antibody Kh2NF-PC is 111%, significantly superior to that of 0.1 mg/kg of humanized Nb-Fc (10%). Therefore, the anti-CD47/PD-L1 bispecific antibody Kh2NF-PC has the best tumor inhibition effect. The results of the mouse weight measurement show that there is no significant difference among groups in terms of the mouse weight.

**Table 7: Tumor Growth Inhibition On Day 33**

| Group | Tumor Volume (mm³) | Tumor Growth Inhibition (%) | Number of Mice Having Complete Regression |
|---|---|---|---|
| h-IgG | 628.98 | N/A | N/A |
| Humanized Nb-Fc (0.1 mg/kg) | 573.65 | 10% | 2 |
| ADI-29341 0.1mg/kg | 144.57 | 88% | 1 |
| Humanized Nb-Fc + ADI-29341 (0.1 + 0.1 mg/kg) | 40.34 | 107% | 3 |
| Kh2NF-PC 0.17mg/kg | 22.86 | 111% | 3 |

### Example 2: Construction, Expression, Purification and Property Identification of Anti-4-1BB/PD-L1 Bispecific Antibody

### Example 2.1: Construction of Anti-4-1BB/PD-L1 Bispecific Antibody

In the present example, an anti-4-1BB/PD-L1 bispecific antibody, named as bispecific antibody Kh2NF-P4, was constructed, and the structural schematic diagram of this process is shown in FIG. 1A.

It can be seen from the structural schematic diagram of FIG. 1A that the bispecific antibody Kh2NF-P4 is composed of three polypeptide chains. Polypeptide chain #1 has an amino acid sequence shown as SEQ ID NO: 25, comprising a VH amino acid sequence derived from anti-4-1BB antibody BMS-663513 and shown as SEQ ID NO: 26, a CHI amino acid sequence derived from human IgG1 and shown as SEQ ID NO: 6 at C-terminus of the VH amino acid sequence and a Fc region amino acid sequence derived from human IgG1 and shown as SEQ ID NO: 7 at C-terminus of the CHI amino acid sequence; polypeptide chain #2 has an amino acid sequence shown as SEQ ID NO: 27, comprising a VL amino acid sequence derived from anti-4-1BB antibody BMS-663513 and shown as SEQ ID NO: 28 and a human κ light chain constant region (CL) amino acid sequence shown as SEQ ID NO: 13 at C-terminus of the VL amino acid sequence; and polypeptide chain #3 has an amino acid sequence shown as SEQ ID NO: 14, comprising a first and second anti-PD-L1 VHH amino acid sequences shown as SEQ ID NO: 16, a linker peptide amino acid sequence shown as SEQ ID NO: 20 between the first anti-PD-L1 VHH amino acid sequence and the second anti-PD-L1 VHH amino acid sequence and a Fc region amino acid sequence derived from human IgG1 and shown as SEQ ID NO: 21 at C-terminus of the second anti-PD-L1 VHH amino acid sequence.

The three chains of the anti-4-1BB/PD-L1 bispecific antibody Kh2NF-P4 were constructed as follows: The C-terminus of the VH of the anti-4-1BB antibody BMS-663513 was connected to the N-terminus of the constant region of human IgG1 to obtain polypeptide chain #1, wherein the Fc region contained LALA mutation to weaken the effector function of the antibody of the present invention, and contained "konb-in-hole" mutation to stably associate with polypeptide chain #3; polypeptide chain #2 was derived from the VL of BMS-663513 and human κ light chain constant region; and polypeptide chain #3 contained a first and second anti-PD-L1 VHHs connected in series, the two anti-PD-L1 VHHs were connected by a flexible peptide of 20 amino acid residues (G₄S)₄, and polypeptide chain #3 was obtained by connecting the C-terminus of the second anti-PD-L1 VHH to the N-terminus of the Fc region amino acid sequence derived from IgG1, wherein the Fc region contained LALA mutation to weaken the effector function of the antibody of the present invention, and contained "knob-in-hole" mutation to stably associate with polypeptide chain #1.

### Example 2.2: Expression and Purification of Anti-4-1BB/PD-L1 Bispecific Antibody

In the present example, the nucleotide sequences coding the three chains of the anti-4-1BB/PD-L1 bispecific antibody Kh2NF-P4 constructed in example 2.1 were transferred into the eukaryotic expression vector pTT5 (Biotechnology Research Institute; Montreal, Canada) through a multiple cloning site, and the bispecific antibody Kh2NF-P4 was expressed and purified in eukaryotic cells.

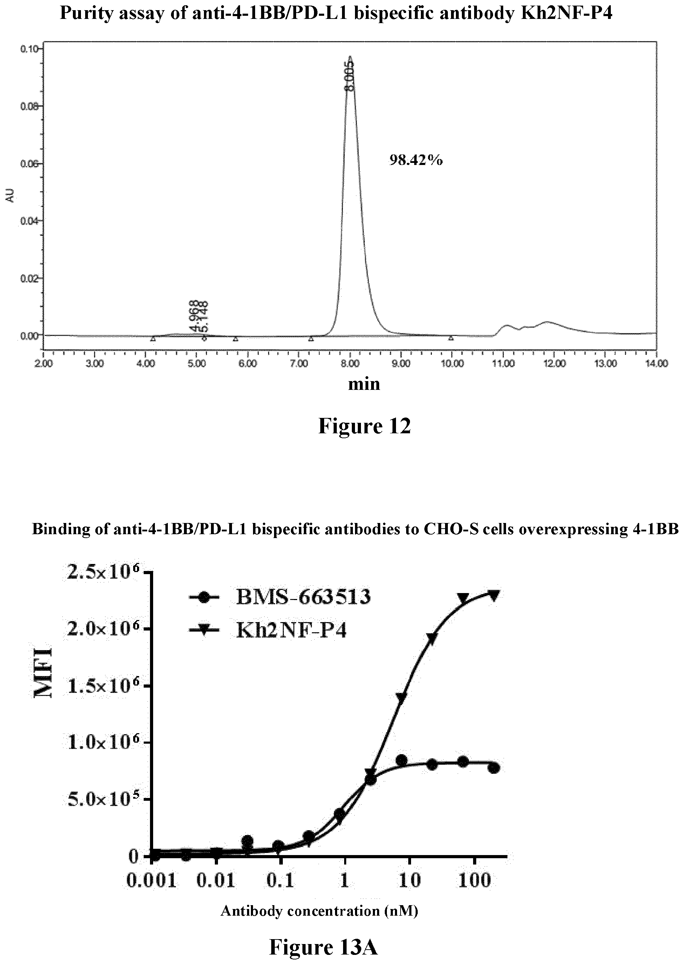

Operations for plasmid transfection as well as expression and purification of the anti-4-1BB/PD-L1 bispecific antibody Kh2NF-P4 are the same as those in example 1.2 above. The SEC result of the bispecific antibody Kh2NF-P4 is shown in FIG. 12, with a purity of 98.42%.

### Example 2.3: Analysis on Binding of the Anti-4-1BB/PD-L1 Bispecific Antibody of the Present Invention to 4-1BB- or PD-L1-Expressing CHO Cells

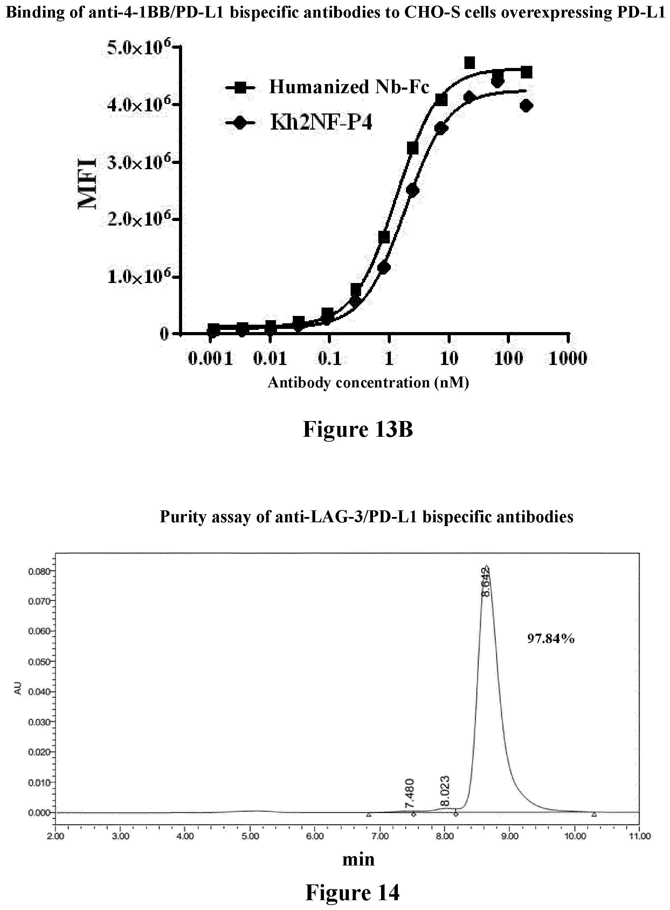

Substantially as described in example 1.4 above, the binding of the anti-4-1BB/PD-L1 bispecific antibody Kh2NF-P4 of the present invention to 4-1BB- or PD-L1-expressing CHO cells was determined by FACS. In short, CHO-S-PD-L1 cells were counted, and the cell suspension was diluted to 1 × 10⁶ cells/ml with cell medium, and then added to a U-bottom 96-well plate at 100 µl/well. The cell suspension was centrifuged at 400 g on a centrifuge for 5 minutes to remove the cell medium. 100 µl of each of serial dilutions of the bispecific antibody Kh2NF-P4 of the present invention and humanized Nb-Fc as a control were added to a U-shaped plate, and then the cells were resuspended and put onto the ice to stand for 30 min. The cell suspension was centrifuged at 400 g for 5 minutes, and then the supernatant was removed, and the cells were washed with PBS to remove the unbound antibodies. The obtained cell suspension was centrifuged at 400 g for 5 min to remove PBS. 100 µl of the solution of PE-conjugated anti-human Fc antibody (Jackson Immuno Research) diluted at a ratio of 1:200 was added to each well, and the cells were incubated in the dark on the ice for 30 min. Then the cell suspension was centrifuged at 400 g for 5 min and the supernatant was removed. The cells were washed with PBS to remove the unbound PE-conjugated anti-human Fc antibody. The cells were resuspended with 100 µl of PBS, and the binding of the antibodies to cells was assayed by FACS. The results are shown in FIG. 13B.

It can be seen from FIG. 13B that the bispecific antibody Kh2NF-P4 of the present invention can bind to PD-L1 expressed on the surface of cells, and has a binding ability similar to that of the parent antibody.

Likewise, the pCHO1.0 vector (Invitrogen) carrying human 4-1BB cDNA (Sino Biological) cloned to a multiple cloning site (MCS) was transfected into Chinese hamster ovary cancer cells (CHO-S) (Invitrogen) to obtain human 4-1BB-overexpressing CHO-S cells (CHO-S-4-1BB cells). FACS assay was carried out for CHO-S-4-1BB. The experimental operation was the same as the above FACS assay for CHO-S-PD-L1 cells except that a different cell line was used and BMS-663513 antibody was used as a control antibody. The results are shown in FIG. 13A.

It can be seen from FIG. 13A that the bispecific antibody Kh2NF-P4 of the present invention can bind to 4-1BB expressed on the surface of cells, and has a binding ability similar to that of the parent antibody.

### Example 3: Construction, Expression, Purification and Property Identification of Anti-LAG-3/PD-L1 Bispecific Antibody

### Example 3.1: Construction of Anti-LAG-3/PD-L1 Bispecific Antibody

In the present example, an anti-LAG-3/PD-L1 bispecific antibody, named as bispecific antibody Kh2NF-PL, was constructed, and the structural schematic diagram of this process is shown in FIG. 1B.

It can be seen from the structural schematic diagram of FIG. 1B that the bispecific antibody Kh2NF-PL is composed of three polypeptide chains. Polypeptide chain #1 has an amino acid sequence shown as SEQ ID NO: 29, comprising a VH amino acid sequence derived from anti-LAG-3 antibody ADI-31853 and shown as SEQ ID NO: 30, a CHI amino acid sequence derived from human IgG1 and shown as SEQ ID NO: 6 at C-terminus of the VH amino acid sequence and a Fc region amino acid sequence derived from human IgG1 and shown as SEQ ID NO: 7 at C-terminus of the CHI amino acid sequence; polypeptide chain #2 has an amino acid sequence shown as SEQ ID NO: 34, comprising a VL amino acid sequence derived from anti-LAG-3 antibody ADI-31853 and shown as SEQ ID NO: 35 and a human κ light chain constant region (CL) amino acid sequence shown as SEQ ID NO: 13 at C-terminus of the VL amino acid sequence; and polypeptide chain #3 has an amino acid sequence shown as SEQ ID NO: 22, comprising a first and second anti-PD-L1 VHH amino acid sequences shown as SEQ ID NO: 16, with no linker peptide amino acid sequence between the first anti-PD-L1 VHH amino acid sequence and the second anti-PD-L1 VHH amino acid sequence; and a Fc region amino acid sequence derived from human IgG1 and shown as SEQ ID NO: 21 at C-terminus of the second anti-PD-L1 VHH amino acid sequence.

The three chains of the anti-LAG-3/PD-L1 bispecific antibody Kh2NF-PL of the present invention were constructed as follows: The C-terminus of the VH of the anti-LAG-3 antibody ADI-31853 was connected to the N-terminus of the constant region of human IgG1 to obtain polypeptide chain #1, wherein the Fc region contained LALA mutation to weaken the effector function of the antibody of the present invention, and contained "konb-in-hole" mutation to stably associate with polypeptide chain #3; polypeptide chain #2 was derived from the VL of ADI-31853 and human κ light chain constant region; and polypeptide chain #3 contained a first and second anti-PD-L1 VHHs connected in series, there was no linker peptide between the two anti-PD-L1 VHHs, and polypeptide chain #3 was obtained by connecting the C-terminus of the second anti-PD-L1 VHH to the N-terminus of the Fc region amino acid sequence derived from IgG1, wherein the Fc region contained LALA mutation to weaken the effector function of the antibody of the present invention, and contained "knob-in-hole" mutation to stably associate with polypeptide chain #1.

### Example 3.2: Expression and Purification of Anti-LAG-3/PD-L1 Bispecific Antibody

In the present example, the nucleotide sequences coding the three chains of the anti-LAG-3/PD-L1 bispecific antibody Kh2NF-PL constructed in example 3.1 were transferred into the eukaryotic expression vector pTT5 (Biotechnology Research Institute; Montreal, Canada) through a multiple cloning site, and the bispecific antibody Kh2NF-PL was expressed and purified in eukaryotic cells.

Operations for plasmid transfection as well as expression and purification of the LAG-3/PD-L1 bispecific antibody Kh2NF-PL are the same as those in example 1.2 above. The SEC result of the bispecific antibody Kh2NF-PL is shown in FIG. 14, with a purity of 97.84%.

### Example 3.3: Analysis on Binding of the Anti-LAG-3/PD-L1 Bispecific Antibody of the Present Invention to LAG-3- or PD-L1-Expressing Cells

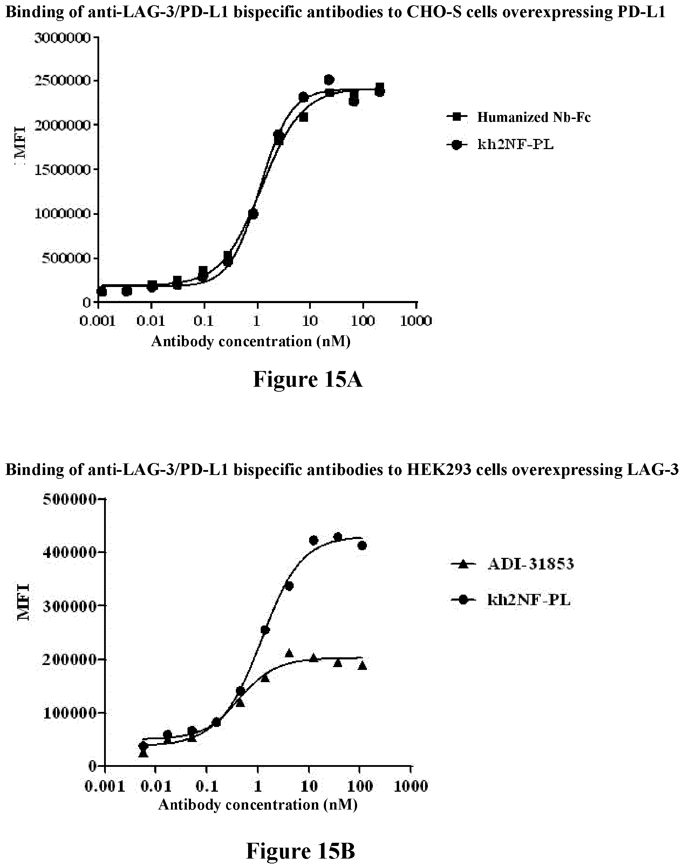

Substantially as described in example 1.4 above, the binding of the anti-LAG-3/PD-L1 bispecific antibody Kh2NF-PL of the present invention to LAG-3- or PD-L1-expressing cells was determined by FACS. In short, CHO-S-PD-L1 cells were counted, and the cell suspension was diluted to 1 × 10⁶ cells/ml with cell medium, and then added to a U-bottom 96-well plate at 100 µl/well. The cell suspension was centrifuged at 400 g on a centrifuge for 5 minutes to remove the cell medium. 100 µl of each of serial dilutions of the bispecific antibody Kh2NF-PL of the present invention and anti-PD-L1 humanized Nb-Fc as a control were added to a U-shaped plate, and then the cells were resuspended and put onto the ice to stand for 30 min. The cell suspension was centrifuged at 400 g for 5 minutes, and then the supernatant was removed, and the cells were washed with PBS to remove the unbound antibodies. The obtained cell suspension was centrifuged at 400 g for 5 min to remove PBS. 100 µl of the solution of PE-conjugated anti-human Fc antibody (Jackson Immuno Research) diluted at a ratio of 1:200 was added to each well, and the cells were incubated in the dark on the ice for 30 min. Then the cell suspension was centrifuged at 400 g for 5 min and the supernatant was removed. The cells were washed with PBS to remove the unbound PE-conjugated anti-human Fc antibody. The cells were resuspended with 100 µl of PBS, and the binding of the antibodies to cells was assayed by FACS. The results are shown in FIG. 15A.

It can be seen from FIG. 15A that the bispecific antibody Kh2NF-PL of the present invention can bind to PD-L1 expressed on the surface of cells, and has a binding ability similar to that of the parent antibody.

The pCHO1.0 vector (Invitrogen) carrying human LAG-3 cDNA (Sino Biological) cloned to a multiple cloning site (MCS) was transfected into HEK293 cells (Invitrogen) to obtain human LAG-3-overexpressing HEK293 cells (293-LAG-3 cells).

Likewise, FACS assay was carried out for 293-LAG-3 cells. The experimental operation was the same as the above FACS assay for CHO-S-PD-L1 cells except that a different cell line was used and anti-LAG-3 antibody ADI-31853 was used as a control antibody. The results are shown in FIG. 15B.

It can be seen from FIG. 15B that the bispecific antibody Kh2NF-PL of the present invention can bind to LAG-3 expressed on the surface of cells, and has a binding ability similar to that of the parent antibody.

### Example 3.4: Assay on Activation of Human T cells by Anti-LAG-3/PD-L1 Bispecific Antibody of the Present Invention

The mixed lymphocyte reaction was employed in the present example to assay the activating effect of the anti-LAG-3/PD-L1 bispecific antibody of the present invention on human T cells. The specific assay process was as follows.

50 ml of fresh blood was collected from a donor, and added with PBS of 2.5-fold volume. The mixture was then gently added to 4 tubes each with 12.5 ml of FiColl (Thermo). The samples were centrifuged at 400 g for 30 min with zero deceleration. The middle white strip layer was pipetted into PBS and washed twice with PBS to obtain isolated PBMCs.

50 ml of isolated PBMC suspension was added with 5 ml of T cell medium X-VIVO 15 (LONZA) and cultured adherently in an incubator at 37 °C and 6% CO₂ for 2 h, and then the cell suspension was taken for CD4+ cell isolation. In addition, 3 ml of dendritic cell (DC) medium (X-VIVO 15 (Lonza) 99%, human AB serum (Access) 1%, HEPES 10 mM, β-Me 50 µM, IL-4 (R&D Systems) 1000U/ml, GM-CSF (R&D Systems) 1000U/ml) was added to the remaining cell suspension, and the cells continued to be cultured; on day 2 of culture, 3 ml of DC medium was then added; and on day 5 of culture, rTNFα (R&D Systems) (1000 U/ml), IL-1β (R&D Systems) (5 ng/ml), IL-6 (R&D Systems) (10 ng/ml) and 1 µM PGE2 (R&D Systems) were added, and isolated DC cells were obtained after two more days of culture as DC cells used in mixed lymphocyte reaction.

CD4+ cell isolation was carried out according to the instructions of Untouched CD4+ T Cell Isolation Kit (Invitrogen, catalog number: 11346D). In short, the aforementioned cell suspension taken from the PBMC suspension that had been statically cultured for 2 h was pipetted to a 20 ml centrifuge tube and centrifuged at 200 g for 10 min. The cell precipitate was resuspended with 500 µL of separating medium, 100 µL of AB type serum and 100 µL of purified antibody provided in the kit, and then incubated at 4 °C for 20 min. The mixture was washed once with separating medium, added with 500 µL of Bead Buffer, and then incubated for 15 min. Beads were removed by a magnetic field. The cells were washed once with T cell medium, resuspended with 8 ml of medium, and cultured at 37 °C and 6% CO₂ to obtain CD4+ cells.

1 × 10⁷ CD4+ cells were resuspended in 4 ml of X-VIVO 15 medium (LONZA), Dynabeads Human T-Activator CD3/CD28 (Invitrogen) was added at a ratio of 1:1, and then the cell suspension was cultured for 3 days to achieve the bead stimulation for CD4+ cells.

Mixed lymphocyte reaction (MLR) was carried out as follows. The aforementioned isolated DC cells were mixed with the bead-stimulated CD4+ cells in a 96-well cell culture plate (Nunc), with a volume of 200 µL, 10,000 DC cells and 100,000 CD4+ cells per well. Serial dilutions of the antibodies and 1 nM SEE (Toxin technology) were added. The IgG4 antibody prepared by the institution was used as a negative control. The mixture was cultured for 3 days. The IL2 concentration was assayed by using IL2 kit (Cisbio). The IgG4 control antibody has a heavy chain (HC) amino acid sequence shown as SEQ ID NO: 39 and a light chain (LC) amino acid sequence shown as SEQ ID NO: 40.

The experimental results are shown in FIG. 16. The bispecific antibodies of the present invention can activate T cells in vitro, and demonstrate an activating effect stronger than that of the anti-PD-L1 or anti-LAG-3 antibody used alone. The activating effect of the bispecific antibodies of the present invention on T cells in vitro is similar to that of the anti-PD-L1 and anti-LAG-3 antibodies used in combination.

Although certain representative embodiments and details have been shown for the purpose of illustrating the present invention, it will be apparent to those skilled in the art that various variations and modifications can be made to these embodiments and details without departing from the scope of the subject matter of the present invention. In this respect, the scope of the present invention is limited only by the following claims.

## Claims

1. A triple-chain antibody, comprising: a first polypeptide chain comprising a first heavy chain variable domain; a second polypeptide chain comprising a first light chain variable domain which is paired with the first heavy chain variable domain to form a first antigen-binding site; and a third polypeptide chain comprising a second single-domain antigen-binding site and a third single-domain antigen-binding site.

2. The triple-chain antibody of claim 1, wherein the first polypeptide chain comprises the first heavy chain variable domain and an immunoglobulin CHI domain, and the second polypeptide chain comprises the first light chain variable domain and an immunoglobulin CL domain.

3. The triple-chain antibody of claims 1 or 2, wherein the third polypeptide has or does not have a linker peptide between the second single-domain antigen-binding site and the third single-domain antigen-binding site, and
preferably, the third polypeptide chain does not comprise an immunoglobulin CHI domain.

4. The triple-chain antibody of any one of claims 1 to 3, wherein the second single-domain antigen-binding site and the third single-domain antigen-binding site are independently selected from a heavy chain variable domain (VH), a light chain variable domain (VL), a heavy chain variable domain of an antibody naturally devoid of a light chain (e.g., a heavy chain variable domain of a heavy chain antibody naturally existing in the Camelidae species), a VH-like single domain in an immunoglobulin known as a novel antigen receptor (NAR) in fish (e.g., IgNAR naturally existing in shark serum), and a recombinant single-domain antigen-binding site (e.g., a camelized human VH domain or a humanized Camelidae antibody heavy chain variable domain); for example, the second single-domain antigen-binding site and the third single-domain antigen-binding site are selected from a heavy chain variable domain of a heavy chain antibody naturally existing in the Camelidae species, a camelized human VH domain and a humanized Camelidae antibody heavy chain variable domain (referred to as "VHH" for short); and therefore, the second single-domain antigen-binding site and the third single-domain antigen-binding site are a first VHH and a second VHH respectively, the first VHH and the second VHH having the same or different sequences and binding the same or different epitopes.

5. The triple-chain antibody of any one of claims 1 to 4, wherein the first polypeptide chain comprises, from N-terminus to C-terminus, the first heavy chain variable domain, the immunoglobulin CHI domain, and an Fc domain; the second polypeptide chain comprises, from N-terminus to C-terminus, the first light chain variable domain and the immunoglobulin CL domain; and the third polypeptide chain comprises, from N-terminus to C-terminus, the second single-domain antigen-binding site, the third single-domain antigen-binding site and an Fc domain, preferably, the immunoglobulin is an IgG1, IgG2 or IgG4, and more preferably, the immunoglobulin is a human IgG1.

6. The triple-chain antibody of claim 5, wherein the Fc domains of the first polypeptide chain and the third polypeptide chain both comprise a hinge region of immunoglobulin constant region, and the first polypeptide chain and the third polypeptide chain are stably associated with each other through disulfide bonds at the hinge regions, for example, the Fc domains of the first polypeptide chain and the third polypeptide chain of the triple-chain antibody both comprise a hinge region having "CPPC" amino acid residues, such that the first polypeptide chain and the third polypeptide chain are stably associated with each other through disulfide bonds formed between the amino acid residues at the hinge regions; and
preferably, the first polypeptide chain and the third polypeptide chain further comprise Y349C and S354C or S354C and Y349C respectively (according to "EU numbering system" of Kabat) in Fc domains thereof, such that the first polypeptide chain and the third polypeptide chain further form interchain disulfide bonds in the Fc regions.

7. The triple-chain antibody of claims 5 or 6, wherein the first polypeptide chain and/or the third polypeptide chain comprise, in the Fc domains, mutations affecting the effector function of the antibody, such as LALA mutation.

8. The triple-chain antibody of any one of claims 5 to 7, wherein the first polypeptide chain and the third polypeptide chain respectively comprise a protuberance and a cavity in Fc domains thereof, or vice versa, the protuberance or cavity in the Fc domain of the first polypeptide chain can be placed at the cavity or protuberance in the Fc domain of the third polypeptide chain, and thus the first polypeptide chain and the third polypeptide chain form a stable "knob-in-hole" association with each other.

9. The triple-chain antibody of any one of claims 2 to 8, wherein the immunoglobulin CHI domain and CL domain respectively comprise a protuberance and a cavity, or vice versa, the protuberance or cavity in the CHI domain can be placed at the cavity or protuberance in the CL domain, and thus the first polypeptide chain and the second polypeptide chain form a stable "knob-in-hole" association with each other.

10. The triple-chain antibody of any one of claims 1 to 9, wherein the first antigen-binding site, the second antigen-binding site, and the third antigen-binding site bind to epitopes on the same or different antigen(s),
for example, the first antigen binding site binds to an epitope of a first antigen, and the second antigen binding site and the third antigen binding site bind to the same or different epitope(s) on a second antigen, thus the triple-chain antibody being a bispecific antibody against the first antigen and the second antigen;
the first antigen binding site binds to an epitope of a first antigen, and the second antigen binding site and the third antigen binding site bind respectively to an epitope of a second antigen and an epitope of a third antigen, and thus the triple-chain antibody is a trispecific antibody.

11. The triple-chain antibody of any one of claims 3 to 10, wherein the linker peptide comprises glycine (G) and serine (S) residues, for example, GGGGS repeats, and preferably four GGGGS repeats.

12. The triple-chain antibody of any one of claims 1 to 11, wherein the antigen is a cytokine, a growth factor, a hormone, a signal transduction protein, an inflammatory mediator, a ligand, a cell surface receptor, or a fragment thereof.

13. The triple-chain antibody of claim 12, wherein the antigen is selected from a tumor-associated antigen, an immune checkpoint molecule, a co-stimulatory molecule in the immune system, and a ligand and/or receptor thereof.

14. The triple-chain antibody of claim 13, wherein the antigen is selected from CD47, PD1, PD-L1, PD-L2, LAG-3, and 4-1BB (CD137).

15. The triple-chain antibody of any one of claims 1 to 14, wherein the triple-chain antibody is an anti-CD47/PD-L1 bispecific antibody, and the three antigen-binding sites thereof respectively bind to CD47 and/or PD-L1 molecules,
for example, the triple-chain antibody comprises a first antigen-binding site comprising a VH1/VL1 pair and specifically binding to CD47 on the first polypeptide chain and the second polypeptide chain, and a first VHH and a second VHH specifically binding to PD-L1 on the third polypeptide chain, or comprises a first antigen-binding site containing a VH1/VL1 pair and specifically binding to PD-L1 on the first polypeptide chain and the second polypeptide chain, and a first VHH and a second VHH specifically binding to CD47 on the third polypeptide chain;
preferably, the first antigen binding site specifically binding to CD47 and comprising the VH1/VL1 pair on the first polypeptide chain and the second polypeptide chain, comprises a VH CDR1 shown in GSIEHYYWS (SEQ ID NO: 3), a VH CDR2 shown in YIYYSGSTNYNPSLKS (SEQ ID NO: 4), a VH CDR3 shown in ARGKTGSAA (SEQ ID NO: 5), a VL CDR1 shown in RASQGISRWLA (SEQ ID NO: 10), a VL CDR2 shown in AASSLQS (SEQ ID NO: 11), and a VL CDR3 shown in QQTVSFPIT (SEQ ID NO: 12) derived from anti-CD47 antibody ADI-29341, or a sequence having one, two, three, four, or five amino acid changes (e.g., amino acid substitutions or deletions) from one or more of the six CDRs; the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or a sequence having one, two, three, four, or five amino acid changes (e.g., amino acid substitutions or deletions) from one or more of the three CDRs,
more preferably, the first antigen binding site specifically binding to CD47 and comprising a VH1/VL1 pair on the first polypeptide chain and the second polypeptide chain, comprises paired heavy-chain variable region/light-chain variable region sequences of SEQ ID NOs: 2/9 derived from anti-CD47 antibody ADI-29341, or a sequence having a sequence identity of at least 90%, 91%, 92%, 93%, 94%, 95%, 96%, 97%, 98%, 99% or higher with the paired heavy-chain variable region/light-chain variable region sequences, the second and the third single-domain antigen binding sites specifically binding to PD-L1 on the third polypeptide chain comprise an amino acid sequence shown in SEQ ID NO: 15 and/or SEQ ID NO: 16, or a sequence substantially identical (e.g., having at least 90%, 92%, 95%, 97%, 98%, 99% or higher identity) thereto,
most preferably, the triple-chain antibody comprises a first polypeptide chain shown in SEQ ID NO: 1, a second polypeptide chain shown in SEQ ID NO: 8, and a third polypeptide chain shown in SEQ ID NO: 14 or SEQ ID NO: 22, or a sequence substantially identical (e.g., having at least 80%, 85%, 90%, 92%, 95%, 97%, 98%, 99% or higher identity) to any one of the sequences.

16. The triple-chain antibody of any one of claims 1 to 14, wherein the triple-chain antibody is an anti-4-1BB/PD-L1 bispecific antibody, and the three antigen-binding sites thereof bind to 4-1BB and/or PD-L1 molecules,
for example, the triple-chain antibody comprises a first antigen-binding site comprising a VH1/VL1 pair and specifically binding to 4-1BB on the first polypeptide chain and the second polypeptide chain, and a first VHH and a second VHH specifically binding to PD-L1 on the third polypeptide chain, or comprises a first antigen-binding site containing a VH1/VL1 pair and specifically binding to PD-L1 on the first polypeptide chain and the second polypeptide chain, and a first VHH and a second VHH specifically binding to 4-1BB on the third polypeptide chain;
preferably, the first antigen binding site specifically binding to 4-1BB and comprising a VH1/VL1 pair on the first polypeptide chain and the second polypeptide chain, comprises all of the six heavy-chain complementarity determining regions (CDRs) and light-chain CDRs contained in the paired heavy-chain variable region/light-chain variable region sequences derived from SEQ ID NOs: 26/28, or a sequence having one, two, three, four, or five amino acid changes (e.g., amino acid substitutions or deletions) from one or more of the six CDRs; the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or a sequence having one, two, three, four, or five amino acid changes (e.g., amino acid substitutions or deletions) from one or more of the three CDRs,
more preferably, the first antigen binding site specifically binding to 4-1BB and comprising a VH1/VL1 pair on the first polypeptide chain and the second polypeptide chain, comprises paired heavy-chain variable region/light-chain variable region sequences derived from SEQ ID NOs: 26/28, or a sequence having a sequence identity of at least 90%, 91%, 92%, 93%, 94%, 95%, 96%, 97%, 98%, 99% or higher with the paired heavy-chain variable region/light-chain variable region sequences, the second and the third single-domain antigen binding sites specifically binding to PD-L1 on the third polypeptide chain comprise an amino acid sequence shown in SEQ ID NO: 15 and/or SEQ ID NO: 16, or a sequence substantially identical (e.g., having at least 80%, 85%, 90%, 92%, 95%, 97%, 98%, 99% or higher identity) thereto,
most preferably, the triple-chain antibody comprises a first polypeptide chain shown in SEQ ID NO: 25, a second polypeptide chain shown in SEQ ID NO: 27, and a third polypeptide chain shown in SEQ ID NO: 14 or SEQ ID NO: 22, or a sequence substantially identical (e.g., having at least 80%, 85%, 90%, 92%, 95%, 97%, 98%, 99% or higher identity) to any one of the sequences.

17. The triple-chain antibody of any one of claims 1 to 14, wherein the triple-chain antibody is an anti-LAG-3/PD-L1 bispecific antibody, and the three antigen-binding sites thereof bind to LAG-3 and/or PD-L1 molecules,
for example, the triple-chain antibody comprises a first antigen-binding site comprising a VH1/VL1 pair and specifically binding to LAG-3 on the first polypeptide chain and the second polypeptide chain, and a first VHH and a second VHH specifically binding to PD-L1 on the third polypeptide chain, or comprises a first antigen-binding site containing a VH1/VL1 pair and specifically binding to PD-L1 on the first polypeptide chain and the second polypeptide chain, and a first VHH and a second VHH specifically binding to LAG-3 on the third polypeptide chain;
preferably, the first antigen binding site specifically binding to LAG-3 and comprising the VH1/VL1 pair on the first polypeptide chain and the second polypeptide chain, comprises a VH CDR1 shown in GSIYSESYYWG (SEQ ID NO: 31), a VH CDR2 shown in SIVYSGYTYYNPSLKS (SEQ ID NO: 32), a VH CDR3 shown in ARVRTWDAAFDI (SEQ ID NO: 33), a VL CDR1 shown in QASQDISNYLN (SEQ ID NO: 36), a VL CDR2 shown in DASNLET (SEQ ID NO: 37), and a VL CDR3 shown in QQVLELPPWT (SEQ ID NO: 38) derived from anti-LAG-3 antibody ADI-31853, or a sequence having one, two, three, four, or five amino acid changes (e.g., amino acid substitutions or deletions) from one or more of the six CDRs; the second and the third single-domain antigen binding sites specifically binding to PD-L1 on the third polypeptide chain comprise a CDR1 shown in SEQ ID NO: 17, a CDR2 shown in SEQ ID NO: 18, a CDR3 shown in SEQ ID NO: 19, or a sequence having one, two, three, four, or five amino acid changes (e.g., amino acid substitutions or deletions) from one or more of the three CDRs,
more preferably, the first antigen binding site specifically binding to LAG-3 and comprising a VH1/VL1 pair on the first polypeptide chain and the second polypeptide chain, comprises paired heavy-chain variable region/light-chain variable region sequences derived from SEQ ID NOs: 30/35, or a sequence having a sequence identity of at least 90%, 91%, 92%, 93%, 94%, 95%, 96%, 97%, 98%, 99% or higher with the paired heavy-chain variable region/light-chain variable region sequences, the second and the third single-domain antigen binding sites specifically binding to PD-L1 on the third polypeptide chain comprise an amino acid sequence shown in SEQ ID NO: 15 and/or SEQ ID NO: 16, or a sequence substantially identical (e.g., having at least 80%, 85%, 90%, 92%, 95%, 97%, 98%, 99% or higher identity) thereto,
most preferably, the triple-chain antibody comprises a first polypeptide chain shown in SEQ ID NO: 29, a second polypeptide chain shown in SEQ ID NO: 34, and a third polypeptide chain shown in SEQ ID NO: 14 or SEQ ID NO: 22, or a sequence substantially identical (e.g., having at least 80%, 85%, 90%, 92%, 95%, 97%, 98%, 99% or higher identity) to any one of the sequences.

18. A polynucleotide, coding the first polypeptide chain, the second polypeptide chain and/or the third polypeptide chain in the triple-chain antibody of any one of claims 1 to 17.

19. A vector, and preferably an expression vector, comprising the polynucleotide coding the first polypeptide chain, the second polypeptide chain and/or the third polypeptide chain in the triple-chain antibody of any one of claims 1 to 17.

20. A host cell, comprising the polynucleotide of claim 18 or the vector of claim 19, wherein, for example, the host cell is a mammal cell, and preferably a CHO cell or an HEK293 cell; and the host cell is a prokaryotic cell, and preferably an E. coli cell.

21. A method for producing the triple-chain antibody of any one of claims 1 to 17, wherein the method comprises: (i) cultivating the host cell of claim 20 under conditions suitable for expressing the triple-chain antibody; and (ii) isolating the triple-chain antibody from the host cell or the cultures.

22. A pharmaceutical composition, comprising the triple-chain antibody of any one of claims 1 to 17 and a pharmaceutically acceptable carrier.

23. The pharmaceutical composition of claim 22, further comprising at least one other active ingredient.

24. Use of the triple-chain antibody of any one of claims 1 to 17 or the pharmaceutical composition of claims 22 or 23 as a medication for treating and/or preventing diseases in an individual or as a diagnostic tool of diseases, wherein the individual is preferably a mammal, and more preferably a human.

25. The use of claim 24, being used for the treatment and/or prevention or diagnosis of an autoimmune disease, an acute or chronic inflammatory disease, an infectious disease (e.g., chronic communicable disease or septicemia), or a tumor, wherein, for example, the tumors is selected from a solid tumor, a hematological cancer (e.g., leukemia, lymphoma, and myeloma such as multiple myeloma), and a metastatic lesion.

26. The use of claim 24, being used for promoting implantation of hematopoietic stem cells in a subject in need.


SEQUENCE LISITNG
<110>  Innovent Biologics (Suzhou) Co., Ltd.
<120>  Triple-Chain Antibody, Method for Preparation and Use thereof
<130>  P00005CN007
<160>  40
<170>  PatentIn version 3.3
<210>  1
<211>  444
<212>  PRT
<213>  Artificial Sequence
<220>
<223>  The first peptide chain of anti-CD47/PDL1 bispecific antibody
<400>  1
Gln Val Gln Leu Gln Glu Ser Gly Pro Gly Leu Val Lys Pro Ser Glu
1               5                   10                  15
Thr Leu Ser Leu Thr Cys Thr Val Ser Gly Gly Ser Ile Glu His Tyr
            20                  25                  30
Tyr Trp Ser Trp Ile Arg Gln Pro Pro Gly Lys Gly Leu Glu Trp Ile
        35                  40                  45
Gly Tyr Ile Tyr Tyr Ser Gly Ser Thr Asn Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg Gly Lys Thr Gly Ser Ala Ala Trp Gly Gln Gly Thr Leu Val Thr
            100                 105                 110
Val Ser Ser Ala Ser Thr Lys Gly Pro Ser Val Phe Pro Leu Ala Pro
        115                 120                 125
Ser Ser Lys Ser Thr Ser Gly Gly Thr Ala Ala Leu Gly Cys Leu Val
    130                 135                 140
Lys Asp Tyr Phe Pro Glu Pro Val Thr Val Ser Trp Asn Ser Gly Ala
145                 150                 155                 160
Leu Thr Ser Gly Val His Thr Phe Pro Ala Val Leu Gln Ser Ser Gly
                165                 170                 175
Leu Tyr Ser Leu Ser Ser Val Val Thr Val Pro Ser Ser Ser Leu Gly
            180                 185                 190
Thr Gln Thr Tyr Ile Cys Asn Val Asn His Lys Pro Ser Asn Thr Lys
        195                 200                 205
Val Asp Lys Lys Val Glu Pro Lys Ser Cys Asp Lys Thr His Thr Cys
    210                 215                 220
Pro Pro Cys Pro Ala Pro Glu Ala Ala Gly Gly Pro Ser Val Phe Leu
225                 230                 235                 240
Phe Pro Pro Lys Pro Lys Asp Thr Leu Met Ile Ser Arg Thr Pro Glu
                245                 250                 255
Val Thr Cys Val Val Val Asp Val Ser His Glu Asp Pro Glu Val Lys
            260                 265                 270
Phe Asn Trp Tyr Val Asp Gly Val Glu Val His Asn Ala Lys Thr Lys
        275                 280                 285
Pro Arg Glu Glu Gln Tyr Asn Ser Thr Tyr Arg Val Val Ser Val Leu
    290                 295                 300
Thr Val Leu His Gln Asp Trp Leu Asn Gly Lys Glu Tyr Lys Cys Lys
305                 310                 315                 320
Val Ser Asn Lys Ala Leu Pro Ala Pro Ile Glu Lys Thr Ile Ser Lys
                325                 330                 335
Ala Lys Gly Gln Pro Arg Glu Pro Gln Val Tyr Thr Leu Pro Pro Cys
            340                 345                 350
Arg Asp Glu Leu Thr Lys Asn Gln Val Ser Leu Trp Cys Leu Val Lys
        355                 360                 365
Gly Phe Tyr Pro Ser Asp Ile Ala Val Glu Trp Glu Ser Asn Gly Gln
    370                 375                 380
Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415
Gln Gly Asn Val Phe Ser Cys Ser Val Met His Glu Ala Leu His Asn
            420                 425                 430
His Tyr Thr Gln Lys Ser Leu Ser Leu Ser Pro Gly
        435                 440
<210> 2
<211> 115
<212> PRT
<213> Artificial Sequence
<220>
<223> VH of anti-CD47 antibody ADI29341
<400> 2
Gln Val Gln Leu Gln Glu Ser Gly Pro Gly Leu Val Lys Pro Ser Glu
1               5                   10                  15
Thr Leu Ser Leu Thr Cys Thr Val Ser Gly Gly Ser Ile Glu His Tyr
            20                  25                  30
Tyr Trp Ser Trp Ile Arg Gln Pro Pro Gly Lys Gly Leu Glu Trp Ile
        35                  40                  45
Gly Tyr Ile Tyr Tyr Ser Gly Ser Thr Asn Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg Gly Lys Thr Gly Ser Ala Ala Trp Gly Gln Gly Thr Leu Val Thr
            100                 105                 110
Val Ser Ser
        115
<210> 3
<211> 9
<212> PRT
<213> Artificial Sequence
<220>
<223> VH CDR1 of anti-CD47 antibody ADI29341
<400> 3
Gly Ser Ile Glu His Tyr Tyr Trp Ser
1               5
<210> 4
<211> 16
<212> PRT
<213> Artificial Sequence
<220>
<223> VH CDR2 of anti-CD47 antibody ADI29341
<400> 4
Tyr Ile Tyr Tyr Ser Gly Ser Thr Asn Tyr Asn Pro Ser Leu Lys Ser
1               5                   10                  15
<210> 5
<211> 9
<212> PRT
<213> Artificial Sequence
<220>
<223> VH CDR3 of anti-CD47 antibody ADI29341
<400> 5
Ala Arg Gly Lys Thr Gly Ser Ala Ala
1               5
<210> 6
<211> 108
<212> PRT
<213> Artificial Sequence
<220>
<223> Derived from human IgG1 CH1
<400> 6

Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr
            100                 105
<210>  7
<211>  221
<212>  PRT
<213>  Artificial Sequence
<220>
<223>  Derived from human IgG1 Fc
<400>  7
Cys Pro Pro Cys Pro Ala Pro Glu Ala Ala Gly Gly Pro Ser Val Phe
1               5                   10                  15
Leu Phe Pro Pro Lys Pro Lys Asp Thr Leu Met Ile Ser Arg Thr Pro
            20                  25                  30
Glu Val Thr Cys Val Val Val Asp Val Ser His Glu Asp Pro Glu Val
        35                  40                  45
Lys Phe Asn Trp Tyr Val Asp Gly Val Glu Val His Asn Ala Lys Thr
    50                  55                  60
Lys Pro Arg Glu Glu Gln Tyr Asn Ser Thr Tyr Arg Val Val Ser Val
65                  70                  75                  80
Leu Thr Val Leu His Gln Asp Trp Leu Asn Gly Lys Glu Tyr Lys Cys
                85                  90                  95
Lys Val Ser Asn Lys Ala Leu Pro Ala Pro Ile Glu Lys Thr Ile Ser
            100                 105                 110
Lys Ala Lys Gly Gln Pro Arg Glu Pro Gln Val Tyr Thr Leu Pro Pro
        115                 120                 125
Cys Arg Asp Glu Leu Thr Lys Asn Gln Val Ser Leu Trp Cys Leu Val
    130                 135                 140
Lys Gly Phe Tyr Pro Ser Asp Ile Ala Val Glu Trp Glu Ser Asn Gly
145                 150                 155                 160
Gln Pro Glu Asn Asn Tyr Lys Thr Thr Pro Pro Val Leu Asp Ser Asp
                165                 170                 175
Gly Ser Phe Phe Leu Tyr Ser Lys Leu Thr Val Asp Lys Ser Arg Trp
            180                 185                 190
Gln Gln Gly Asn Val Phe Ser Cys Ser Val Met His Glu Ala Leu His
        195                 200                 205
Asn His Tyr Thr Gln Lys Ser Leu Ser Leu Ser Pro Gly
    210                 215                 220
<210>  8
<211>  214
<212>  PRT
<213>  Artificial Sequence
<220>
<223>  The second peptide chain of anti-CD47/PDL1 bispecific antibody
<400>  8
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Arg Trp
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly

50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Val Ser Phe Pro Ile
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
<210>  9
<211>  107
<212>  PRT
<213>  Artificial Sequence
<220>
<223>  VL of anti-CD47 antibody ADI29341
<400>  9
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Arg Trp
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Val Ser Phe Pro Ile
                85                  90                  95
Thr Phe Gly Gly Gly Thr Lys Val Glu Ile Lys
            100                 105
<210>  10
<211>  11
<212>  PRT
<213>  Artificial Sequence
<220>
<223>  VL CDR1 of anti-CD47 antibody ADI29341
<400>  10
Arg Ala Ser Gln Gly Ile Ser Arg Trp Leu Ala
1               5                   10
<210>  11
<211>  7
<212>  PRT
<213>  Artificial Sequence
<220>
<223>  VL CDR2 of anti-CD47 antibody ADI29341
<400>  11
Ala Ala Ser Ser Leu Gln Ser
1               5
<210>  12
<211>  9
<212>  PRT

<213>  Artificial Sequence
<220>
<223>  VL CDR3 of anti-CD47 antibody ADI29341
<400>  12
Gln Gln Thr Val Ser Phe Pro Ile Thr
1               5
<210>  13
<211>  107
<212>  PRT
<213>  Homo sapiens
<220>
<223>  Human kappa light chain amino acid sequence
<400>  13
<210>  14
<211>  510
<212>  PRT
<213>  Artificial Sequence
<220>
<223>  The third peptide chain of bispecific antibody (with linker peptide
between VHHs)
<400>  14
Gln Val Gln Leu Gln Glu Ser Gly Gly Gly Leu Val Gln Pro Gly Gly
1               5                   10                  15
Ser Leu Arg Leu Ser Cys Ala Ala Ser Ala Tyr Thr Ile Ser Arg Asn
            20                  25                  30
Ser Met Gly Trp Phe Arg Gln Ala Pro Gly Lys Gly Leu Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Asp Asn Ser Lys Asn Thr Leu Tyr Leu
65                  70                  75                  80
Glu Met Asn Ser Leu Arg Ala Glu Asp Thr Ala Val Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Leu Val
        115                 120                 125
Thr Val Ser Ser Gly Gly Gly Gly Ser Gly Gly Gly Gly Ser Gly Gly
    130                 135                 140
Gly Gly Ser Gly Gly Gly Gly Ser Gln Val Gln Leu Gln Glu Ser Gly
145                 150                 155                 160
Gly Gly Leu Val Gln Pro Gly Gly Ser Leu Arg Leu Ser Cys Ala Ala
                165                 170                 175
Ser Ala Tyr Thr Ile Ser Arg Asn Ser Met Gly Trp Phe Arg Gln Ala
            180                 185                 190
Pro Gly Lys Gly Leu Glu Gly Val Ala Ala Ile Glu Ser Asp Gly Ser
        195                 200                 205
Thr Ser Tyr Ser Asp Ser Val Lys Gly Arg Phe Thr Ile Ser Leu Asp
    210                 215                 220

Asn Ser Lys Asn Thr Leu Tyr Leu Glu Met Asn Ser Leu Arg Ala Glu
225                 230                 235                 240
Asp Thr Ala Val Tyr Tyr Cys Ala Ala Pro Lys Val Gly Leu Gly Pro
                245                 250                 255
Arg Thr Ala Leu Gly His Leu Ala Phe Met Thr Leu Pro Ala Leu Asn
            260                 265                 270
Tyr Trp Gly Gln Gly Thr Leu Val Thr Val Ser Ser Asp Lys Thr His
        275                 280                 285
Thr Cys Pro Pro Cys Pro Ala Pro Glu Ala Ala Gly Gly Pro Ser Val
    290                 295                 300
Phe Leu Phe Pro Pro Lys Pro Lys Asp Thr Leu Met Ile Ser Arg Thr
305                 310                 315                 320
Pro Glu Val Thr Cys Val Val Val Asp Val Ser His Glu Asp Pro Glu
                325                 330                 335
Val Lys Phe Asn Trp Tyr Val Asp Gly Val Glu Val His Asn Ala Lys
            340                 345                 350
Thr Lys Pro Arg Glu Glu Gln Tyr Asn Ser Thr Tyr Arg Val Val Ser
        355                 360                 365
Val Leu Thr Val Leu His Gln Asp Trp Leu Asn Gly Lys Glu Tyr Lys
    370                 375                 380
Cys Lys Val Ser Asn Lys Ala Leu Pro Ala Pro Ile Glu Lys Thr Ile
385                 390                 395                 400
Ser Lys Ala Lys Gly Gln Pro Arg Glu Pro Gln Val Cys Thr Leu Pro
                405                 410                 415
Pro Ser Arg Asp Glu Leu Thr Lys Asn Gln Val Ser Leu Ser Cys Ala
            420                 425                 430
Val Lys Gly Phe Tyr Pro Ser Asp Ile Ala Val Glu Trp Glu Ser Asn
        435                 440                 445
Gly Gln Pro Glu Asn Asn Tyr Lys Thr Thr Pro Pro Val Leu Asp Ser
    450                 455                 460
Asp Gly Ser Phe Phe Leu Val Ser Lys Leu Thr Val Asp Lys Ser Arg
465                 470                 475                 480
Trp Gln Gln Gly Asn Val Phe Ser Cys Ser Val Met His Glu Ala Leu
                485                 490                 495
His Asn His Tyr Thr Gln Lys Ser Leu Ser Leu Ser Pro Gly
            500                 505                 510
<210>  15
<211>  132
<212>  PRT
<213>  Camelus linnaeus
<220>
<223> Camel VHH
<400>  15
Gln Val Gln Leu Gln Glu Ser Gly Gly Gly Ser Val Gln Ala Gly Gly
1               5                   10                  15
Ser Leu Arg Leu Ser Cys Gln Ala Ser Ala Tyr Thr Ile Ser Arg Asn
            20                  25                  30
Ser Met Gly Trp Phe Arg Gln Ala Pro Gly Lys Gln Arg Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Gly Asn Ala Lys Asn Thr Leu Tyr Leu
65                  70                  75                  80
Glu Met Asn Ser Leu Lys Pro Glu Asp Thr Ala Met Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Gln Val
        115                 120                 125
Thr Val Ser Ser
    130
<210>  16
<211>  132

<212>  PRT
<213>  Artificial Sequence
<220>
<223>  Humanized VHH
<400>  16
Gln Val Gln Leu Gln Glu Ser Gly Gly Gly Leu Val Gln Pro Gly Gly
1               5                   10                  15
Ser Leu Arg Leu Ser Cys Ala Ala Ser Ala Tyr Thr Ile Ser Arg Asn
            20                  25                  30
Ser Met Gly Trp Phe Arg Gln Ala Pro Gly Lys Gly Leu Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Asp Asn Ser Lys Asn Thr Leu Tyr Leu
65                  70                  75                  80
Glu Met Asn Ser Leu Arg Ala Glu Asp Thr Ala Val Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Leu Val
        115                 120                 125
Thr Val Ser Ser
    130
<210>  17
<211>  10
<212>  PRT
<213>  Camelus linnaeus
<400>  17
Ala Tyr Thr Ile Ser Arg Asn Ser Met Gly
1               5                   10
<210>  18
<211>  7
<212>  PRT
<213>  Camelus linnaeus
<400>  18
Ile Glu Ser Asp Gly Ser Thr
1               5
<210>  19
<211>  26
<212>  PRT
<213>  Camelus linnaeus
<400>  19
Ala Ala Pro Lys Val Gly Leu Gly Pro Arg Thr Ala Leu Gly His Leu
1               5                   10                  15
Ala Phe Met Thr Leu Pro Ala Leu Asn Tyr
            20                  25
<210>  20
<211>  20
<212>  PRT
<213>  Artificial Sequence
<400>  20
Gly Gly Gly Gly Ser Gly Gly Gly Gly Ser Gly Gly Gly Gly Ser Gly
1               5                   10                  15
Gly Gly Gly Ser
            20
<210>  21
<211>  226
<212>  PRT
<213>  Artificial Sequence
<220>
<223>  Derived from human IgG1 Fc
<400>  21
Asp Lys Thr His Thr Cys Pro Pro Cys Pro Ala Pro Glu Ala Ala Gly

1               5                   10                  15
Gly Pro Ser Val Phe Leu Phe Pro Pro Lys Pro Lys Asp Thr Leu Met
            20                  25                  30
Ile Ser Arg Thr Pro Glu Val Thr Cys Val Val Val Asp Val Ser His
        35                  40                  45
Glu Asp Pro Glu Val Lys Phe Asn Trp Tyr Val Asp Gly Val Glu Val
    50                  55                  60
His Asn Ala Lys Thr Lys Pro Arg Glu Glu Gln Tyr Asn Ser Thr Tyr
65                  70                  75                  80
Arg Val Val Ser Val Leu Thr Val Leu His Gln Asp Trp Leu Asn Gly
                85                  90                  95
Lys Glu Tyr Lys Cys Lys Val Ser Asn Lys Ala Leu Pro Ala Pro Ile
            100                 105                 110
Glu Lys Thr Ile Ser Lys Ala Lys Gly Gln Pro Arg Glu Pro Gln Val
        115                 120                 125
Cys Thr Leu Pro Pro Ser Arg Asp Glu Leu Thr Lys Asn Gln Val Ser
    130                 135                 140
Leu Ser Cys Ala Val Lys Gly Phe Tyr Pro Ser Asp Ile Ala Val Glu
145                 150                 155                 160
Trp Glu Ser Asn Gly Gln Pro Glu Asn Asn Tyr Lys Thr Thr Pro Pro
                165                 170                 175
Val Leu Asp Ser Asp Gly Ser Phe Phe Leu Val Ser Lys Leu Thr Val
            180                 185                 190
Asp Lys Ser Arg Trp Gln Gln Gly Asn Val Phe Ser Cys Ser Val Met
        195                 200                 205
His Glu Ala Leu His Asn His Tyr Thr Gln Lys Ser Leu Ser Leu Ser
    210                 215                 220
Pro Gly
225
<210>  22
<211>  490
<212>  PRT
<213>  Artificial Sequence
<220>
<223> The third peptide chain of bispecific antibody (without linker peptide
between VHHs)
<400>  22
Gln Val Gln Leu Gln Glu Ser Gly Gly Gly Leu Val Gln Pro Gly Gly
1               5                   10                  15
Ser Leu Arg Leu Ser Cys Ala Ala Ser Ala Tyr Thr Ile Ser Arg Asn
            20                  25                  30
Ser Met Gly Trp Phe Arg Gln Ala Pro Gly Lys Gly Leu Glu Gly Val
        35                  40                  45
Ala Ala Ile Glu Ser Asp Gly Ser Thr Ser Tyr Ser Asp Ser Val Lys
    50                  55                  60
Gly Arg Phe Thr Ile Ser Leu Asp Asn Ser Lys Asn Thr Leu Tyr Leu
65                  70                  75                  80
Glu Met Asn Ser Leu Arg Ala Glu Asp Thr Ala Val Tyr Tyr Cys Ala
                85                  90                  95
Ala Pro Lys Val Gly Leu Gly Pro Arg Thr Ala Leu Gly His Leu Ala
            100                 105                 110
Phe Met Thr Leu Pro Ala Leu Asn Tyr Trp Gly Gln Gly Thr Leu Val
        115                 120                 125
Thr Val Ser Ser Gln Val Gln Leu Gln Glu Ser Gly Gly Gly Leu Val
    130                 135                 140
Gln Pro Gly Gly Ser Leu Arg Leu Ser Cys Ala Ala Ser Ala Tyr Thr
145                 150                 155                 160
Ile Ser Arg Asn Ser Met Gly Trp Phe Arg Gln Ala Pro Gly Lys Gly
                165                 170                 175
Leu Glu Gly Val Ala Ala Ile Glu Ser Asp Gly Ser Thr Ser Tyr Ser
            180                 185                 190
Asp Ser Val Lys Gly Arg Phe Thr Ile Ser Leu Asp Asn Ser Lys Asn
        195                 200                 205

Thr Leu Tyr Leu Glu Met Asn Ser Leu Arg Ala Glu Asp Thr Ala Val
    210                 215                 220
Tyr Tyr Cys Ala Ala Pro Lys Val Gly Leu Gly Pro Arg Thr Ala Leu
225                 230                 235                 240
Gly His Leu Ala Phe Met Thr Leu Pro Ala Leu Asn Tyr Trp Gly Gln
                245                 250                 255
Gly Thr Leu Val Thr Val Ser Ser Asp Lys Thr His Thr Cys Pro Pro
            260                 265                 270
Cys Pro Ala Pro Glu Ala Ala Gly Gly Pro Ser Val Phe Leu Phe Pro
        275                 280                 285
Pro Lys Pro Lys Asp Thr Leu Met Ile Ser Arg Thr Pro Glu Val Thr
    290                 295                 300
Cys Val Val Val Asp Val Ser His Glu Asp Pro Glu Val Lys Phe Asn
305                 310                 315                 320
Trp Tyr Val Asp Gly Val Glu Val His Asn Ala Lys Thr Lys Pro Arg
                325                 330                 335
Glu Glu Gln Tyr Asn Ser Thr Tyr Arg Val Val Ser Val Leu Thr Val
            340                 345                 350
Leu His Gln Asp Trp Leu Asn Gly Lys Glu Tyr Lys Cys Lys Val Ser
        355                 360                 365
Asn Lys Ala Leu Pro Ala Pro Ile Glu Lys Thr Ile Ser Lys Ala Lys
    370                 375                 380
Gly Gln Pro Arg Glu Pro Gln Val Cys Thr Leu Pro Pro Ser Arg Asp
385                 390                 395                 400
Glu Leu Thr Lys Asn Gln Val Ser Leu Ser Cys Ala Val Lys Gly Phe
                405                 410                 415
Tyr Pro Ser Asp Ile Ala Val Glu Trp Glu Ser Asn Gly Gln Pro Glu
            420                 425                 430
Asn Asn Tyr Lys Thr Thr Pro Pro Val Leu Asp Ser Asp Gly Ser Phe
        435                 440                 445
Phe Leu Val Ser Lys Leu Thr Val Asp Lys Ser Arg Trp Gln Gln Gly
    450                 455                 460
Asn Val Phe Ser Cys Ser Val Met His Glu Ala Leu His Asn His Tyr
465                 470                 475                 480
Thr Gln Lys Ser Leu Ser Leu Ser Pro Gly
                485                 490
<210>  23
<211>  449
<212>  PRT
<213>  Artificial Sequence
<220>
<223> IgG1 HC
<400>  23
Glu Val Arg Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Ala Met Gly Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Thr Ser Arg Asp Asp Ser Lys Asn Ala Leu Tyr
65                  70                  75                  80
Leu Gln Met Asn Ser Leu Arg Ala Glu Asp Thr Ala Val Tyr Tyr Cys
                85                  90                  95
Ala Arg Gly Gly Pro Gly Trp Tyr Ala Ala Asp Val Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Ala Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys
<210>  24
<211>  214
<212>  PRT
<213>  Artificial Sequence
<220>
<223>  IgG1 LC
<400>  24
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asp Leu Pro Ala Phe
                85                  90                  95
Ala Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln

145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
<210>  25
<211>  450
<212>  PRT
<213>  Artificial Sequence
<220>
<223>  The first peptide chain of anti-4-1BB/PDL1 bispecific antibody
<400>  25
Gln Val Gln Leu Gln Gln Trp Gly Ala Gly Leu Leu Lys Pro Ser Glu
1               5                   10                  15
Thr Leu Ser Leu Thr Cys Ala Val Tyr Gly Gly Ser Phe Ser Gly Tyr
            20                  25                  30
Tyr Trp Ser Trp Ile Arg Gln Ser Pro Glu Lys Gly Leu Glu Trp Ile
        35                  40                  45
Gly Glu Ile Asn His Gly Gly Tyr Val Thr Tyr Asn Pro Ser Leu Glu
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg Asp Tyr Gly Pro Gly Asn Tyr Asp Trp Tyr Phe Asp Leu Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Ala Ala Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Cys Arg Asp Glu Leu Thr Lys Asn Gln Val Ser
        355                 360                 365
Leu Trp Cys Leu Val Lys Gly Phe Tyr Pro Ser Asp Ile Ala Val Glu

370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly
    450
<210>  26
<211>  121
<212>  PRT
<213>  Artificial Sequence
<220>
<223>  VH of anti-4-1BB antibody BMS-663513
<400>  26
<210>  27
<211>  216
<212>  PRT
<213>  Artificial Sequence
<220>
<223>  The second peptide chain of anti-4-1BB/PDL1 bispecific antibody
<400>  27
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Arg Ser Asn Trp Pro Pro
                85                  90                  95
Ala Leu Thr Phe Gly Gly Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175

Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
<210>  28
<211>  109
<212>  PRT
<213>  Artificial Sequence
<220>
<223>  VL of anti-4-1BB antibody BMS-663513
<400>  28
<210>  29
<211>  449
<212>  PRT
<213>  Artificial Sequence
<220>
<223>  The first peptide chain of anti-LAG-3/PD-L1 antibody
<400>  29
Gln Leu Gln Leu Gln Glu Ser Gly Pro Gly Leu Val Lys Pro Ser Glu
1               5                   10                  15
Thr Leu Ser Leu Thr Cys Thr Val Ser Gly Gly Ser Ile Tyr Ser Glu
            20                  25                  30
Ser Tyr Tyr Trp Gly Trp Ile Arg Gln Pro Pro Gly Lys Gly Leu Glu
        35                  40                  45
Trp Ile Gly Ser Ile Val Tyr Ser Gly Tyr Thr Tyr Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Val Arg Thr Trp Asp Ala Ala Phe Asp Ile Trp Gly Gln
            100                 105                 110
Gly Thr Met Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Ala Ala Gly Gly

225                     230                     235                     240
Pro Ser Val Phe Leu Phe Pro Pro Lys Pro Lys Asp Thr Leu Met Ile
                245                     250                     255
Ser Arg Thr Pro Glu Val Thr Cys Val Val Val Asp Val Ser His Glu
            260                     265                     270
Asp Pro Glu Val Lys Phe Asn Trp Tyr Val Asp Gly Val Glu Val His
        275                     280                     285
Asn Ala Lys Thr Lys Pro Arg Glu Glu Gln Tyr Asn Ser Thr Tyr Arg
    290                     295                     300
Val Val Ser Val Leu Thr Val Leu His Gln Asp Trp Leu Asn Gly Lys
305                     310                     315                     320
Glu Tyr Lys Cys Lys Val Ser Asn Lys Ala Leu Pro Ala Pro Ile Glu
                325                     330                     335
Lys Thr Ile Ser Lys Ala Lys Gly Gln Pro Arg Glu Pro Gln Val Tyr
            340                     345                     350
Thr Leu Pro Pro Cys Arg Asp Glu Leu Thr Lys Asn Gln Val Ser Leu
        355                     360                     365
Trp Cys Leu Val Lys Gly Phe Tyr Pro Ser Asp Ile Ala Val Glu Trp
    370                     375                     380
Glu Ser Asn Gly Gln Pro Glu Asn Asn Tyr Lys Thr Thr Pro Pro Val
385                     390                     395                     400
Leu Asp Ser Asp Gly Ser Phe Phe Leu Tyr Ser Lys Leu Thr Val Asp
                405                     410                     415
Lys Ser Arg Trp Gln Gln Gly Asn Val Phe Ser Cys Ser Val Met His
            420                     425                     430
Glu Ala Leu His Asn His Tyr Thr Gln Lys Ser Leu Ser Leu Ser Pro
        435                     440                     445
Gly

<210>  30
<211>  120
<212>  PRT
<213>  Artificial Sequence
<220>
<223>  VH of anti-LAG-3 antibody ADI-31853
<400>  30
Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Tyr Ser Glu
            20                  25                  30
Ser Tyr Tyr Trp Gly Trp Ile Arg Gln Pro Pro Gly Lys Gly Leu Glu
        35                  40                  45
Trp Ile Gly Ser Ile Val Tyr Ser Gly Tyr Thr Tyr Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Val Arg Thr Trp Asp Ala Ala Phe Asp Ile Trp Gly Gln
            100                 105                 110
Gly Thr Met Val Thr Val Ser Ser
        115                 120
<210>  31
<211>  11
<212>  PRT
<213>  Artificial Sequence
<220>
<223>  VH CDR1 of anti-LAG-3 antibody ADI-31853
<400>  31
Gly Ser Ile Tyr Ser Glu Ser Tyr Tyr Trp Gly
1               5                   10
<210>  32

<211>  16
<212>  PRT
<213>  Artificial Sequence
<220>
<223>  VH CDR2 of anti-LAG-3 antibody ADI-31853
<400>  32
Ser Ile Val Tyr Ser Gly Tyr Thr Tyr Tyr Asn Pro Ser Leu Lys Ser
1               5                   10                  15
<210>  33
<211>  12
<212>  PRT
<213>  Artificial Sequence
<220>
<223>  VH CDR3 of anti-LAG-3 antibody ADI-31853
<400>  33
Ala Arg Val Arg Thr Trp Asp Ala Ala Phe Asp Ile
1               5                   10
<210>  34
<211>  215
<212>  PRT
<213>  Artificial Sequence
<220>
<223>  The second peptide chain of anti-LAG-3/PD-L1 bispecific antibody
<400>  34
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Asp Ala Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Leu Glu Leu Pro Pro
                85                  90                  95
Trp Thr Phe Gly Gly Gly Thr Lys Val Glu Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215
<210>  35
<211>  108
<212>  PRT
<213>  Artificial Sequence
<220>
<223>  VL of anti-LAG-3 antibody ADI-31853
<400>  35
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45
Tyr Asp Ala Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Leu Glu Leu Pro Pro
                85                  90                  95
Trp Thr Phe Gly Gly Gly Thr Lys Val Glu Ile Lys
            100                 105
<210>  36
<211>  11
<212>  PRT
<213>  Artificial Sequence
<220>
<223>  VL CDR1 of anti-LAG-3 antibody ADI-31853
<400>  36
Gln Ala Ser Gln Asp Ile Ser Asn Tyr Leu Asn
1               5                   10
<210>  37
<211>  7
<212>  PRT
<213>  Artificial Sequence
<220>
<223>  VL CDR2 of anti-LAG-3 antibody ADI-31853
<400>  37
Asp Ala Ser Asn Leu Glu Thr
1               5
<210>  38
<211>  10
<212>  PRT
<213>  Artificial Sequence
<220>
<223>  VL CDR3 of anti-LAG-3 antibody ADI-31853
<400>  38
Gln Gln Val Leu Glu Leu Pro Pro Trp Thr
1               5                   10
<210>  39
<211>  446
<212>  PRT
<213>  Artificial Sequence
<220>
<223>  IgG4 HC
<400>  39
Glu Val Arg Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Ala Met Gly Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Thr Ser Arg Asp Asp Ser Lys Asn Ala Leu Tyr
65                  70                  75                  80
Leu Gln Met Asn Ser Leu Arg Ala Glu Asp Thr Ala Val Tyr Tyr Cys
                85                  90                  95
Ala Arg Gly Gly Pro Gly Trp Tyr Ala Ala Asp Val Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp

145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Lys
        435                 440                 445
<210>  40
<211>  214
<212>  PRT
<213>  Artificial Sequence
<220>
<223>  IgG4 LC
<400>  40
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asp Leu Pro Ala Phe
                85                  90                  95
Ala Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln

145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210